(19) Europäisches Patentamt
European Patent Office
Office européen des brevets

(11) EP 1 020 459 B1

(12) EUROPEAN PATENT SPECIFICATION

(45) Date of publication and mention of the grant of the patent:
**06.04.2005 Bulletin 2005/14**

(21) Application number: **98921738.5**

(22) Date of filing: **20.05.1998**

(51) Int Cl.[7]: **C07D 401/04**, C07D 471/04, C07D 498/06, C07D 207/09, A61K 31/47, A61K 31/535, A61P 31/04

(86) International application number:
**PCT/JP1998/002219**

(87) International publication number:
**WO 1998/052939 (26.11.1998 Gazette 1998/47)**

# (54) CIS-DISUBSTITUTED AMINOCYCLOALKYL-PYRROLIDINE DERIVATIVES

CIS-DISUBSTITUIERTE AMINOCYCLOALKYL-PYRROLIDIN-DERIVATE

DERIVES D'AMINOCYCLOALKYLE-PYRROLIDINE CIS-DISUBSTITUES

(84) Designated Contracting States:
**AT BE CH CY DE DK ES FI FR GB GR IE IT LI LU MC NL PT SE**

(30) Priority: **21.05.1997 JP 13141397**
**29.05.1997 JP 14064397**

(43) Date of publication of application:
**19.07.2000 Bulletin 2000/29**

(73) Proprietor: **DAIICHI PHARMACEUTICAL CO., LTD.**
**Chuo-ku, Tokyo 103-0027 (JP)**

(72) Inventors:
- **TAKEMURA, Makoto, Daiichi Pharmaceutical Co., Ltd Edogawa-ku, Tokyo 134-0081 (JP)**
- **TAKAHASHI, Hisashi, Daiichi Pharmaceutical Co.,Ltd Edogawa-ku, Tokyo 134-0081 (JP)**
- **SUGITA, Kazuyuki, Daiichi Pharmaceutical Co., Ltd Edogawa-ku, Tokyo 134-0081 (JP)**
- **OHKI, Hitoshi, Daiichi Pharmaceutical Co., Ltd Edogawa-ku, Tokyo 134-0081 (JP)**
- **MIYAUCHI, Satoru, Daiichi Pharmaceutical Co., Ltd Edogawa-ku, Tokyo 134-0081 (JP)**
- **MIYAUCHI, Rie, Daiichi Pharmaceutical Co., Ltd Edogawa-ku, Tokyo 134-0081 (JP)**

(74) Representative: **Kinzebach, Werner, Dr. et al**
**Patentanwälte**
**Reitstötter, Kinzebach und Partner**
**Postfach 86 06 49**
**81633 München (DE)**

(56) References cited:
**EP-A- 0 347 851**
**WO-A-95/10519**
**WO-A-97/19072**
**JP-A- 8 277 284**
**JP-A- 62 234 082**
**US-A- 5 157 128**
**US-A- 5 580 872**
**US-A- 5 599 816**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 020 459 B1

## Description

TECHNICAL FIELD :

This invention.relates to antimicrobial compounds which are useful as drugs, veterinary drugs, fishery drugs marine drugs and antimicrobial preservatives and antimicrobial agents and antimicrobial preparations containing these compounds.

BACKGROUND ART:

Since norfloxacin was found out, attempts have been made to improve the antimicrobial activities and pharmacokinetics of synthetic quinolone antimicrobial agents. As a result, a number of compounds are clinically available today as chemotherapeutic drugs efficacious against systemic infectious diseases.

In recent years, however, bacteria hyposensitive to these drugs have been increasing in the field of clinical medicine. Moreover, some bacteria tolerant to drugs other than these synthetic quinolone antimicrobial agents become hyposensitive thereto, for example, *Staphylococcus aureus* (MRSA) insensitive to β-lactam antibiotics. Therefore, it has been urgently required to develop highly efficacious drugs in the field of clinical medicine.

In addition, it is reported that some antimicrobial agents sometimes induce convulsion when administered together with nonsteroidal anti-inflammatory drugs or they have some side effects such as phototoxicity. Thus, it is needed to develop quinolones with elevated safety.

US-A-5,580,872 describes 4-oxo-4-H-quinolizine-3-carboxylic acid compounds which may be substituted at the 7-position by a pyrrolidinyl group, i.e. compounds of the formula

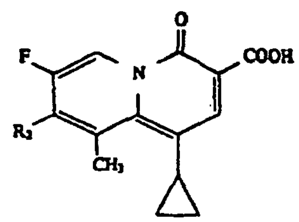

wherein $R^2$ may represent a radical of the formula

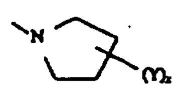

The pyrrolidinyl group, in turn, may be substituted with an amino group (Y is $-NR^{11}R^{12}$) or an amino alkyl group (Y is $-C(R^{21})(R^{22})NH_2$). It is said that $R^{21}$ and $R^{22}$ taken together with the carbon atom to which they are attached may form a cycloalkyl group having 3 to 6 carbon atoms. Such compounds include those of the above formula wherein $R^2$ represents a radical of the formula

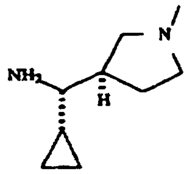 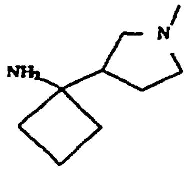 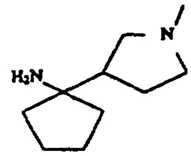 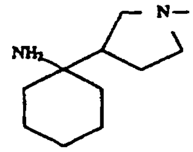

EP-A 0 347 851 describes 4-oxo-3-quinoline carboxylic acid compounds which are substituted at the 7-position by a pyrrolidinyl group, i.e., compounds of the formula

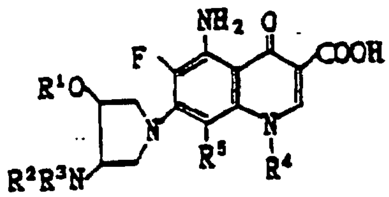

In these quinoline carboxylic acids the pyrrolidinyl group has an optionally $C_1$-$C_4$-alkyl-substituted amino group and a $C_1$-$C_4$-alkoxy group.

US-A-5,157,128 describes 4-oxo-1,8-naphthyridin-3-carboxylic acid and 4-oxo-3-quinoline carboxylic acid compounds which are substituted at the 7-position by a pyrrolidinyl group. This pyrrolidinyl group, in turn, is substituted by an aminoalkyl group, such as the 1-amino-1-methylethyl group of the compound of the formula

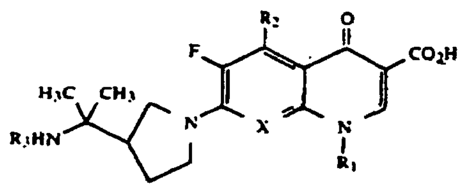

EP-A-0 911 328 (WO 97/ 19072) relates to quinolone derivatives wherein the pyrrolidinyl group at the 7-position of the quinolone skeleton is substituted by a 1-aminocycloalk-1-yl group of the formula

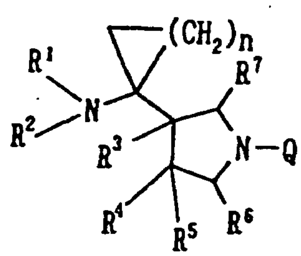

This pyrrolidinyl group may have further substituents. For instance, $R^4$ and $R^5$ may represent a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, wherein the alkyl group may have at least one substituent selected from the group consisting of a hydroxyl group, a halogen atom and an alkoxyl group having 1 to 6 carbon atoms. The compounds described in EP-A-0 911 328 include those wherein the 1-aminocycloalk-1-yl moiety and the substituent $R^4$ are located in a cis-configuration, such as 5-amino-7-[cis-4-(1-aminocyclopropyl)-3-hydroxymethyl-1-pyrrolidinyl]-1-cyclopropyl-6-fluoro-1,4-dihydro-8-methyl-4-oxoquinoline-3-carboxylic acid; 5-amino-7-[4-(R)-(1-aminocyclopropyl)-3-(S)-fluoro-1-pyrrolidinyl]-6-fluoro-1-[2-(S)-fluoro-1-(R)-cyclopropyl]-1,4-dihydro-8-methyl-4-oxoquinoline-3-carboxylic acid hydrochloride; 5-amino-7-[4-(R)-(1-aminocyclopropyl)-3-(S)-fluoro-1-pyrrolidinyl]-6-fluoro-1-[2-(S)-fluoro-1-(R)-cyclopropyl)-1,4-dihydro-8-methoxy-4-oxoquinoline-3-carboxylic acid; 10-[4-(R)-(1-aminocyclopropyl)-3-(S)-fluoro-1-pyrrolidinyl)-9-fluoro-2,3-dihydro-3-(S)-methyl-7-oxo-7H-pyrido[1.2.3-de][1.4]benzoxazin-6-carboxylic acid; 7-[4-(R)-(1-aminocyclopropyl)-3-(S)-fluoro-1-pyrrolidinyl]-6-fluoro-1-[2-(S)-fluoro-1-(R)-cyclopropyl]-1,4-dihydro-8-methoxy-4-oxoquinoline-3-carboxylic acid; 5-amino-7-[cis-4-(1-aminocyclobutyl)-3-fluoro-1-pyrrolidinyl)-6-fluoro-1-[2-(S)-fluoro-1-(R)-cyclopropyl]-1,4-dihydro-8-methyl-4-oxoquinoline-3-carboxylic acid.

DISCLOSURE OF INVENTION:

Under these circumstances, the present inventors have conducted extensive studies to provide compounds satisfying the above-mentioned requirements. As a result, they have successfully found out that cis-substituted aminocycloalkylpyrrolidine derivatives represented by the following formula (I), their salts and hydrates thereof have broad antibacterial spectra, in particular, potent antimicrobial activities on quinolone-tolerant bacteria including gram positive bacteria, in particular, MRSA and, at the same time, favorable pharmacokinetics and high safety, thus completing the present invention.

Accordingly, the present invention relates to compounds represented by formula (I), its salts and hydrates thereof:

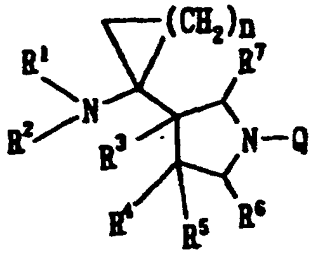

(I)

wherein

$R^1$ represents a hydrogen atom or an alkyl group having 1 to 6 carbon atoms;

$R^2$ represents a hydrogen atom or an alkyl group having 1 to 6 carbon atoms, provided that said alkyl group may have one or more substituents selected from the group consisting of a hydroxyl group, a halogen atom and an alkoxyl group having 1 to 6 carbon atoms;

$R^3$ and $R^5$, each represents a hydrogen atom;

$R^4$ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, provided that said alkyl group may have one or more substituents selected from the group consisting of a hydroxyl group, a halogen atom and an alkoxyl group having 1 to 6 carbon atoms and $R^4$ and the substituent on the pyrrolidine ring of the following formula:

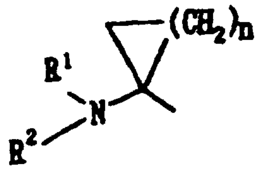

are located in the cis-configuration;

$R^6$ and $R^7$, each independently represents a hydrogen atom or an alkyl group having 1 to 6 carbon atoms;

n is an integer of from 1 to 3; and

Q represents a partial structure represented by the following formula:

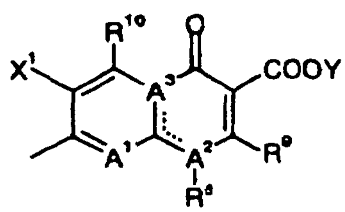

wherein $R^8$ represents an alkyl group having 1 to 6 carbon atoms, an alkenyl group having 2 to 6 carbon atoms, a halogenoalkyl group having 1 to 6 carbon atoms, a substituted or unsubstituted cyclic alkyl group having 3 to 6 carbon atoms, a substituted or unsubstituted aryl group, a substituted or unsubstituted heteroaryl group, an alkoxyl group having 1 to 6 carbon atoms or an alkylamino group having 1 to 6 carbon atoms;

$R^9$ represents a hydrogen atom or an alkylthio group having 1 to 6 carbon atoms;

$R^9$ and $R^8$ may form together with a part of the mother nucleus a cyclic structure optionally containing a sulfur atom as a constituting atom thereof and optionally having an alkyl group having 1 to 6 carbon atoms as a substituent;

$R^{10}$ represents a hydrogen atom, an amino group, a hydroxyl group, a thiol group, a halogenomethyl group, an alkyl group having 1 to 6 carbon atoms, an alkenyl group having 2 to 6 carbon atoms, an alkynyl group having 2 to 6

carbon atoms or an alkoxyl group having 1 to 6 carbon atoms, provided that said amino group may have one or more substituents selected from the group consisting of a formyl group, an alkyl group having 1 to 6 carbon atoms and an acyl group having 2 to 5 carbon atoms;

$X^1$ represents a halogen atom or a hydrogen atom;

$A^1$ represents a nitrogen atom or a partial structure represented by the following formula (II):

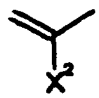

**(II)**

wherein $X^2$ represents a hydrogen atom, an amino group, a halogen atom, a cyano group, a halogenomethyl group, a halogenomethoxyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms, provided that said amino group may have one or more substituents selected from the group consisting or a formyl group, an alkyl group having 1 to 6 carbon atoms and an acyl group having 2 to 5 carbon atoms, and $X^2$ and $R^8$ may form together with a part of the mother nucleus a cyclic structure optionally containing an oxygen atom, a nitrogen atom or a sulfur atom as a constituting atom thereof and optionally having an alkyl group having 1 to 6 carbon atoms as a substituent;

$A^2$ and $A^3$, each represents a nitrogen atom or a carbon atom, provided that $A^2$ and $A^3$ may form together with the carbon atom to which they are bonded a partial structure represented by the following formula:

$$>C=C(A^1=)-N(R^8)-$$

or a partial structure represented by the following formula:

$$>N-C(A^1=)=C(R^8)-;$$

and

Y represents a hydrogen atom, a phenyl group, an acetoxymethyl group, a pivaloyloxymethyl group, an ethoxycarbonyl group, a choline group, a dimethylaminoethyl group, a 5-indanyl group, a phthalidinyl group, a 5-alkyl-2-oxo-1,3-dioxol-4-ylmethyl group, a 3-acetoxy-2-oxobutyl group, an alkyl group having 1 to 6 carbon atoms, an alkoxylmethyl group having 2 to 7 carbon atoms or a phenylalkyl group composed of an alkyl group having 1 to 6 carbon atoms and a phenyl group, provided that the following compounds are excluded:

5-amino-7-[cis-4-(1-aminocyclopropyl)-3-hydroxymethyl-1-pyrrolidinyl]-1-cyclopropyl-6-fluoro-1,4-dihydro-8-methyl-4-oxoquinoline-3-carboxylic acid;
5-amino-7-[4-(R)-(1-aminocyclopropyl)-3-(S)-fluoro-1-pyrrolidinyl]-6-fluoro-1-[2-(S)-fluoro-1-(R)-cyclopropyl]-1,4-dihydro-8-methyl-4-oxoquinoline-3-carboxylic acid hydrochloride;
5-amino-7-[4-(R)-(1-aminocyclopropyl)-3-(S)-fluoro-1-pyrrolidinyl]-6-fluoro-1-[2-(S)-fluoro-1-(R)-cyclopropyl]-1,4-dihydro-8-methoxy-4-oxoquinoline-3-carboxylic acid;
10-[4-(R)-(1-aminocyclopropyl)-3-(S)-fluoro-1-pyrrolidinyl]-9-fluoro-2,3-dihydro-3-(S)-methyl-7-oxo-7H-pyrido[1.2.3-de] [1.4]benzoxazin-6-carboxylic acid;
7-[4-(R)-(1-aminocyclopropyl)-3-(S)-fluoro-1-pyrrolidinyl]-6-fluoro-1-[2-(S)-fluoro-1-(R)-cyclopropyl)-1,4-dihydro-8-methoxy-4-oxoquinoline-3-carboxylic acid;
5-amino-7-[cis-4-(1-aminocyclobutyl)-3-fluoro-1-pyrrolidinyl]-6-fluoro-1-[2-(S)-fluoro-1-(R)-cyclopropyl]-1,4-dihydro-8-methyl-4-oxoquinoline-3-carboxylic acid.

According to a particular embodiment, the present invention relates to compounds represented by formula (Ia), its salts and hydrates thereof:

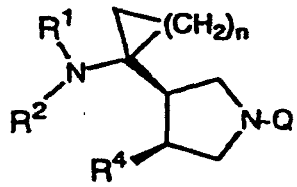

wherein $R^1$, $R^2$, $R^4$, n and Q are as defined above.

Further, the present invention relates to compounds represented by formula (I), its salts and hydrates thereof:

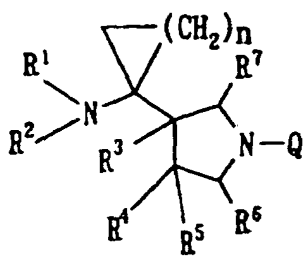

(I)

wherein

$R^1$ represents a hydrogen atom or an alkyl group having 1 to 6 carbon atoms;

$R^2$ represents a hydrogen atom or an alkyl group having 1 to 6 carbon atoms, provided that said alkyl group may have one or more substituents selected from the group consisting of a hydroxyl group, a halogen atom and an alkoxyl group having 1 to 6 carbon atoms;

$R^3$ and $R^5$, each represents a hydrogen atom;

$R^4$ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, provided that said alkyl group may have one or more substituents selected from the group consisting of a hydroxyl group, a halogen atom and an alkoxyl group having 1 to 6 carbon atoms and $R^4$ and the substituent on the pyrrolidine ring of the following formula:

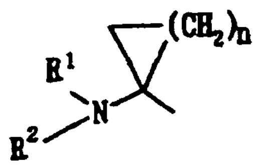

are located in the cis-configuration;

$R^6$ and $R^7$, each independently represents a hydrogen atom or an alkyl group having 1 to 6 carbon atoms;

n is an integer of from 1 to 3; and

Q represents a partial structure represented by the following formula:

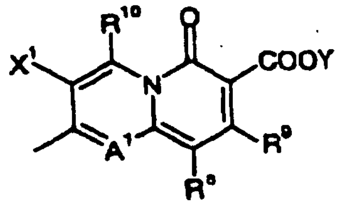

wherein $R^8$ represents an alkyl group having 1 to 6 carbon atoms, an alkenyl group having 2 to 6 carbon atoms, a halogenoalkyl group having 1 to 6 carbon atoms, a substituted or unsubstituted cyclic alkyl group having 3 to 6 carbon atoms, a substituted or unsubstituted aryl group, a substituted or unsubstituted heteroaryl group, an alkoxyl group having 1 to 6 carbon atoms or an alkylamino group having 1 to 6 carbon atoms;

$R^9$ represents a hydrogen atom or an alkylthio group having 1 to 6 carbon atoms;

$R^9$ and $R^8$ may form together with a part of the mother nucleus a cyclic structure optionally containing a sulfur atom as a constituting atom thereof and optionally having an alkyl group having 1 to 6 carbon atoms as a substituent;

$R^{10}$ represents a hydrogen atom, an amino group, a hydroxyl group, a thiol group, a halogenomethyl group , an alkyl group having 1 to 6 carbon atoms, an alkenyl group having 2 to 6 carbon atoms, an alkynyl group having 2 to 6 carbon atoms or an alkoxyl group having 1 to 6 carbon atoms, provided that said amino group may have one or more substituents selected from the group consisting of a formyl group, an alkyl group having 1 to 6 carbon atoms and an acyl group having 2 to 5 carbon atoms;

$X^1$ represents a halogen atom or a hydrogen atom;

$A^1$ represents a nitrogen atom or a partial structure represented by the following formula (II):

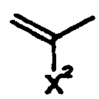

(II)

wherein $X^2$ represents a hydrogen atom, an amino group, a halogen atom, a cyano group, a halogenomethyl group, a halogenomethoxyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms, provided that said amino group may have one or more substituents selected from the group consisting of a formyl group, an alkyl group having 1 to 6 carbon atoms and an acyl group having 2 to 5 carbon atoms, and $X^2$ and $R^8$ may form together with a part of the mother nucleus a cyclic structure optionally containing an oxygen atom, a nitrogen atom or a sulfur atom as a constituting atom thereof and optionally having an alkyl group having 1 to 6 carbon atoms as a substituent;

Y represents a hydrogen atom, a phenyl group, an acetoxymethyl group, a pivaloyloxymethyl group, an ethoxycarbonyl group, a choline group, a dimethylaminoethyl group, a 5-indanyl group, a phthalidinyl group, a 5-alkyl-2-oxo-1,3-dioxol-4-ylmethyl group, a 3-acetoxy-2-oxobutyl group, an alkyl group having 1 to 6 carbon atoms, an alkoxylmethyl group having 2 to 7 carbon atoms or a phenylalkyl group composed of an alkyl group having 1 to 6 carbon atoms and a phenyl group.

Further, the present invention relates to compounds represented by formula (I), its salts and hydrates thereof:

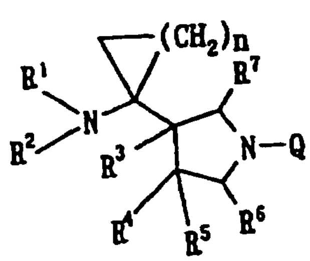

(I)

wherein

$R^1$ represents a hydrogen atom or an alkyl group having 1 to 6 carbon atoms;

$R^2$ represents a hydrogen atom or an alkyl group having 1 to 6 carbon atoms, provided that said alkyl group may have one or more substituents selected from the group consisting of a hydroxyl group, a halogen atom and an alkoxyl group having 1 to 6 carbon atoms;

$R^3$ and $R^5$, each represents a hydrogen atom;

$R^4$ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, provided that said alkyl group may have one or more substituents selected from the group consisting of a hydroxyl group, a halogen atom and an alkoxyl group having 1 to 6 carbon atoms and $R^4$ and the substituent on the pyrrolidine ring of the following formula:

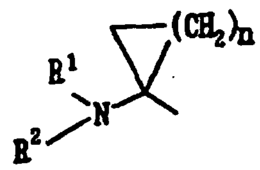

are located in the cis-configuration;

$R^6$ and $R^7$, each independently represents a hydrogen atom or an alkyl group having 1 to 6 carbon atoms;

n is an integer of from 1 to 3; and

Q represents a partial structure represented by the following formula:

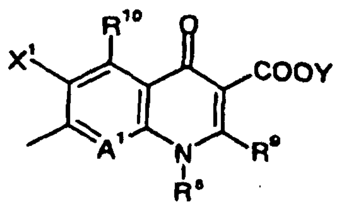

wherein $R^8$ represents an alkyl group having 1 to 6 carbon atoms, an alkenyl group having 2 to 6 carbon atoms, a halogenoalkyl group having 1 to 6 carbon atoms, a substituted or unsubstituted cyclic alkyl group having 3 to 6 carbon atoms, a substituted or unsubstituted aryl group, a substituted or unsubstituted heteroaryl group, an alkoxyl group having 1 to 6 carbon atoms or an alkylamino group having 1 to 6 carbon atoms;

$R^9$ represents a hydrogen atom or an alkylthio group having 1 to 6 carbon atoms;

$R^9$ and $R^8$ may form together with a part of the mother nucleus a cyclic structure optionally containing a sulfur atom as a constituting atom thereof and optionally having an alkyl group having 1 to 6 carbon atoms as a substituent;

$R^{10}$ represents an amino group having one or more substituents selected from the group consisting of a formyl group, an alkyl group having 1 to 6 carbon atoms and an acyl group having 2 to 5 carbon atoms, a hydroxyl group, a thiol group, a halogenomethyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms;

$X^1$ represents a halogen atom or a hydrogen atom;

$A^1$ represents a nitrogen atom or a partial structure represented by the following formula (II):

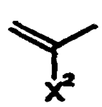

(II)

wherein $X^2$ represents a hydrogen atom, an amino group, a halogen atom, a cyano group, a halogenomethyl

group, a halogenomethoxyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms, provided that said amino group may have one or more substituents selected from the group consisting or a formyl group, an alkyl group having 1 to 6 carbon atoms and an acyl group having 2 to 5 carbon atoms;

Y represents a hydrogen atom, a phenyl group, an acetoxymethyl group, a pivaloyloxymethyl group, an ethoxycarbonyl group, a choline group, a dimethylaminoethyl group, a 5-indanyl group, a phthalidinyl group, a 5-alkyl-2-oxo-1,3-dioxol-4-ylmethyl group, a 3-acetoxy-2-oxobutyl group, an alkyl group having 1 to 6 carbon atoms, an alkoxylmethyl group having 2 to 7 carbon atoms or a phenylalkyl group composed of an alkyl group having 1 to 6 carbon atoms and a phenyl group.

Further, the present invention relates to compounds represented by formula (I), its salts and hydrates thereof:

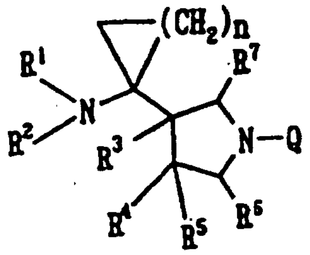

(I)

wherein

$R^1$ represents a hydrogen atom or an alkyl group having 1 to 6 carbon atoms;

$R^2$ represents a hydrogen atom or an alkyl group having 1 to 6 carbon atoms, provided that said alkyl group may have one or more substituents selected from the group consisting of a hydroxyl group, a halogen atom and an alkoxyl group having 1 to 6 carbon atoms;

$R^3$ and $R^5$, each represents a hydrogen atom;

$R^4$ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, provided that said alkyl group may have one or more substituents selected from the group consisting of a hydroxyl group, a halogen atom and an alkoxyl group having 1 to 6 carbon atoms or an alkylthio group having 1 to 6 carbon atoms, provided that said alkyl group may have one or more substituents selected from the group consisting of a hydroxyl group, a halogen atom and an alkoxyl group having 1 to 6 carbon atoms and $R^4$ and the substituent on the pyrrolidine ring of the following formula:

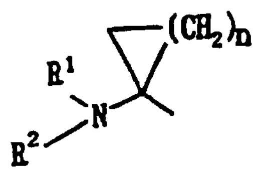

are located in the cis-configuration;

$R^6$ and $R^7$, each independently represents a hydrogen atom or an alkyl group having 1 to 6 carbon atoms;

n is an integer of from 1 to 3; and

Q represents a partial structure represented by the following formula:

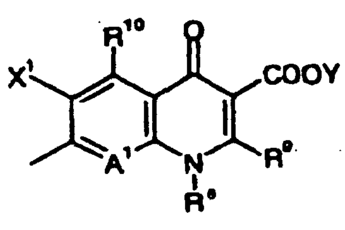

wherein $R^8$ represents an alkyl group having 1 to 6 carbon atoms, an alkenyl group having 2 to 6 carbon atoms, a halogenoalkyl group having 1 to 6 carbon atoms, a substituted or unsubstituted cyclic alkyl group having 3 to 6 carbon atoms, a substituted or unsubstituted aryl group, a substituted or unsubstituted heteroaryl group, an alkoxyl group having 1 to 6 carbon atoms or an alkylamino group having 1 to 6 carbon atoms;

$R^9$ represents a hydrogen atom or an alkylthio group having 1 to 6 carbon atoms;

$R^9$ and $R^8$ may form together with a part of the mother nucleus a cyclic structure optionally containing a sulfur atom as a constituting atom thereof and optionally having an alkyl group having 1 to 6 carbon atoms as a substituent;

$R^{10}$ represents a hydrogen atom, an amino group, a hydroxyl group, a thiol group, a halogenomethyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms, provided that said amino group may have one or more substituents selected from the group consisting of a formyl group, an alkyl group having 1 to 6 carbon atoms and an acyl group having 2 to 5 carbon atoms;

$X^1$ represents a halogen atom or a hydrogen atom;

$A^1$ represents a nitrogen atom or a partial structure represented by the following formula (II):

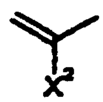

(II)

wherein $X^2$ represents a hydrogen atom, an amino group, a halogen atom, a cyano group, a halogenomethyl group, a halogenomethoxyl group, an alkenyl group having 2 to 6 carbon atoms or an alkynyl group having 2 to 6 carbon atoms, provided that said amino group may have one or more substituents selected from the group consisting or a formyl group, an alkyl group having 1 to 6 carbon atoms and an acyl group having 2 to 5 carbon atoms; and

Y represents a hydrogen atom, a phenyl group, an acetoxymethyl group, a pivaloyloxymethyl group, an ethoxycarbonyl group, a choline group, a dimethylaminoethyl group, a 5-indanyl group, a phthalidinyl group, a 5-alkyl-2-oxo-1,3-dioxol-4-ylmethyl group, a 3-acetoxy-2-oxobutyl group, an alkyl group having 1 to 6 carbon atoms, an alkoxylmethyl group having 2 to 7 carbon atoms or a phenylalkyl group composed of an alkyl group having 1 to 6 carbon atoms and a phenyl group.

Further, the present invention relates to compounds represented by formula (I), its salts and hydrates thereof:

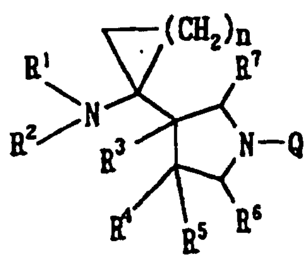

(I)

wherein

$R^1$ represents a hydrogen atom or an alkyl group having 1 to 6 carbon atoms;

$R^2$ represents a hydrogen atom or an alkyl group having 1 to 6 carbon atoms, provided that said alkyl group may have one or more substituents selected from the group consisting of a hydroxyl group, a halogen atom and an alkoxyl group having 1 to 6 carbon atoms;

$R^3$ and $R^5$, each represents a hydrogen atom;

$R^4$ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, provided that said alkyl group may have one or more substituents selected from the group consisting of a hydroxyl group, a halogen atom and an alkoxyl group having 1 to 6 carbon atoms and $R^4$ and the substituent on the pyrrolidine ring of the following formula:

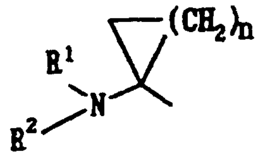

are located in the cis-configuration;

$R^6$ and $R^7$, each independently represents a hydrogen atom or an alkyl group having 1 to 6 carbon atoms;

n is an integer of from 1 to 3; and

Q represents a partial structure represented by the following formula:

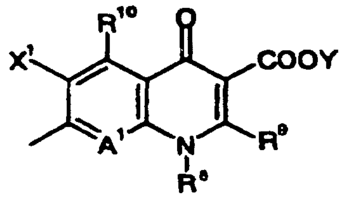

wherein $R^9$ represents a hydrogen atom or an alkylthio group having 1 to 6 carbon atoms;

$R^{10}$ represents an amino group, a hydroxyl group, a thiol group, a halogenomethyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms, provided that said amino group may have one or more substituents selected from the group consisting of a formyl group, an alkyl group having 1 to 6 carbon atoms and an acyl group having 2 to 5 carbon atoms;

$X^1$ represents a halogen atom or a hydrogen atom;

$A^1$ represents a partial structure represented by the following formula (II):

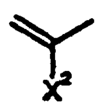

(II)

wherein $X^2$ and $R^8$ form together with a part of the mother nucleus a cyclic structure optionally containing an oxygen atom, a nitrogen atom or a sulfur atom as a constituting atom thereof and optionally having an alkyl group having 1 to 6 carbon atoms as a substituent; and

Y represents a hydrogen atom, a phenyl group, an acetoxymethyl group, a pivaloyloxymethyl group, an ethoxycarbonyl group, a choline group, a dimethylaminoethyl group, a 5-indanyl group, a phthalidinyl group, a 5-alkyl-2-oxo-1,3-dioxol-4-ylmethyl group, a 3-acetoxy-2-oxobutyl group, an alkyl group having 1 to 6 carbon atoms, an alkoxylmethyl group having 2 to 7 carbon atoms or a phenylalkyl group composed of an alkyl group having 1 to 6 carbon atoms and a phenyl group.

The present invention further provides:

the compounds of the formula (I), wherein Q has a structure represented by the following formula, its salts and hydrates thereof:

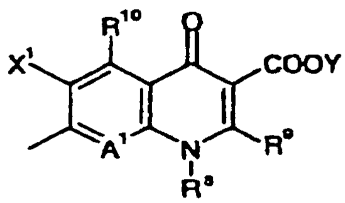

or the following formula:

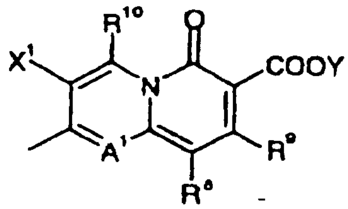

wherein $R^8$, $R^9$, $R^{10}$, $A^1$, $X^1$ and Y are each as defined above;
the compounds of the formula (I), wherein Q has a structure represented by the following formula, its salts and hydrates thereof:

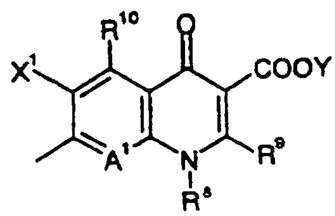

wherein $R^8$, $R^9$, $R^{10}$, $A^1$, $X^1$ and Y are each as defined above;
the compounds of the formula (I), wherein Q is a 6-carboxy-9-fluoro-2,3-dihydro-3-(S)-methyl-7-oxo-7H-pyrido [1,2,3-de][1,4]benzoxazin-10-yl group (following formula), its salts and hydrates thereof:

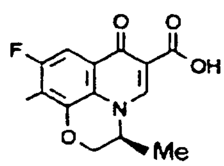

;

the compound of the formula (I), wherein Q is an 8-amino-6-carboxy-9-fluoro-2,3-dihydro-3-(S)-methyl-7-oxo-7H-pyrido[1,2,3-de][1,4]benzoxazin-10-yl group (following formula), its salts and hydrates thereof:

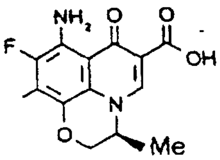

;

the compounds of the formula (I), wherein Q is a 5-amino-3-carboxy-6-fluoro-1-[2-(S)-fluoro-1-(R)-cyclopropyl]-1,4-dihydro-8-methoxy-4-oxoquinolin-7-yl group (following formula), its salts and hydrates thereof:

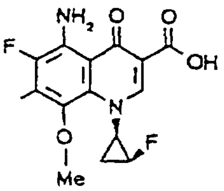

;

the compounds of the formula (I), wherein Q is a 5-amino-3-carboxy-6-fluoro-1-[2-(S)-fluoro-1-(R)-cyclopropyl]-1,4-dihydro-8-methyl-4-oxoquinolin-7-yl group (following formula), its salts and hydrates thereof:

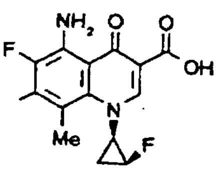

;

the compounds of the formula (I), wherein Q is a 3-carboxy-6-fluoro-1-[2-(S)-fluoro-1-(R)-cyclopropyl]-1,4-dihydro-8-methoxy-4-oxoquinolin-7-yl group (following formula), its salts and hydrates thereof:

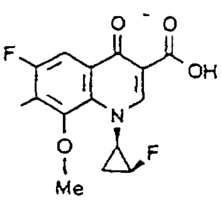

;

the compound of the formula (I), wherein the substituent $R^4$ is a halogen atom, its salts and hydrates thereof;
the compounds of the formula (I), wherein the substituent $R^4$ is a fluorine atom, its salts and hydrates thereof;
the compounds of the formula (I), wherein n is 1 or 2, its salts and hydrates thereof;
the compounds of the formula (I), wherein n is 1, its salts and hydrates thereof;
the compounds of the formula (I), wherein the substituent $R^4$ is a fluorine atom and n is 1 or 2, its salts and hydrates thereof;
the compounds of the formula (I), wherein the substituent $R^4$ is a fluorine atom and n is 1, its salts and hydrates thereof;
the compounds of the formula (I), wherein the substituent $R^8$ is a halogenocyclopropyl group, its salts and hydrates

thereof;
the compounds of the formula (I), wherein the substituent $R^8$ is a 1,2-cis-2-halogenocyclopropyl group, its salts and hydrates thereof;
the compounds of the formula (I), wherein the substituent $R^8$ is a stereochemically pure one, its salts and hydrates thereof;
the compounds of the formula (I), wherein the substituent $R^8$ is a (1R,2S)-2-halogenocyclopropyl group, its salts and hydrates thereof;
the compounds of the formula (I), wherein the substituent $R^8$ is a (1R,2S)-2-fluorocyclopropyl group, its salts and hydrates thereof;
the compounds represented by the formula (I), which are stereochemically pure ones, its salts and hydrates thereof;
the compounds of the formula (I), wherein the substituent $X^1$ is a halogen atom, its salts and hydrates thereof;
the compounds of the formula (I), wherein $X^1$ is a fluorine atom, its salts and hydrates thereof;
8-amino-10-[4-(R)-(1-aminocyclopropyl)-3-(S)-fluoro-1-pyrrolidinyl]-9-fluoro-2,3-dihydro-3-(S)-methyl-7-oxo-1H-pyrido[1,2,3-de][1,4]benzoxazine-6-carboxylic acid, its salts and hydrates thereof;
drugs containing as the active ingredient the above-mentioned compounds, hydrates thereof, salts of the compounds or hydrates of the salts.
antimicrobial agents containing as the active ingredient the above-mentioned compounds, hydrates thereof, salts of the compounds or hydrates of the salts; etc.

The present invention further relates to compounds represented by the following formula (VI), its salts and hydrates thereof:

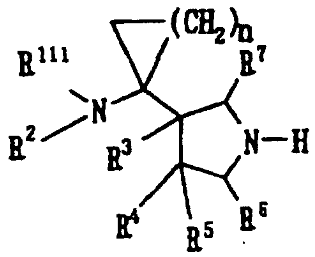

(VI)

wherein $R^{111}$ represents a hydrogen atom, an alkyl group having 1 to 6 carbon atoms or a protective group of the amino group;

$R^2$ represents a hydrogen atom or an alkyl group having 1 to 6 carbon atoms, provided that said alkyl group may have one or more substituents selected from the group consisting of a hydroxyl group, a halogen atom and an alkoxyl group having 1 to 6 carbon atoms;

$R^3$ and $R^5$, each represents a hydrogen atom;

$R^4$ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, provided that said alkyl group may have one or more substituents selected from the group consisting of a hydroxyl group, a halogen atom and an alkoxyl group having 1 to 6 carbon atoms and $R^4$ and the substituent on the pyrrolidine ring of the following formula:

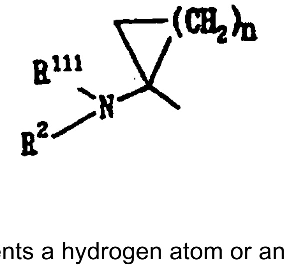

are located in the cis-configuration;

$R^6$ and $R^7$, each independently represents a hydrogen atom or an alkyl group having 1 to 6 carbon atoms;

n is an integer of from 1 to 3;

provided that the following compounds and addition salts thereof are excluded:

cis-4-( 1-aminocyclopropyl)-3-(hydroxymethylpyrrolidine);
4-(R)-(1-aminocyclopropyl)-3-(S)-fluoropyrrolidine;
cis-4-(1-aminocyclobutyl)-3-fluoropyrrolidine;

in which compounds the amino group may be protected.

The present invention further relates to compounds represented by the following formula (VI)

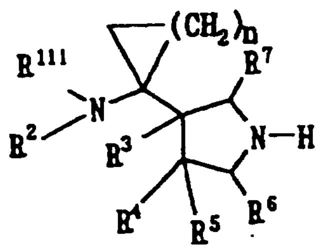

(VI)

wherein $R^{111}$ represents a hydrogen atom, an alkyl group having 1 to 6 carbon atoms or a protective group of the amino group;

R2 represents a hydrogen atom or an alkyl group having 1 to 6 carbon atoms, provided that said alkyl group may have one or more substituents selected from the group consisting of a hydroxyl group, a halogen atom and an alkoxyl group having 1 to 6 carbon atoms;

$R^3$ and $R^5$, each represents a hydrogen atom;

$R^4$ represents a hydroxyl group, a chlorine atom, a carbamoyl group, an alkyl group having 1 to 6 carbon atoms, an alkoxyl group having 1 to 6 carbon atoms, 2-hydroxyethyl, 3-hydroxypropyl or an alkylthio group having 1 to 6 carbon atoms, provided that said alkyl group may have one or more substituents selected from the group consisting of a halogen atom and an alkoxyl group having 1 to 6 carbon atoms and $R^4$ and the substituent on the pyrrolidine ring of the following formula:

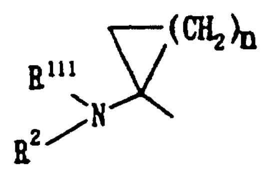

are located in the cis-configuration;

$R^6$ and $R^7$, each independently represents a hydrogen atom or an alkyl group having 1 to 6 carbon atoms;

n is an integer of from 1 to 3.

The present invention further relates to compounds represented by the following formula (VII):

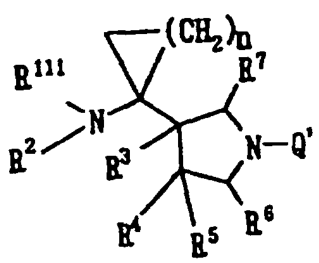

(VII)

wherein $R^{111}$ represents a hydrogen atom, an alkyl group having 1 to 6 carbon atoms or a protective group of the amino group;

$R^2$ represents a hydrogen atom or an alkyl group having 1 to 6 carbon atoms, provided that said alkyl group may have one or more substituents selected from the group consisting of a hydroxyl group, a halogen atom and an alkoxyl group having 1 to 6 carbon atoms;

$R^3$ and $R^5$, each represents a hydrogen atom;

$R^4$ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, provided that said alkyl group may have one or more substituents selected from the group consisting of a hydroxyl group, a halogen atom and an alkoxyl group having 1 to 6 carbon atoms; and $R^4$ and the substituent on the pyrrolidine ring of the following formula:

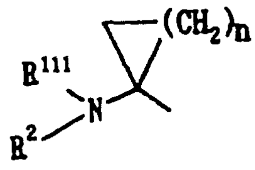

are located in the cis-configuration;

$R^6$ and $R^7$, each independently represents a hydrogen atom or an alkyl group having 1 to 6 carbon atoms;

n is an integer of from 1 to 3; and

Q' represents a protective group for the amino group selected from the group consisting of optionally substituted alkoxylcarbonyl groups, optionally substituted aralkyloxycarbonyl groups, optionally substituted acyl groups, optionally substituted alkyl groups, optionally substituted aralkyl groups and substituted silyl groups,

provided that the following compounds are excluded:

1-benzyloxycarbonyl-4-(R)-( 1-tert-butoxycarbonylamino-cyclopropyl)-3-(S)-fluoropyrrolidine;
cis-1-[ 1-(S)-phenylethyl]-4-(1-tert-butoxycarbonylamino-cyclobutyl)-3-fluoropyrrolidine.

The present invention further relates to compounds represented by the following formula (VII):

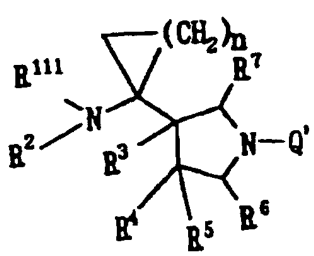

(VII)

wherein $R^{111}$ represents a hydrogen atom, an alkyl group having 1 to 6 carbon atoms or a protective group of the amino group;

$R^2$ represents a hydrogen atom or an alkyl group having 1 to 6 carbon atoms, provided that the alkyl group may have one or more substituents selected from the group consisting of a hydroxyl group, a halogen atom and an alkoxyl group having 1 to 6 carbon atoms;

$R^3$ and $R^5$, each represents a hydrogen atom;

$R^4$ represents a hydroxyl group, a chlorine atom, a carbamoyl group, an alkyl group having 1 to 6 carbon atoms, an alkoxyl group having 1 to 6 carbon atoms or an alkylthio group having 1 to 6 carbon atoms, provided that the alkyl group may have one or more substituents selected from the group consisting of a hydroxyl group, a halogen atom and an alkoxyl group having 1 to 6 carbon atoms; and

$R^4$ and the substituent on the pyrrolidine ring of the following formula:

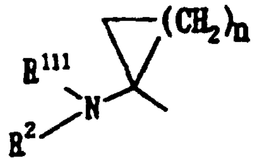

are located in the cis-configuration;

$R^6$ and $R^7$, each independently represents a hydrogen atom or an alkyl group having 1 to 6 carbon atoms;

n is an integer of from 1 to 3;

Q' represents a protective group for the amino group selected from the group consisting of optionally substituted alkoxylcarbonyl groups, optionally substituted aralkyloxycarbonyl groups, optionally substituted acyl groups, optionally substituted alkyl groups, optionally substituted aralkyl groups and substituted silyl groups.

The present invention furthermore relates to the above-mentioned compounds, wherein the substituent $R^4$ is a halogen atom, its salts and hydrates thereof;

the above-mentioned compounds, wherein the substituent $R^4$ is a fluorine atom, their salts and hydrates thereof;

the above-mentioned compounds, wherein n is 1 or 2, its salts and hydrates thereof;

the above-mentioned compounds, wherein n is 1, its salts and hydrates thereof;

the above-mentioned compounds, wherein the substituent $R^4$ is a fluorine atom and n is 1 or 2, its salts and hydrates thereof;

the above-mentioned compounds, wherein the substituent $R^4$ is a fluorine atom and n is 1, its salts and hydrates thereof;

4-(R)-(1-aminocyclopropyl)-3-(S)-fluoro-1-pyrrolidine, its salts and hydrates thereof; etc.

Now, the substituents in the compounds of the present invention represented by the formula (I) will be explained.

The substituent $R^1$ represents a hydrogen atom or an alkyl group having 1 to 6 carbon atoms. This alkyl group may be either a liner or branched one having 1 to 6 carbon atoms. Preferable examples thereof include methyl, ethyl, n-propyl and isopropyl groups.

$R^2$ represents a hydrogen atom or an alkyl group having 1 to 6 carbon atoms. This alkyl group may have one or more substituents selected from the group consisting of a hydroxyl group, a halogen atom, an alkylthio group having 1 to 6 carbon atoms and an alkoxy group having 1 to 6 carbon atoms

This alkyl group may be either a liner or branched one having 1 to 6 carbon atoms. Preferable examples thereof include methyl, ethyl, n-propyl and isopropyl groups.

When this alkyl group is substituted by a hydroxyl group, the alkyl group may be either a linear or branched one having 1 to 6 carbon atoms and the hydroxyl group is preferably attached to the terminal carbon atom of the alkyl group. Preferable examples of the alkyl group having a hydroxyl group include hydroxymethyl, 2-hydroxyethyl, 2-hydroxypropyl and 3-hydroxypropyl groups.

When this alkyl group is substituted by a halogen atom, the alkyl group may be either a linear or branched one having 1 to 6 carbon atoms and a fluorine atom is preferable as the halogen atom.

When this alkyl group is substituted by an alkylthio group, the alkyl group may be either a linear or branched one having 1 to 6 carbon atoms. Also, the alkylthio group may be either a linear or branched one having 1 to 6 carbon atoms. Examples of the alkyl group having an alkylthio group include alkylthiomethyl, alkykthioethyl and alkylthiopropyl groups. It is preferable that the alkylthio group has up to 3 carbon atoms too. Namely, still preferably examples thereof include methylthiomethyl, ethylthiomethyl and methylthioethyl groups.

When this alkyl group is substituted by an alkoxyl group, the alkyl group may be either a linear or branched one having 1 to 6 carbon atoms. Also, the alkoxyl group may be either a linear or branched one having 1 to 6 carbon atoms. Examples of the alkyl group having an alkoxyl group include alkoxymethyl, alkoxethyl and alkoxypropyl groups. It is preferable that the alkoxyl group has up to 3 carbon atoms too. Namely, still preferably examples thereof include methoxymethyl, ethoxymethyl and methoxyethyl groups.

The substituents $R^3$ and $R^5$, each represents a hydrogen atom. These hydrogen atoms are located in the cis-configuration with regard to the pyrrolidine ring.

The substituent $R^4$ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms. This alkyl group may have one or more substituents selected from the group consisting of a hydroxyl group, a halogen atom and an alkoxy group having 1 to 6 carbon atoms.

Preferable examples of the halogen atom are fluorine and chlorine atoms.

Although the alkyl group may either a linear or branched one having 1 to 6 carbon atoms, preferable examples thereof include methyl, ethyl, n-propyl and isopropyl groups.

Although the alkoxyl group may either a linear or branched one having 1 to 6 carbon atoms, preferable examples thereof include methoxyl and ethoxyl groups.

Although the alkylthio group may either a linear or branched one having 1 to 6 carbon atoms, preferable examples thereof include methylthio and ethylthio groups.

When this alkyl group is substituted by a hydroxyl group, the alkyl group may be either a linear or branched one having 1 to 6 carbon atoms and the hydroxyl group is preferably attached to the terminal carbon atom of the alkyl group. Preferable examples of the hydroxylated alkyl group having 1 to 6 carbon atoms include hydroxymethyl, 2-hydroxyethyl and 3-hydroxypropyl groups.

When this alkyl group is substituted by a halogen atom, preferable examples of the. halogen atom include fluorine and chlorine atoms; and a fluorine atom is still preferable. The alkyl group may be either a linear or branched one having 1 to 6 carbon atoms.

When this alkyl group having 1 to 6 carbon atoms is substituted by an alkoxyl group, each alkyl moiety may be either a linear or branched one having 1 to 6 carbon atoms. Preferable examples thereof include alkoxymethyl or alkoxethyl groups and methoxymethyl, ethoxymethyl and 2-methoxyethyl groups are still preferable therefor.

The substituent $R^4$ and the substituent on the pyrrolidine ring of the following formula:

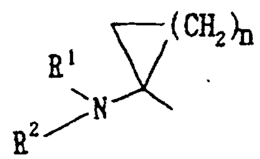

are located in the cis-configuration, which is one of the characteristics of the compounds of the present invention.

The substituents $R^6$ and $R^7$, each independently represents a hydrogen atom or an alkyl group having 1 to 6 carbon atoms. Although the alkyl group may either a linear or branched one having 1 to 6 carbon atoms, preferable examples thereof include methyl, ethyl, n-propyl and isopropyl groups.

n is an integer of from 1 to 3. Namely, the ring may range from a cyclopropane ring to a cyclopentane ring. In the compounds of the present invention, this moiety has a cyclic structure, which is another characteristics of the present invention. It is particularly preferable that n is 1.

Q is a partial structure of a fused heterocycles represented by the following formula:

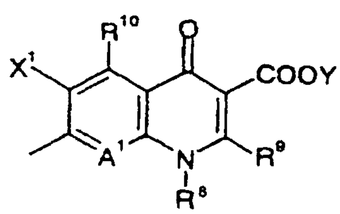

or

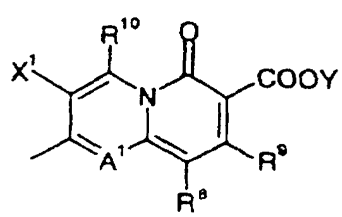

The substituent $R^8$ represents an alkyl group having 1 to 6 carbon atoms, an alkenyl group having 2 to 6 carbon atoms, a halogenoalkyl group having 1 to 6 carbon atoms, a substituted or unsubstituted cyclic alkyl group having 3 to 6 carbon atoms; a substituted or unsubstituted aryl group, a substituted or unsubstituted heteroaryl group, an alkoxyl group having 1 to 6 carbon atoms or an alkylamino group having 1 to 6 carbon atoms.

An ethyl group is particularly preferable as the alkyl group having 1 to 6 carbon atoms. A vinyl or 1-isopropenyl group is preferable as the alkenyl group having 2 to 6 carbon atoms. A 2-fluoroethyl group is preferable as the halogenoalkyl group having 1 to 6 carbon atoms. A cyclopropyl and 2-halogenocyclopropyl groups are preferable as the substituted or unsubstituted cyclic alkyl group having 3 to 6 carbon atoms. As the halogen atom in the 2-halogenocyclopropyl group, a fluorine atom is preferable in particular.

Examples of the substituted or unsubstituted aryl group include a phenyl group, etc. optionally having 1 to 3 substituents selected from the group consisting of a halogen atom (e.g., fluorine, chlorine or bromine), a lower alkyl group having 1 to 6 carbon atoms, a hydroxyl group, an amino group, a nitro group, a lower alkoxyl group having 1 to 6 carbon atoms, etc. Preferable examples thereof include phenyl, 2-fluorophenyl, 4-fluorophenyl, 2,4-difluorophenyl and 2-fluoro-4-hydroxyphenyl groups.

A heteroaryl group is a substituent derived from an aromatic heterocyclic compound containing at least one heteroatom selected from among nitrogen, oxygen and sulfur atoms. Examples thereof include pyridyl and pyrimidyl groups. Preferable examples of the substituents on these rings include an alkyl group and a halogen atom. A methoxyl group is preferable as the alkoxyl group having 1 to 6 carbon atoms, while a methylamino group is preferable as the alkylamino group having 1 to 6 carbon atoms.

Preferable examples-of the substituent $R^8$ include a cyclic alkyl group and a halogenocycloalkyl group. Among these substituents, a cyclopropyl group or a 2-halogenocyclopropyl group is preferable therefor. As the halogen atom, a fluorine atom is preferable.

The substituent $R^9$ represents a hydrogen atom or an alkylthio group having 1 to 6 carbon atoms. Alternatively, $R^9$ and $R^8$ may form together with a part of the mother nucleus (containing $A^2$ to which $R^8$ is bonded and the carbon atom to which $R^9$ is bonded) a cyclic structure. The ring thus formed may contain a sulfur atom as a constituting atom thereof and have an alkyl group having 1 to 6 carbon atoms as a substituent. The ring thus formed is a 4- to 6-membered one which is either saturated, partly saturated or unsaturated.

The substituent $X^1$ represents a halogen atom or a hydrogen atom. When it is a halogen atom, a fluorine atom is preferable therefor. Among all, a fluorine or hydrogen atom is preferable as this substituent.

The substituent $R^{10}$ represents a hydrogen atom, an amino group, a hydroxyl group, a thiol group, a halogenomethyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms. The amino group may have one or more substituents selected from the group consisting of a formyl group, an alkyl group having 1 to 6 carbon atoms and an acyl group having 2 to 5 carbon atoms.

Although the alkyl group may either a linear or branched one having 1 to 6 carbon atoms, preferable examples thereof include methyl, ethyl, n-propyl and isopropyl groups. The alkenyl group is a linear or branched one having 2 to 6 carbon atoms and a vinyl group is preferable therefor. Although the alkynyl group may either a linear or branched one having 2 to 6 carbon atoms, an ethynyl group is preferable therefor. One to three fluorine atoms are particularly preferable as the halogen in the haolenomethyl group. Although the alkoxyl group may be one having 1 to 6 carbon atoms, a methoxymethyl group is preferable therefor.

Preferable examples of the substituent $R^{10}$ include alkyl and amino groups. Among all, a methyl group and an unsubstituted amino group are particularly preferable therefor.

When the substituent $R^{10}$ is an amino, a hydroxyl group or a thiol group, it may be protected by protective groups usually employed in the art.

Examples of such protective groups include alkoxycarbonyl groups (e.g., tert-butoxycarbonyl and 2,2,2-trichloroethoxycarbonyl), aralkyloxycarbonyl groups (e.g., benzyloxcarbonyl, p-methoxybenzyloxycarbonyl and p-nitrobenzyloxycarbonyl), acyl groups (e.g., acetyl, methoxyacetyl, trifluoroacetyl, chloroacetyl, pivaloyl, formyl and benzoyl), alkyl or aralkyl groups (e.g., tert-butyl, benzyl, p-nitrobenzyl, p-methoxybenzyl and triphenylmethyl), ethers (e.g., methoxymethyl, tert-butoxymethyl, tetrahydropyranyl and 2,2,2-trichloroethoxymethyl) and substituted silyl groups (e.g., trimethylsilyl, isopropyldimethylsilyl, tert-butyldimethylsilyl, tribenzylsily, and tert-butyldiphenylsilyl). Compounds carrying substituents protected by these groups are preferable particularly as intermediates in production processes.

When $A^1$ represents a partial structure represented by the following formula:

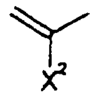

$X^2$ represents a hydrogen atom, an amino group, a halogen atom, a cyano group, a halogenomethyl group, a halogenomethoxy group, an alkyl group having 1 to 6 carbon atoms, an alkenyl group having 2 to 6 carbon atoms, an alkynyl group having 2 to 6 carbon atoms or an alkoxyl group having 1 to 6 carbon atoms. The amino group may have one or more substituents selected from the group consisting of a formyl group, an alkyl group having 1 to 6 carbon atoms and an acyl group having 2 to 5 carbon atoms.

Although the alkyl group may be either a linear or branched one having 1 to 6 carbon atoms, methyl and ethyl groups are preferable therefor. Although the alkenyl group may be either a linear or branched one having 2 to 6 carbon atoms, a vinyl group is preferable therefor. Although the alkynyl group may be either a linear or branched one having 2 to 6 carbon atoms, an ethynyl group is preferable therefor. One to three fluorine atoms are particularly preferable as the halogen in the halogenomethyl group. Although the alkoxyl group may be one having 1 to 6 carbon atoms, a methoxyl group is preferable therefor. One to three fluorine atoms are particularly preferable as the halogen in the halogenomethoxyl group.

Among these substituents, an alkyl or alkoxyl group is preferable, and methyl and methoxyl groups are still preferable.

$X^2$ and $R^8$ may form together with a part of the mother nucleus (containing $A^2$ to which $R^8$ is bonded and the carbon atoms to which $X^2$ is bonded) a cyclic structure which is a 4- to 7-membered ring being either saturated, partly saturated or unsaturated. This ring may contain an oxygen atom, a nitrogen atom or a sulfur atom as a constitutent atom thereof and optionally have an alkyl group having 1 to 6 carbon atoms as a substituent.

As an example of the fused ring system thus formed, 2,3-dihydro-7-oxo-7H-pyrido[1,2,3-de][1,4]benzoxazin-6-carboxylic acid structure is the preferable one and the 3(S)-methyl compound is particularly preferable.

When $A^1$ is a partial structure represented by the following formula:

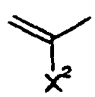

,

examples of the preferable combination of $R^{10}$ with $X^2$ include those wherein $R^{10}$ is an amino group, a hydrogen atom, a hydroxyl group or an alkyl group having 1 to 6 carbon atoms and $X^2$ is an alkyl group having 1 to 6 carbon atoms, an alkoxyl group having 1 to 6 carbon atoms, a halogen atom, a halogenomethoxyl group or a hydrogen atom.

Examples of the still preferable combination thereof are those wherein $R^{10}$ is an amino group, a hydrogen atom, a hydroxyl group or a methyl group and $X^2$ is a methyl group, a methoxyl group, a fluorine atom, a chlorine atom, a difluoromethoxyl group or a hydrogen atom.

Examples of the particularly preferable combination thereof are those wherein $R^{10}$ is an amino group, a hydrogen atom, a hydroxyl group or a methyl group and $X^2$ is a methyl group or a methoxyl group.

Preferable examples of $R^{10}$ and $X^2$ are cited above. On the other hand, a fluorine atom is preferable as $X^1$.

When the substituents $X^1$ and $X^2$ are each a halogen atom, it is particularly preferable that $X^1$ is a fluorine atom and $X^2$ is a fluorine or chlorine atom.

Next, the halogenocyclopropyl group represented by $R^8$ will be explained.

Examples of the halogen atom as the substituent include fluorine and chlorine atoms and a fluorine atom is particularly preferable therefor.

With respect to the stereochemical environment in this moiety, it is particularly preferable that the halogen atom and the pyridonecarboxylate moiety are located at the cis-configuration regarding the cyclopropane ring.

There are so-called enantiomers with respect to the cis-2-halogenocyclopropyl moiety of $R^8$. These isomers each shows a potent antimicrobial activity and a high safety.

The compounds of the present invention show excellent characteristics owing to the substituent represented by the following formula (III) located on the pyrrolidine ring:

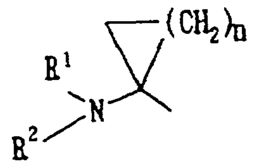

(III)

The compounds of the present invention are further characterized in that this substituent and the substituent $R^4$ are located in the cis-configuration. (As a matter of course, the substituents $R^3$ and $R^5$ are located in the cis-configuration too.) It has been clarified that the compounds of the present invention have excellent characteristics in safety since these substituents are located in the cis-configuration. That is to say, favorable properties such as a decrease in the acute toxicity and negativeness in the micronuclear test are thus confirmed. Especially, it has been clarified that the compounds of the present invention, which are characterized in that the substituent of the formula (III) and the substituent $R^4$ are located in the cis-configuration, are superior in a decrease in the acute toxicity as compared with those compounds having the substituent of the formula (III) and the substituent $R^4$ in the trans-configuration.

The excellent characteristics in safety of the compounds of the present invention are obvious when the cyclic moiety in the substituent represented by the formula (III) is a 3-membered ring. Also, these characteristics become obvious when the substituent $R^4$ is a fluorine atom. As preferable examples of the compounds according to the present invention, namely, those wherein n is 1 and the substituent $R^4$ is a fluorine atom are exemplified.

In the compounds represented by the formula (I) of the present invention, the substituent $R^4$ and the substituent having a cyclic structure of the formula (III) are located in the cis-configuration. More particularly speaking, the following two isomers occur with respect to this moiety:

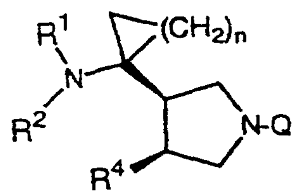

;

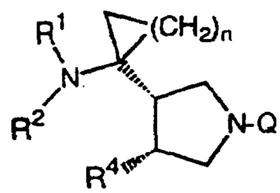

The present inventors consider that the isomer represented by the following formula is preferred to another one:

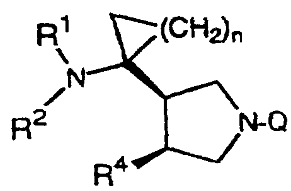

When a compound represented by the formula (I) according to the present invention has a structure allowing the existence of diastereomers, it is preferable that a compound comprised of a single diastereomer is administered to human being or animals. The term "comprised of a single diastereomer" as used herein means not only one being completely free from other diastereomer(s) but also one having a certain degree of chemically purity. That is to say, it may contain other diastereomer(s) so long as neither the physical constants nor the physiological activities thereof are affected thereby.

Also, the term "stereochemically pure" as used herein means a compound consisting of one of isomers, when the compound has two or more isomers due to asymmetric carbon atom(s) contained therein. The term "pure" of this case can be understood in the same manner as the abovementioned case.

The pyridonecarboxylic acid derivatives of the present invention may be in a free state. Alternatively, they may be converted into acid addition salts or carboxylates thereof. Examples of the acid addition salts include inorganic acid salts (e.g., hydrochloride, sulfate, nitrate, hydrobromide, hydroiodide and phosphate) and organic acid salts (e.g., acetate, methanesulfonate, benzenesulfonate, toluenesulfonate, citrate, maleate, fumarate and lactate).

Examples of the carboxylates include alkali metal salts (e.g., lithium salt, sodium salt and potassium salt), alkaline earth metal salts (e.g., magnesium salt and calcium salt), ammonium salt, triethylamine salt, N-methylglucamine salt and tris-(hydroxymethyl)aminomethane salt. Either inorganic salts or organic salts are usable therefor.

These free pyridonecarboxylic acid derivatives or their acid addition salts or carboxylates may occur as hydrates thereof.

On the other hand, quinolone derivatives wherein the carboxylate moiety (-COOY) is an ester are useful as intermediates in the synthesis or prodrugs. For example, alkyl esters, benzyl esters, alkoxyalkyl esters, phenylalkyl esters and phenyl esters are useful as intermediates in the synthesis.

The esters usable as prodrugs are those which are easily cleaved *in vivo* to thereby form free carboxylates. Examples thereof include acetoxymethyl ester, pivaloyloxymethyl ester, ethoxycarbonyl ester, choline ester, dimethylaminoethyl ester, 5-indanyl ester and oxoalkyl esters such as phthalidinyl ester, 5-alkyl-2-oxo-1,3-dioxol-4-ylmethyl ether and 3-acetoxy-2-oxobutyl ester and the like.

The compounds represented by the formula (I) of the present invention can be produced by various methods. A preferable example of these method comprises reacting a compound represented by the following formula (IV):

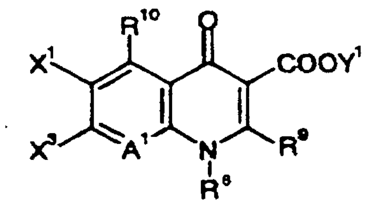

(IV)

wherein $X^3$ represents a group serving as a leaving group such as a fluorine atom, a chlorine atom, a bromine atom, a substituted or unsubstituted phenylsulfonyl group or a substituted or unsubstituted alkylsulfonyl group having 1 to 3 carbon atoms;

$Y^1$ means Y as defined in the above formula (I) or a boron-containing group represented by the following formula:

$$-B(Y^{11})Y^{12}$$

wherein $Y^{11}$ and $Y^{12}$ represent each a fluorine atom or an alkylcarbonyloxy group having 2 to 4 carbon atoms; and

$R^8$, $R^9$, $R^{10}$, $A^1$ and $X^1$ are as defined in the above formula (I);

or a compound represented by the following formula (V) :

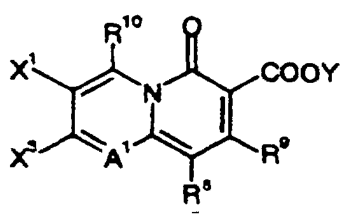

(V)

wherein $X^3$ represents a group serving as a leaving group such as a fluorine atom, a chlorine atom, a bromine atom, a substituted or unsabstuted phenylsulfonyl group or a substituted or unsubstituted alkylsulfonyl group having 1 to 3 carbon atoms; and

$R^8$, $R^9$, $R^{10}$, $A^1$, $X^1$ and Y are as defined in the above formula (I);

with a compound represented by the following formula (VI) or its acid addition salts:

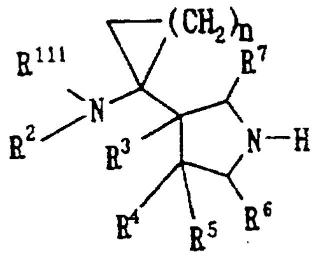

(VI)

wherein $R^{111}$ has the same meaning as that of $R^1$ as defined in the above formula (I) or represents a protective group of amino group; and

$R^2$, $R^3$, $R^4$, $R^5$, $R^6$, $R^7$ and n are as defined in the above formula (I);

provided that the substituent $R^4$ and the substituent containing the cyclic structure bonded to the carbon atom adjacent to the carbon atom to which the substituent $R^4$ is bonded are located in the cis-configuration.

Examples of the acid addition salt include inorganic acid salts and organic acid salts. More particularly speaking, organic acid salts (e.g., hydrochloride, sulfate, nitrate, hydrobromide, hydroiodide and phosphate) and organic acid salts (e.g., sulfonates such as methanesulfonate, benzenesulfonate and toluenesulfonate, and carboxylates such as acetate, citrate, maleate, fumarate and lactate) are exemplified.

The reaction may be performed with the use of a solvent or without using any solvent. Any solvent may be employed in the reaction, so long as it remains inert under the reaction conditions. For example, dimethyl sulfoxide, pyridine, acetonitrile, ethanol, chloroform, dimethylformamide, dimethylacetamide, N-methylpyrrolidone, tetrahydrofuran, water, 3-methoxybutanol and mixtures thereof are exemplified as a solvent.

It is preferable that the reaction is performed in the presence of an acid acceptor such as an inorganic base or organic acid base (e.g., alkali metal or alkaline earth metal carbonate or hydrogencarbonate, triethylamine, pyridine or 1,8-diazabicyclundecene).

The reaction temperature usually ranges from room temperature to 200 °C, preferably from about 25 °C to 150 °C. The reaction is continued for 15 minutes to 48 hours. In usual, it may be completed within about 30 minutes to 15 hours.

The protective group of amino group may be an arbitrary one generally employed in the art. Examples thereof include optionally substituted alkoxycarbonyl groups (e.g., tert-butoxycarbonyl and 2,2,2-trichloroethoxycarbonyl), optionally substituted aralkyloxycarbonyl groups (e.g., benzyloxycarbonyl, p-methoxybenzyloxycarbony and p-nitrobenzyloxycarbonyl), optionally substituted acyl groups (e.g., acetyl, methoxyacetyl, trifluoroacetyl, chloroacetyl, pivaloyl, formyl and benzoyl), optionally substituted alkyl groups and optionally substituted aralkyl groups (e.g., tert-butyl, benzyl, p-nitrobenzyl, p-methoxybenzyl and triphenylmethyl), ethers (e.g., metoxymethyl, tert-butoxymethyl, tetrahydropyranyl and 2,2,2-trichloroethoxymethyl) and silyl groups (e.g., trimethylsilyl, isopropyldimethylsilyl, tert-butyldimethylsilyl, tribenzylsilyl and tert-butyldiphenylsilyl).

When Y and $Y^1$ represent each an alkyl group having 1 to 6 carbon atoms, an alkoxymethyl group having 2 to 7 carbon atoms or a phenylalkyl group composed of an alkylene group having 1 to 6 carbon atoms with a phenyl group, the compound can be converted into the corresponding carboxylic acid by treating under acidic or basic conditions generally employed in the hydrolysis of carboxylates.

When $Y^1$ is a structure represented by the following formula:

$$-B(Y^{11})Y^{12}$$

the compound of the formula (IV) is reacted with the compound of the formula (VI) and then treated under acidic or basic conditions to thereby convert the reaction product into the corresponding carboxylic acid.

When deprotection is needed, the protective group is removed under appropriate procedure known in the art for the protective group used to thereby give the target compound of the formula (I).

The compound represented by the formula (VI) can be formed by removing Q' from a compound represented by the following formula (VII) :

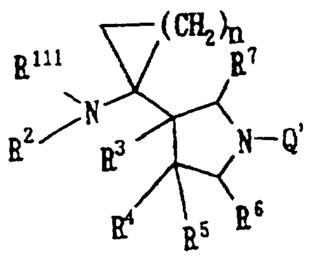

(VII)

wherein $R^{111}$ represents a hydrogen atom, an alkyl group having 1 to 6 carbon atoms or a protective group of amino group;

$R^2$ represents a hydrogen atom or an alkyl group having 1 to 6 carbon atoms, provided that the alkyl group may have one or more substituents selected from the group consisting of a hydroxyl group, a halogen atom and an alkoxy group having 1 to 6 carbon atoms;

$R^3$ and $R^5$, each represents a hydrogen atom;

$R^4$ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, provided that the alkyl group may have one or more substituents selected from the group consisting of a hydroxyl group, a halogen atom and an alkoxy group having 1 to 6 carbon atoms; and

$R^4$ and the substituent on the pyrrolidine ring of the following formula:

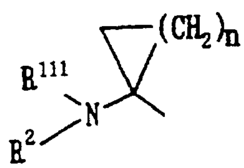

are located in the cis-configuration (as a matter of course, the substituents $R^3$ and $R^5$ are located at the cis-configuration too);

$R^6$ and $R^7$, each independently represents a hydrogen atom or an alkyl group having 1 to 6 carbon atoms;

n is an integer of from 1 to 3; and

Q represents a protective group for amino group selected from the group consisting of optionally substituted alkoxycarbonyl groups, optionally substituted aralkyloxycarbonyl groups, optionally substituted acyl groups, optionally substituted alkyl groups, optionally substituted aralkyl groups and substituted silyl groups.

The compound represented by the formula (VII) may present as salts or hydrates thereof or hydrates of the salts. Examples of the acid addition salts include inorganic acid salts and organic acid salts. More particularly speaking, inorganic acid salts (e.g., hydrochloride, sulfate, nitrate, hydrobromide, hydroiodide and phosphate) and organic acid salts (e.g., sulfonates such as methanesulfonate, benzenesulfonate and toluenesulfonate, and carboxylates such as acetate, citrate, maleate, fumarate and lactate) are exemplified.

When $R^{111}$ and Q' are both protective group of amino group, they may be either the same or different from each other. To produce the compound (I) , it is advantageous that these protective groups for amino group are those which are removed under different reaction conditions.

Examples of the protective groups for $R^{111}$ and Q' include optionally substituted alkoxycarbonyl groups, optionally substituted aralkyloxycarbonyl groups, optionally substituted acyl groups, optionally substituted alkyl groups, optionally substituted aralkyl groups and substituted silyl groups.

Particular examples thereof include optionally substituted alkoxycarbonyl groups (e.g., tert-butoxycarbonyl and 2,2,2-trichloroethoxycarbonyl), optionally substituted aralkyloxycarbonyl groups (e.g., benzyloxycarbonyl, p-methoxybenzyloxycarbony and p-nitrobenzyloxycarbonyl), optionally substituted acyl groups (e.g., acetyl, methoxyacetyl, trifluoroacetyl, chloroacetyl, pivaloyl, formyl and benzoyl), optionally substituted alkyl groups and optionally substituted aralkyl groups (e.g., tert-butyl, benzyl, p-nitrobenzyl, p-methoxybenzyl and triphenylmethyl), ethers (e.g., metoxymethyl,-tert-butoxymethyl, tetrahydropyranyl and 2,2,2-trichloroethoxymethyl) and substituted silyl groups (e.g., trimethylsilyl, isopropyldimethylsilyl, tert-butyldimethylsilyl, tribenzylsilyl and tert-butyldiphenylsilyl).

The compound of the formula (VII) can be produced as the cis-compound by forming a compound (pyrroline

derivative) wherein the carbon atom to which the substituent $R^4$ is bonded and the adjacent carbon atom is bonded via a double bond followed by catalytic reduction. Alternatively, the cis-compound can be produced by once forming a compound wherein the substituent $R^4$ and the substituent moiety having the cyclic structure are located at the trans-configuration and then inverting the configuration of the substituent $R^4$.

Because of having potent antimicrobial effects, the compounds of the present invention are usable as drugs for human being, animals and fishes or preservatives for agricultural chemicals and foods.

When used as drugs for human being, the compounds of the present invention are administered to an adult in a dose of from 50 mg to 1 g per day, preferably from 100 mg to 300 mg per day.

When used as drugs for animals, the dose of the compounds of the present invention varies depending on the purpose (therapeutic use or prevention, etc.) of the administration, the type and size of the animal to be treated, the pathogenic bacterium and the severity of the infection. In general, a daily dose ranges from 1 mg to 200 mg per kg body weight, preferably from 5 mg to 100 mg per kg.

Such a compound is administered in the above daily dose once to 4 times in a day. If necessary, it may be administered in a dose exceeding the above-mentioned level.

The compounds of the present invention are efficacious against infective microorganisms over a wide range and can treat, prevent or ameliorate diseases caused by these microorganisms.

Examples of the bacteria and bacterium-like microorganisms against which the compounds of the present invention are efficacious include *Staphylococcus, Streptococcus pyogenes,* hemolytic streptococcus, enterococcus, pneumococcus, the genus peptostreptococcus, *Neisseria gonorrhoeae, Escherichia coli,* the genus citrobacter, the genus shigella, *Klebsiella pneumoniae,* the genus enterobacter, the genus serratia, the genus proteus, *Pseudomonas aeruginosa,* influenza virus, the genus acinetobacter, the genus campylobacter and *Chlamydia trachomatis.*

Examples of the diseases induced by these pathogenic microorganisms include folliculitis, furuncle, carbuncle, erysipelas, phlegmon, lymphantitis, lymphnoditis, panaritium (felon), subcutaneous tumor, hidrosadenitis, aggregated acne, infectious atheroma, anal abscess, mastitis, superfacial secondary infection such as-trauma, burn and operative wound, pharyngolaryngitis, acute bronchitis, tonsillitis, chronic bronchitis, bronchiectasis, diffuse bronchiolitis, secondary infection in chronic respiratory diseases, pneumonia, pyelonephritis, cystitis, prostatitis, epididymitis, gonococcal urethritis, non-gonococcal urethritis, cholecystitis, cholangitis, bacillary dysentery, enteritis, uterine adnexitis, intrauterine infection, bartholinitis, tarsitis, hordeolum, dacryocystitis, tarsadenitis, corneal ulcer, oititis media, sinusitis, periodontium inflammation, pericoronitis, jaw inflammation, peritonitis, endocarditis, sepsis, meningitis and skin infectious diseases.

The compounds of the present invention are also efficacious against microorganisms causing infectious diseases in animals, for example, escherichia, salmonella, pasteurella, hemophilus, bordetella, staphylococcus and mycoplasma. Particular examples of the diseases caused by these microorganisms include bird diseases (e.g., *E. coli* infection, pullorum disease, avian paratyphoid, avian cholera, infectious coryza, staphylococcus infection and mycoplasma infection), swine diseases (e.g., *E. coli* infection, salmonellosis, pasteurellosis, hemophilus infection, atrophic rhinitis, exudativesuperfacial inflammation and mycoplasma infection), bovine diseases (e.g., *E. coli* infection, salmonellosis, hemorrhagic septicemia, mycoplasma infection, bovine pleuropneumonia and bovine mastitis), canine diseases (e.g., coliemia, salmonellosis, hemorrhagic septicemia, uterine empyema and cystitis) and feline diseases (e.g., exudative pleurisy, cystitis, chronic rhinitis, *Haemophilus* infection, kitten diarrhea and mycoplasma infection).

Antimicrobial agents comprising the compounds of the present invention may be processed into appropriate preparations depending on the administration method by using various processes commonly employed in the art. Examples of the dosage forms of the antimicrobial preparations for oral use containing the compounds of the present invention as the principal agent include tablets, dusts, granules, capsules, solutions, syrups, elixirs and oily and aqueous suspension.

When employed as injections, the preparations may contain stabilizers, antiseptics and solubilizers. A solution optionally containing these auxiliary components may be packed into a container and processed into a solid preparation by freeze-drying, etc. to give a product to be prepared before suing. Such a preparation may be packed in a container in a single dose. Alternatively, it may be packed in a single container in a number of doses.

Examples of external preparations include solutions, suspensions, emulsions, ointments, gels, creams, lotions and sprays.

Solid preparations may be produced by blending the active compounds with pharmaceutically acceptable additives appropriately selected from among excipients, fillers, binders, disintegration agents, dissolution accelerators, moistening agents, lubricants, etc. followed by processing.

Examples of liquid preparations include solutions, suspensions and emulsions. These preparations may contain suspending agents, emulsifiers, etc. as additives.

The methods for administering the compounds of the present invention to animals include an oral administration method comprising directly adding to feed; an oral administration method comprising once preparing solutions followed by direct administration or addition to drinking water or feed; and an injection method.

To administer to animals, the compounds of the present invention can be processed into dusts, fine subtilaes, soluble dusts, syrups, solutions or injections by the techniques commonly employed in the art.
Next, formulation examples will be given.

| Formulation Example 1 [Capsule]: | |
|---|---|
| Compound of Example 3 | 100.0 mg |
| Corn starch | 23.0 mg |
| CMC calcium | 22.5 mg |
| Hydroxymethylcellulose | 3.0 mg |
| Magnesium stearate | 1.5 mg |
| Total | 150.0 mg |

| Formulation Example 2 [Solution]: | |
|---|---|
| Compound of Example 5 | 1 - 10 g |
| Acetic acid or sodium hydroxide | 0.5 - 2 g |
| Ethyl parahydoxybenzoate | 0.1 g |
| Purified water | 87.9 - 98.4 g |
| Total | 100 g |

| Formulation Example 3 [Dust to be added to animal feed]: | |
|---|---|
| Compound of Example 7 | 1 - 10 g |
| Corn starch | 98.5 - 89.5 g |
| Soft silicic anhydride | 0.5 g |
| Total | 100 g |

<u>BEST MODE FOR CARRYING OUT INVENTION</u>:

To further illustrate the present invention in greater detail, and not by way of limitation, the following Examples will be given. The antimicrobial activity of each target compound was evaluated in accordance with the standard method specified by Japan Society of Chemotherapy and expressed in MIC (µg/ml).

[Referential Example 1-1]

<u>Ethyl 3-(1-tert-butoxycarbonylaminocyclopropyl)propiolate</u>

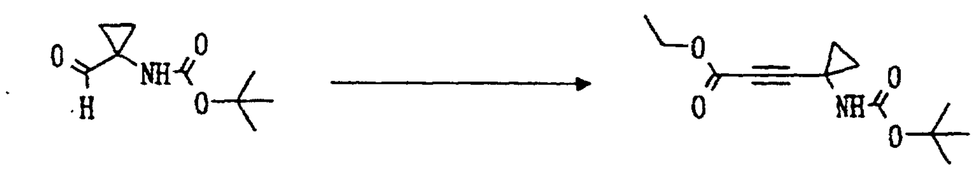

Under a nitrogen atmosphere, chloromethyltrimethylphosphonium chloride (5.156 g, 14.85 mmol) was suspended in dry tetrahydrofuran (30 ml). After cooling the suspension to give an internal temperature of - 55 °C, a 1.68 M solution of n-butyllithium in n-hexane (8.87 ml, 14.90 mmol) was dropped thereinto over 5 minutes. Then the reaction suspension was stirred under ice cooling for 30 minutes and then at room temperature for additional 3 hours followed by cooling to give an internal temperature of - 55 °C. Into this reaction suspension was dropped a solution of 1-tert-butoxycarbonylaminocyclopropane carbaldehyde (2.49 g, 13.50 mmol) in dry tetrahydrofuran (10 ml) over 10 minutes

and the resultant mixture was stirred at - 50°C for 1 hour and then under ice cooling for additional 30 minutes. The reaction suspension was cooled to - 78 °C and a 1.68 M solution of n-butyllithium in n-hexane (17.68 ml, 29.70 mmol) was dropped thereinto over 10 minutes followed by stirring at - 78 °C for 20 minutes. Next, ethyl chloroformate (1.61 ml, 16.88 mmol) was dropped into this reaction suspension followed by stirring at - 78 °C for 1.5 hours and then under ice cooling for 1 hour. Under ice cooling, a saturated aqueous solution of sodium chloride (30 ml) was added to the reaction suspension and the organic layer was separated. The aqueous layer was extracted with diethyl ether (30 ml × 2) and the combined organic layer was washed with a saturated aqueous solution of sodium chloride (30 ml) and dried over anhydrous magnesium sulfate. After filtering, the filtrate was concentrated under reduced pressure and the residue was subjected to flash silica gel column chromatography (eluent: n-hexane : ethyl acetate = 5 : 1) to give 2.178 g (63.9 %) of the title compound as a colorless oily substance.

$^1$H-NMR (400 MHz, $CDCl_3$) δ: 5.04 (brs, 1H), 4.27 (q, J = 7.16 Hz, 2H), 1.44 (s, 9H), 1.28 (t, J = 7.16 Hz, 3H), 1.15 (m, 2H),1.06 (m, 2H).

[Referential Example 1-2]

Ethyl 1-benzyl-4-(1-tert-butoxycarbonylaminocyclopropyl)-3-pyrroline-3-carboxylate

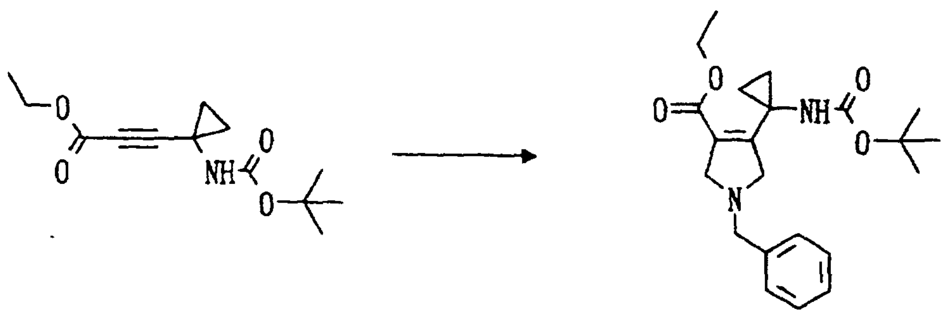

N-Benzyl-N-(n-butoxymethyl)trimethylsilylmethylamine (2.006 g, 7.176 mmol) and ethyl 3-(1-tert-butoxycarbonylaminocyclopropyl)propiolate (1.136 g, 4.485 mmol) were dissolved in dry dichloromethane (9 ml). While stirring at room temperature, a 1.0 M solution of trifluoroacetic acid in dichloromethane (0.72 ml, 0.72 mmol) was added thereto and the liquid reaction mixture was stirred for 3 hours. Then a saturated aqueous solution of sodium bicarbonate (20 ml) was added to the liquid reaction mixture followed by extraction with dichloromethane (20 ml × 3). The combined organic layer was washed with a saturated aqueous solution of sodium chloride (30 ml) and dried over anhydrous magnesium sulfate. After filtering, the filtrate was concentrated under reduced pressure and the residue was subjected to flash silica gel column chromatography (eluent: chloroform) to give 1.449 g (83.6 %) of the title compound as a colorless oily substance.

$^1$H-NMR (400 MHz, $CDCl_3$) δ: 7.40 - 7.11 (m, 5H), 5.17 (brs,. 1H), 4.12 (q, J = 6.83 Hz, 2H), 3.85 (m, 2H), 3.72 (m, 2H), 3.67 (s, 2H), 1.44 (s, 9H), 1.24 (t, J = 6.83 Hz, 3H), 1.14 (m, 2H), 1.01 (m, 2H).

[Referential Example 1-3]

Ethyl cis-1-benzyl-4-(1-tert-butoxycarbonylaminocyclopropyl)-pyrrolidine-3-carboxylate

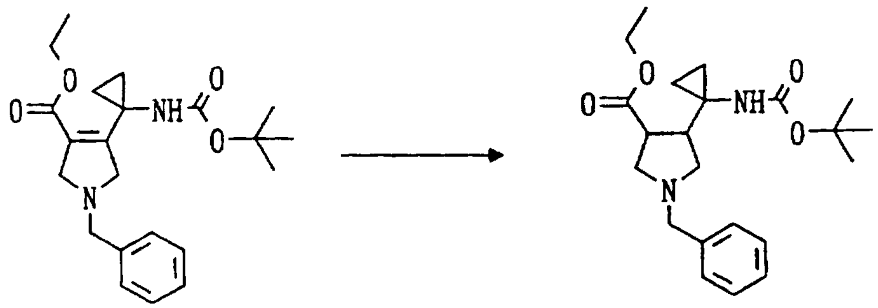

Under a nitrogen gas stream, bis(bicyclo[2.2.1]hepta-2,5-diene)rhodium (I) perchlorate (54.5 mg, 0.14 mmol) and 1,2-bis(diphenylphosphino)ethane (67.4 mg, 0.17 mmol) were dissolved in degassed methanol (25 ml) and stirred at room temperature for 10 minutes. To this catalyst solution was added a solution of ethyl 1-benzyl-4-(1-tert-butoxycarbonylaminocyclopropyl)-3-pyrroline-3-carboxylate (1.090 g, 2.820 mmol) in dry and degassed methanol (15 ml). The obtained liquid reaction mixture was then stirred under a hydrogen atmosphere (1 kg/cm$^2$) at room temperature for 2.5 hours. After adding active carbon (1 g), the liquid reaction mixture was allowed to stand at room temperature for 30 minutes and then filtered through celite (washed with methanol). The filtrate was concentrated under reduced pressure and the residue was subjected to flash silica gel column chromatography (eluent: n-hexane : ethyl acetate = 5 : 1) to give 1.071 g (97.8 %) of the title compound as colorless crystals.

$^1$H-NMR (400 MHz, $CDCl_3$) δ: 7.40 - 7.19 (m, 5H), 5.07 (brs, 1H), 4.13 (q, J = 7.33 Hz, 2H), 3.63 (s, 2H), 2.87 (m, 1H), 2.67 (m, 1H), 2.54 (m, 1H), 2.35 (m, 1H), 2.15 (m, 1H), 1.79 (m, 1H), 1.46 (s, 9H), 1.23 (t, J = 7.33 Hz, 3H), 0.85 (m, 2H), 0.69 (m, 2H).

[Referential Example 1-4]

cis-1-Benzyl-4-(1-tert-butoxycarbonylaminocyclopropyl)-3-hydroxymethylpyrrolidine

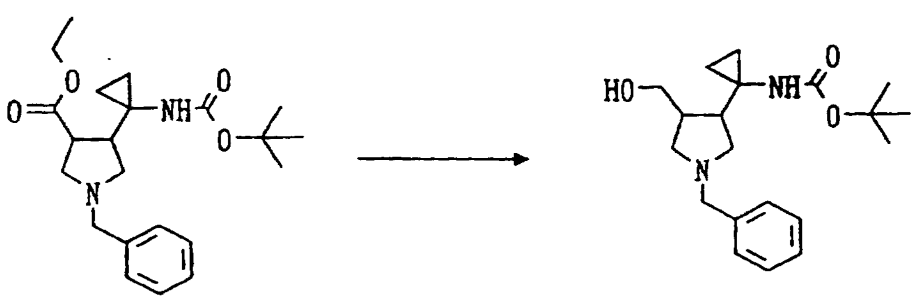

Under a nitrogen gas stream, lithium aluminum hydride (195.6 mg, 5.135 mmol) was suspended in dry tetrahydrofuran (40 ml). Under stirring at - 15 °C, a solution of ethyl cis-1-benzyl-4-(1-tert-butoxycarbonylaminocyclopropyl)-pyrrolidine-3-carboxylate (1.001 g, 2.577 mmol) in dry tetrahydrofuran (10 ml) was dropped thereinto over 15 minutes. After stirring the reaction suspension under ice cooling for 3.5 hours, cooling water (5 ml) was slowly added thereto and the mixture was stirred at room temperature for additional 15 minutes. The reaction suspension was filtered through celite (washed with diethyl ether). The filtrate was concentrated under reduced pressure and dried to give 833.9 mg (93.4 %) of the title compound as a colorless oily substance.

$^1$H-NMR (400 MHz, $CDCl_3$) δ: 7.39 - 7.00 (m, 5H), 5.10 (brs, 1H), 3.69 (m, 2H), 3.58 (s, 2H), 2.99 (m, 1H), 2.61 (m, 1H), 2.51 (m, 1H), 2.27 (m, 1H), 2.00 (m, 1H), 1.94 (brs, 1H), 1.74 (m, 1H), 1.42 (s, 9H), 0.90 (m, 1H), 0.74

- 0.61 (m, 3H).

[Referential Example 1-5)

cis-4-(1-tert-Butoxycarbonylaminocyclopropyl)-3-hydroxymethylpyrrolidine

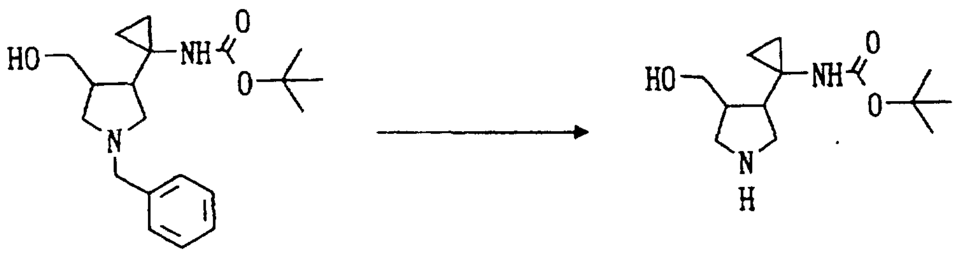

cis-1-Benzyl-4-(1-tert-butoxycarbonylaminocyclopropyl)-3-hydroxymethylpyrrolidine (820.1 mg, 2.376 mmol) was dissolved in methanol (50 ml). After adding 5 % palladium-carbon catalyst (moisture content: 55.6 %, 750 mg), the mixture was stirred under elevated hydrogen pressure (4.5 kg/$cm^2$) over day and night. After filtering off the catalyst through celite (washed with methanol), the filtrate was concentrated under reduced pressure to give 578.8 mg (91.0 %) of the title compound as a white amorphous substance.

$^1$H-NMR (400 MHz, $CDCl_3$) δ: 5.05 (brs, 1H), 3.72 (m, 2H), 3.15 (m, 2H), 2.82 (m, 2H), 2.29 (m, 1H), 1.94 (br, 2H), 1.76 (m, 1H), 1.42 (s, 9H), 0.92 (m, 2H), 0.82 (m, 1H), 0.61 (m, 1H).

[Example 1] ; for reference purpose

5-Amino-7-cis-4-(1-aminocyclopropyl)-3-hydroxymethyl-1-pyrrolidinyl-1-cyclopropyl-6-fluoro-1,4-dihydro-8-methyl-4-oxoquinoline-3-carboxylic acid

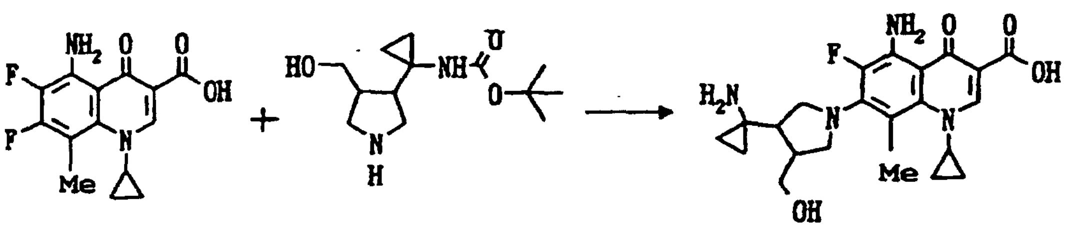

cis-4-(1-tert-Butoxycarbonylaminocyclopropyl)-3-hydroxymethylpyrrolidine (550.1 mg, 2.146 mmol) was dissolved in dimethyl sulfoxide (15 ml) and triethylamine (3.5 ml) and 5-amino-1-cyclopropyl-6,8-difluoro-1,4-dihydro-8-methyl-4-oxoquinoline-3-carboxylic acid (300.2 mg, 1.020 mmol) were added thereto. Then the mixture was stirred under a nitrogen atmosphere in an oil bath at 150 °C for 22 hours. After allowing to cool, dimethyl sulfoxide was evaporated. The residue was dissolved in chloroform (100 ml), washed successively with a 10 % aqueous solution of citric acid (100 ml) and a saturated aqueous solution of sodium chloride (50 ml). The organic layer was dried over anhydrous magnesium sulfate. After filtering, the filtrate was concentrated under reduced pressure. Under ice cooling, conc. hydrochloric acid (10 ml) was dropped into the residue followed by stirring for 1 hour. The liquid reaction mixture was washed with dichloromethane (20 ml x 4) and the pH value of the aqueous layer was adjusted to 12 with a 15 % aqueous solution of sodium hydroxide followed by washing with dichloromethane (20 ml × 2). The pH value of this aqueous solution was adjusted to 7.2 with 1 N hydrochloric acid followed by extraction with-chloroform (100 ml × 4). The combined organic layer was dried over anhydrous magnesium sulfate and filtered. The filtrate was concentrated

under reduced pressure and the crude product thus obtained was recrystallized from a 2-propanol/diisopropyl ether system. The crystals thus obtained were dried under reduced pressure at 70 °C for 18 hours to give 112.4 mg (25.6 %) of the title compound as yellow crystals.

Melting point: 158.8 - 159.9 °C (decomp.).

$^1$H-NMR (400 MHz, 0.1 N-NaOD) δ: 8.39 (s, 1H), 3.99 (m, 1H), 3.80 (dd, J = 11.23, 5.37 Hz, 1H), 3.62 (m, 2H), 3.51 (d, J = 7.32, 2H), 3.41 (t, J = 7.81 Hz, 1H), 2.45 (m, 1H), 2.37 (s, 3H), 1.71 (q, J = 7.81, 1H), 1.18 (m, 2H), 0.74 (m, 1H), 0.70 (m, 1H), 0.55 (m, 4H).

| Elemental analysis data: as $C_{22}H_{27}FN_4O_4$ | | | |
|---|---|---|---|
| calcd. | C, 61.31; | H, 6.32; | N, 13.02 |
| found | C, 61.25; | H, 6.32; | N, 12.74. |

[Referential Example 2-1]

4-(S)-(1-Ethoxycarbonylcyclopropyl)-3-(R)-fluoro-1-[1-(S)-phenylethyl)-2-pyrrolidone

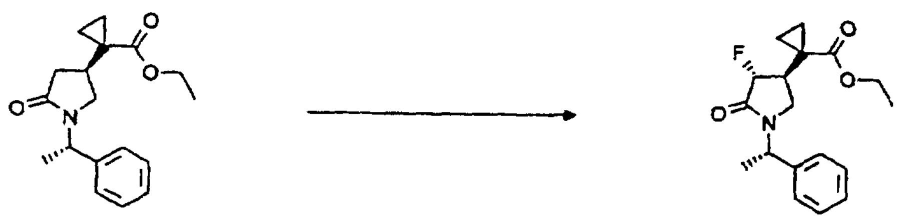

Under a nitrogen atmosphere, diisopropylamine (3.99 ml, 30.4 mol) was dissolved in dry tetrahydrofuran (50 ml). After cooling the solution to - 78 °C, a 1.68 M solution of n-butyllithium in n-hexane (18.1 ml, 30.4 mmol) was dropped thereinto over 10 minutes. Then the liquid reaction mixture was stirred at - 10 °C for 20 minutes and cooled to - 78 °C. Next, a solution of 4-(S)-(1-ethoxycarbonylcyclopropyl)-1-[1-(S)-phenylethyl]-2-pyrrolidone (7.052 g, 23.40 mmol) in dry tetrahydrofuran (30 ml) was dropped thereinto over 15 minutes. The liquid reaction mixture was stirred at - 78 °C for 1 hour. Then a solution of N-fluorobenzenedisulfonimide (11.81 g, 37.44 mmol) in dry tetrahydrofuran (60 ml) was dropped thereinto at the same temperature over 25 minutes. The liquid reaction mixture was stirred at - 78 °C for 2 hours and then heated to room temperature followed by stirring for additional 20 minutes. Under ice cooling, a saturated aqueous solution of ammonium chloride (200 ml) was added to the liquid reaction mixture. The organic layer was separated and the aqueous layer was extracted with diethyl ether (200 ml × 2). The combined organic layer was washed with water (200 ml × 3) and dried over anhydrous magnesium sulfate. After filtering, the filtrate was concentrated under reduced pressure and the residue was subjected to flash silica gel column chromatography (eluent: n-hexane : ethyl acetate = 3 : 1) to give 5.276 g (70.6 %) of the title compound as a colorless oily substance.

$^1$H-NMR (400 MHz, $CDCl_3$) δ: 0.76 - 0.81 (1H, m), 0.89 - 0.93 (1H, m), 1.09 (3H, t, J = 6.84 Hz), 1.24 - 1.34 (2H, m), 1.58 (3H, d, J = 7.33 Hz), 2.23 (1H, dq, J = 28.32, 8.30 Hz), 2.88 - 2.93 (1H, m), 3.48 (1H, t, J = 9.28 Hz), 3.92 - 4.08 (2H, m), 5.14 (1H, dd, J = 53.71, 7.81 Hz), 5.54 (1H, q, J = 7.33 Hz), 7.27 - 7.34 (5H, m).

[Referential Example 2-2]

4-(S)-(1-Ethoxycarbonylcyclopropyl)-3-(S)-fluoro-1-[1-(S)-phenylethyl]1-2-pyrrolidone

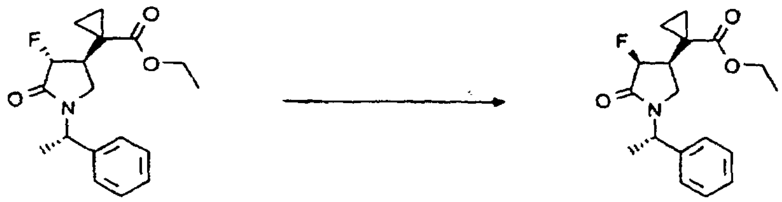

Under a nitrogen atmosphere, diisopropylamine (7.22 ml, 51.52 mmol) was dissolved in dry tetrahydrofuran (100 ml). After cooling the solution to - 78 °C, a 1.68 M solution of n-butyllithium in n-hexane (28.1 ml, 47.21 mmol) was dropped thereinto over 15 minutes. Then the liquid reaction mixture was stirred at 0 °C for 10 minutes and cooled to - 78 °C. Next, a solution of 4-(S)-(1-ethoxycarbonylcyclopropyl)-3-(R)-fluoro-1-[1-(S)-phenylethyl]-2-pyrrolidone (13.72 g, 42.96 mmol) in dry tetrahydrofuran (40 ml) was dropped thereinto over 20 minutes. The liquid reaction mixture was stirred at - 78 °C for 20 minutes. Then a solution of 2,6-di-tert-butylphenol (10.63 g, 51.52 mmol) in dry tetrahydrofuran (40 ml) was dropped thereinto over 20 minutes. The liquid reaction mixture was stirred at - 78 °C for 10 minutes and then heated to room temperature. Under ice cooling, a saturated aqueous solution of ammonium chloride (200 ml) was added to the liquid reaction mixture. The organic layer was separated and the aqueous layer was extracted with diethyl ether (200 ml x 2). The combined organic layer was washed with water (400 ml x 2) and dried over anhydrous magnesium sulfate. After filtering, the filtrate was concentrated under reduced pressure and the residue was subjected to flash silica gel column chromatography (eluent: n-hexane : ethyl acetate = 3 : 1) to give 10.19 g (74.2 %) of the title compound as a colorless oily substance.

$^1$H-NMR (400 MHz, $CDCl_3$) δ: 0.57 - 0.63 (1H, m), 0.78 - 0.84 (1H, m), 1.07 - 1.13 (1H, m), 1.26 (3H, t, J = 7.09 Hz), 1.23 - 1.29 (1H, m), 1.54 (3H, d, J = 7.32 Hz), 2.59 (1H, t, J = 9.77 Hz), 3.05 (1H, dq, J = 28.81, 8.30 Hz), 3.25 (1H, t, J = 9.77 Hz), 4.00 - 4.16 (2H, m), 5.15 (1H, dd, J = 52.73, 6.35 Hz), 5.53 (1H, q, J = 7.32 Hz), 7.27 - 7.38 (5H, m).

[Referential Example 2-3]

4-(S)-(1-Ethoxycarbonylcyclopropyl)-3-(S)-fluoro-1-[1-(S)-phenylethyl]-2-pyrrolidinthione

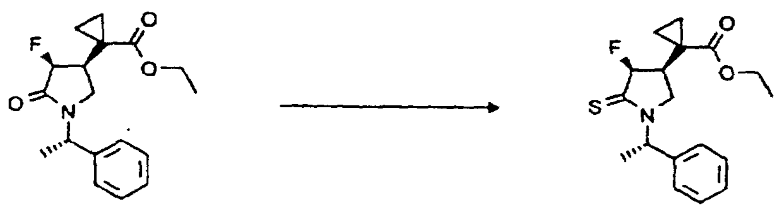

4-(S)-(1-Ethoxycarbonylcyclopropyl)-3-(S)-fluoro-1-[1-(5)-phenylethyl)-2-pyrrolidone (6.86 g, 21.48 mmol) was dissolved in dry toluene (100 ml). After adding Lawesson reagent (5.21 g, 12.89 mmol), the mixture was heated at 60 °C for 30 minutes. After allowing the liquid reaction mixture to cool, toluene was evaporated under reduced pressure and the residue was subjected to flash silica gel column chromatography (eluent: n-hexane : ethyl acetate = 4 : 1) to give 6.49 g (90.1 %) of the title compound as a pale yellow oily substance.

$^1$H-NMR (400 MHz, $CDCl_3$) δ: 0.59 - 0.66 (1H, m), 0.86 - 0.92 (1H, m), 1.08 - 1.15 (1H, m), 1.20 (3H, t, J =

7.33 Hz), 1.24 - 1.31 (1H, m), 1.60 (3H, d, J = 7.32 Hz), 2.85 (1H, dd, J = 11.23, 9.28 Hz), 3.16 (1H, dq, J = 30.27, 8.30 Hz), 3.50 (1H, dd, J = 11.23, 9.28 Hz), 4.04 - 4.15 (2H, m), 5.32 (1H, dd, J = 52.73, 5.38 Hz), 6.28 - 6.34 (1H, m), 7.30 - 7.41 (5H, m).

[Referential Example 2-4]

4-(S)-(1-Ethoxycarbonylcyclopropyl)-3-(S)-fluoro-1-[1-(S)-phenylethyl]pyrrolidine

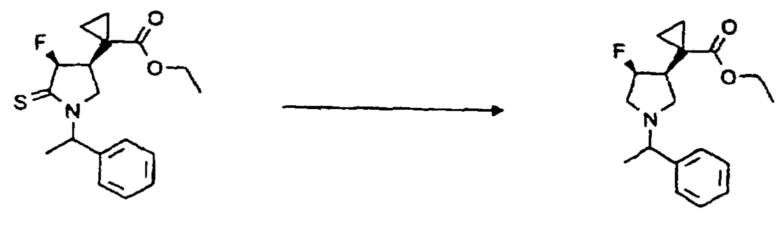

4-(S)-(1-Ethoxycarbonylcyclopropyl)-3-(S)-fluoro-1-[1-(S)-phenylethyl]-2-pyrrolidinthione (6.49 g, 19.35 mmol) was dissolved in dry tetrahydrofuran (150 ml). After adding Raney nickel catalyst (15 ml), the mixture was stirred at room temperature for 30 minutes. After eliminating the catalyst by filtering through celite (washed with tetrahydrofuran), the filtrate was concentrated under reduced pressure. The residue was dissolved in diethyl ether (200 ml) and the obtained solution was washed with a 10 % aqueous solution of ammonia (200 ml x 2) and a saturated aqueous solution of sodium chloride (150 ml) and dried over anhydrous sodium sulfate. After filtering, the filtrate was concentrated under reduced pressure to give 5.08 g (86.0 %) of the title compound as a colorless oily substance.

$^1$H-NMR (400 MHz, $CDCl_3$) δ: 0.54 - 0.60 (1H, m), 0.95 - 1.08 (2H, m), 1.22 (3H, t, J = 7.33 Hz), 1.25 - 1.32 (1H, m), 1.35 (3H, d, J = 6.35 Hz), 1.99 (1H, t, J = 9.28 Hz), 2.42 (1H, t, J = 8.30 Hz), 2.63 (1H, ddd, J = 33.21, 11.72, 1.95 Hz), 2.99 (1H, dm, J = 28.32 Hz), 3.25 - 3.37 (2H, m), 4.03 - 4.16 (2H, m), 5.33 (1H, dm, J = 55.67 Hz), 7.21 - 7.36 (5H, m).

[Referential Example 2-5]

1-Benzyloxycarbonyl-4-(S)-(1-ethoxycarbonylcyclopropyl)-3-(S)-fluoropyrrolidine

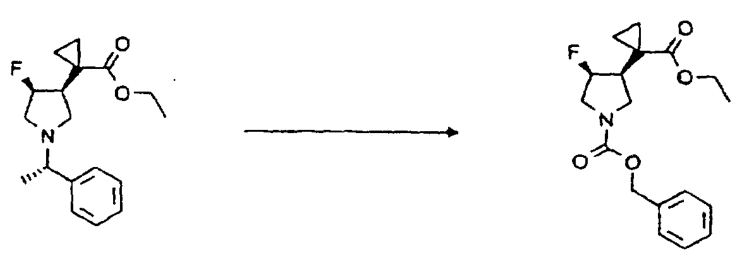

4-(S)-(1-Ethoxycarbonylcyclopropyl)-3-(S)-fluoro-1-[1-(S)-phenylethyl]pyrrolidine (5.08 g, 16.63 mmol) was dissolved in dry dichloromethane (50 ml). Under ice cooling, benzyl chloroformate (3.56 ml, 25.0 mmol) was dropped into this solution. Then the liquid reaction mixture was heated under reflux for 1 hour and dichloromethane was evaporated under reduced pressure. The residue was subjected to flash silica gel column chromatography (eluent: n-hexane : ethyl acetate = 3 : 1) to give 4.67 g (83.7 %) of the title compound as a colorless oily substance.

$^1$H-NMR (400 MHz, $CDCl_3$) δ: 0.71 - 0.78 (1H, m), 1.11 - 1.23 (2H, m), 1.24 (3H, t, J = 6.84 Hz), 1.29 - 1.37 (1H, m), 2.93 - 3.00 (1H, m), 3.10 (1H, dm, J = 34.67 Hz), 3.54 - 3.84 (2H, m), 4.09 - 4.18 (2H, m), 5.14 (2H, s), 5.34 (1H, ddm, J = 53.71, 16.6 Hz), 7.29 - 7.38 (5H, m).

[Referential Example 2-6]

1-[1-Benzyloxycarbonyl-4-(S)-fluoro-3-(S)-pyrrolidinyl)-cyclopropanecarboxylic acid

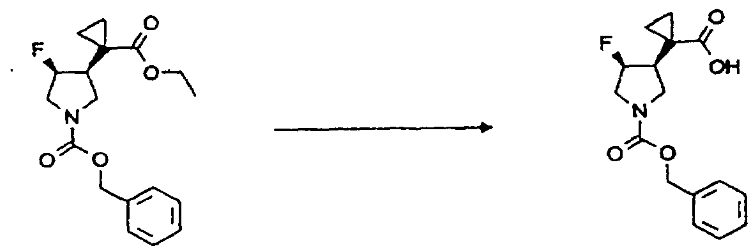

1-Benzyloxycarbonyl-4-(S)-(2-ethoxycarbonylcyclopropyl)-3-(S)-fluoropyrrolidine (4.67 g, 13.92 mmol) was dissolved in ethanol (50 ml). Then a 1 N aqueous solution of sodium hydroxide (50 ml) was dropped into this solution. Then the liquid reaction mixture was stirred at 40 °C for 1.5 hours and ethanol was evaporated under reduced pressure. Water (50 ml) was added to the residue followed by washing with chloroform (100 ml). The aqueous layer was separated and acidified by dropping 1 N hydrochloric acid thereinto. Next, it was extracted successively with chloroform (200 ml x 2) and diethyl ether (100 ml). The combined organic layer was dried over anhydrous sodium sulfate. After filtering, the filtrate was concentrated under reduced pressure to give 3.94 g (92.1 %) of the title compound as a colorless amorphous substance.

$^1$H-NMR (400 MHz, $CDCl_3$) δ: 0.79 - 0.89 (1H, m), 1.18 - 1.35 (2H, m), 1.37 - 1.47 (1H, m), 2.90 - 3.18 (2H, m), 3.50 - 3.84 (3H, m), 5.13 (2H, s), 5.31 (1H, ddm, J = 53.22, 15.13 Hz), 7.26 - 7.42 (5H, m).

[Referential Example 2-7]

1-Benzyloxycarbonyl-4-(R)-(1-tert-butoxycarbonylaminocyclopropyl)-3-(S)-fluoropyridine

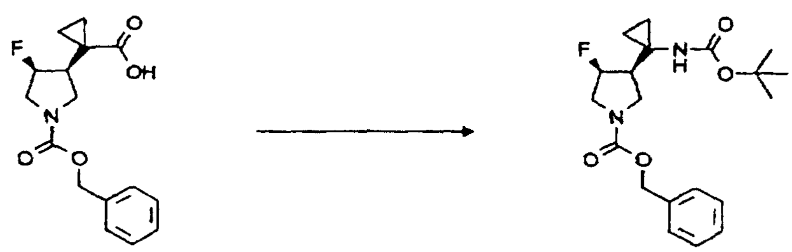

1-[1-Benzyloxycarbonyl-4-(S)-fluoro-3-(S)-pyrrolidinyl]cyclopropanecarboxylic acid (3.22 g, 10.48 mmol) was dissolved in dry acetonitrile (80 ml). After adding N,N'-carbonyldiimidazole (2.55 g, 15.73 mmol), the liquid reaction mixture was stirred at room temperature for 30 minutes. Next, ammonia gas was bubbled thereinto at the same temperature. Then the liquid reaction mixture was concentrated under reduced pressure. Water (80 ml) was added to the residue followed by extraction with chloroform (80 ml x 2). The combined organic layer was dried over anhydrous sodium sulfate. After filtering, the filtrate was concentrated under reduced pressure. The residue was dissolved in tert-butyl alcohol (100 ml) and lead tetraacetate (7.93 g, 15. 70 mmol) was added thereto. After heating under reflux for 30 minutes, the liquid reaction mixture was allowed to cool and diethyl ether (50 ml) and sodium hydrogencarbonate (10 g) were added thereto. Then the mixture was stirred at room temperature for 10 minutes and filtered. The filtrate was concentrated under reduced pressure. After adding ethyl acetate (150 ml) to the residue, the mixture was washed with a saturated aqueous solution of sodium bicarbonate and dried over anhydrous sodium sulfate. After filtering, the filtrate was concentrated under reduced pressure and the residue was subjected to flash silica gel column chromatography (eluent: n-hexane : ethyl acetate = 3 : 2) to give 3.216 g (81.2 %) of the title compound as a colorless oily substance.

$^1$H-NMR (400 MHz, $CDCl_3$) δ: 0.65 - 0.74 (1H, m), 0.70 - 0.84 (1h, m), 0.85 - 1.00 (2H, m), 1.42 (9H, s), 2.21 (1H, ddm, J = 80.57, 36.14 Hz), 3.08 - 3.24 (2H, m), 3.48 - 3.84 (3H, m), 5.02 (1H, brs), 5.13 (2H, s), 5.15 (1H, brd, J = 53.72 Hz), 7.28 - 7.38 (5H, m).

[Example 2]

[Example 2]; for reference purpose

<u>5-Amino-7-[4-(R)-(1-aminocyclopropyl)-3-(S)-fluoro-1-pyrrolidinyl]-6-fluoro-1-[2-(S)-fluoro-1-(R)-cyclopropyl]-1,4-dihydro-8-methyl-4-oxoquinoline-3-carboxylic acid hydrochloride</u>

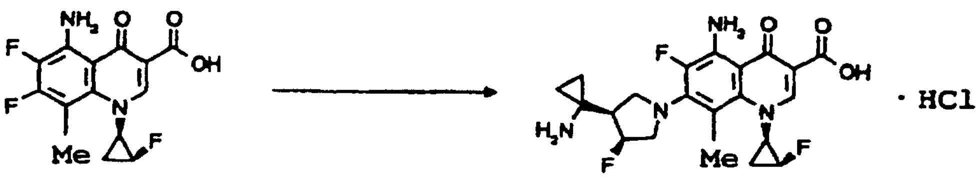

1-Benzyloxycarbonyl-4-(R)-(1-tert-butoxycarbonylaminocyclopropyl)-3-(S)-fluoropyrrolidine (1.43 g, 3.78 mmol) was dissolved in ethanol (60 ml). After adding 5 % palladium-carbon catalyst (moisture content: 55.6 %, 1.5 g), the mixture was stirred under a hydrogen atmosphere for 3 hours. After filtering off the catalyst through celite (washed with methanol), the filtrate was concentrated under reduced pressure. The residue thus obtained was dissolved in dimethyl sulfoxide (12 ml) and 5-amino-6,7-difluoro-1-[2-(S)-fluoro-1-(R)-cyclopropyl]-1,4-dihydro-8-methyl-4-oxoquinoline-3-carboxylic acid (1.18 g, 3.78 mmol) and triethylamine (3 ml) were added thereto. Then the mixture was stirred under a nitrogen atmosphere at 130 °C for 3 days. After allowing to cool, dimethyl sulfoxide was evaporated. The residue was dissolved in chloroform (80 ml), washed successively with a 10 % aqueous solution of citric acid (80 ml) and a saturated aqueous solution of sodium chloride (100 ml). The organic layer was dried over anhydrous sodium sulfate. After filtering, the filtrate was concentrated under reduced pressure. The residue was subjected to flash silica gel column chromatography (eluent: chloroform : methanol = 9 : 1) followed by concentration of the eluate under reduced pressure. Under ice cooling, conc. hydrochloric acid (10 ml) was dropped into the residue followed by stirring at room temperature for 50 minutes. After adding 1 N hydrochloric acid (30 ml), the liquid reaction mixture was washed with chloroform (50 ml x 2) and its pH value was adjusted to 12.0 with an aqueous solution of sodium hydroxide followed by washing with chloroform (100 ml). The pH value of this aqueous solution was adjusted to 7.4 with 1 N hydrochloric acid followed by extraction with chloroform (150 ml x 3). The combined organic layer was dried over anhydrous sodium sulfate and filtered. The filtrate was concentrated under reduced pressure and 1 N hydrochloric acid (2.0 ml) was dropped into the residue under ice cooling. After stirring at the same temperature for 5 minutes, the liquid reaction mixture was concentrated under reduced pressure (azeotropic distillation with ethanol, thrice). The residue was recrystallized from ethanol and dried under reduced pressure to give 230 mg (12.1 %) of the title compound as a yellow powder.

Melting point: 213 - 218 °C (decamp.).

$^1$H-NMR (400 MHz, 0.1 N-NaOD) δ: 0.55 - 0.71 (4H, m), 1 - 2.35 (1H, m), 3.32 (1H, t, J = 8.79 Hz), 3.49 (1H, dd, J = 25.88, 12.21 Hz), 3.85 - 3.97 (2H, m), 4.11 (1H, ddm, J = 40.77, 12.45 Hz), 4.97 (1H, dm, J = 70.31 Hz), 5.49 (1H, brd, J = 55.18 Hz), 8.27 (1H, d, J = 3.42 Hz).

| Elemental analysis data: as $C_{21}H_{23}F_3N_4O_3 \cdot HCl \cdot 1.25H_2O$ | | | |
|---|---|---|---|
| calcd. | C, 50.40; | H, 5.33; | N, 10.87 |
| found | C, 50.45; | H, 5.44; | N, 11.21. |

[Example 3] ; for reference purpose

5-Amino-7-[4-(R)-(1-aminocyclopropyl)-3-(S)-fluoro-1-pyrrolidinyl]-6-fluoro-1-[2-(S)-fluoro-1-(R)-cyclopropyl]-1,4-dihydro-8-methoxy-4-oxoquinoline-3-carboxylic acid

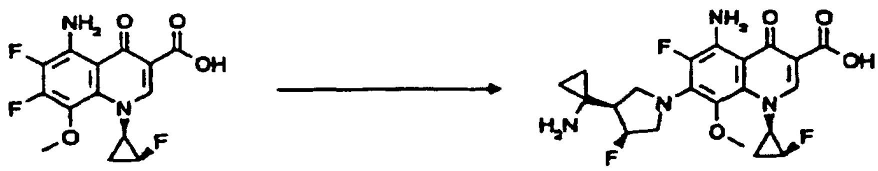

1-Benzyloxycarbonyl-4-(R)-(1-tert-butoxycarbonylaminocyclopropyl)-3-(S)-fluoropyrrolidine (400 mg, 1.06 mmol) was dissolved in ethanol (20 ml). After adding 5 % palladium-carbon catalyst (moisture content: 55.6 %, 500 mg), the mixture was stirred under a hydrogen atmosphere for 18 hours. After filtering off the catalyst through celite (washed with methanol), the filtrate was concentrated under reduced pressure. The residue thus obtained was dissolved in dimethyl sulfoxide (8 ml) and 5-amino-6,7-difluoro-1-[2-(S)-fluoro-1-(R)-cyclopropyl]-1,4-dihydro-8-methoxy-4-oxoquinoline-3-carboxylic acid (289 mg, 0.88 mmol) and triethylamine (2 ml) were added thereto. Then the mixture was stirred under a nitrogen atmosphere at 100 °C for 26 hours. After allowing to cool, dimethyl sulfoxide was evaporated. The residue was dissolved in chloroform (80 ml), washed with a 10 % aqueous solution of citric acid (80 ml). The organic layer was dried over anhydrous sodium sulfate. After filtering, the filtrate was concentrated under reduced pressure. The residue was subjected to flash silica gel column chromatography (eluent: chloroform : methanol = 9 : 1) followed by concentration of the eluate under reduced pressure. Under ice cooling, conc. hydrochloric acid (5 ml) was dropped into the residue followed by stirring at room temperature for 20 minutes. After adding 1 N hydrochloric acid (30 ml), the liquid reaction mixture was washed with chloroform (50 ml x 2) and its pH value was adjusted to 12.0 with an aqueous solution of sodium hydroxide followed by washing with chloroform (100 ml x 2). The pH value of this aqueous solution was adjusted to 7.4 with 1 N hydrochloric acid followed by extraction with chloroform (200 ml x 3). The combined organic layer was dried over anhydrous sodium sulfate and filtered. The filtrate was concentrated under reduced pressure and the residue was recrystallized from ethanol and dried under reduced pressure to give 170 mg (42.6 %) of the title compound as a yellow powder.

Melting point: 211 - 213 °C (decomp.).

$^1$H-NMR (400 MHz, 0.1 N-NaOD) δ: 0.57 - 0.74 (4H, m), 1.12 - 1.27 (1H, m), 1.36 - 1.48 (1H, m), 2.24 (1H, dm, J = 37.60 Hz), 3.46 (3H, s), 3.53 (1H, t, J = 8.79 Hz), 3.69 (1H, dd, J = 25.40, 12.21 Hz), 3,86 - 3.94 (2H, m), 4.10 (1H, ddm, J = 42.48, 12.70 Hz), 5.00 (1H, dm, J = 63.97 Hz), 5.49 (1H, brd. J = 54.69 Hz), 8.19 (1H, d, J = 3.91 Hz).

| Elemental analysis data: as $C_{21}H_{23}F_3N_4O_4$ | | | |
|---|---|---|---|
| calcd. | C, 55.75; | H, 5.12; | N, 12.38 |
| found | C, 55.78; | H, 5.20; | N, 12.28. |

[Example 4] ; for reference purpose

10-[4-(R)-(1-Aminocyclopropyl)-3-(S)-fluoro-1-pyrrolidinyl-9-fluoro-2,3-dihydro-3-(S)-methyl-7-oxo-7H-pyrido (1,2,3-de] [1,4]benzoxazine-6-carboxylic acid

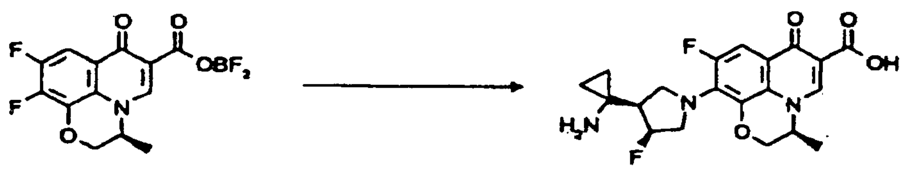

1-Benzyloxycarbonyl-4-(R)-(1-tert-butoxycarbonylaminocyclopropyl)-3-(S)-fluoropyrrolidine (913 mg, 2.41 mmol) was dissolved in methanol (50 ml). After adding 5 % palladium-carbon catalyst (moisture content: 55.6 %, 1.0 g), the mixture was stirred under a hydrogen atmosphere for 3 hours. After filtering off the catalyst through celite (washed with methanol), the filtrate was concentrated under reduced pressure. The residue thus obtained was dissolved in dimethyl sulfoxide (15 ml) and 9,10-difluoro-2,3-dihdyro-3-(S)-methyl-7-oxo-7H-pyrido[1,2,3-de][1,4]benzoxazine-6-carboxylic acid-$BF_2$ chelate (661 mg, 2.01 mmol) and triethylamine (336 μl, 2.41 mmol) were added thereto. Then the mixture was stirred at room temperature for 3 days. After concentrating the liquid reaction mixture under reduced pressure, water was added to the residue. The yellow crystals thus precipitated were collected by filtration and washed with water. The obtained crystals were suspended in a solution (200 ml) of methanol : water = 1 : 1. After adding triethylamine (4 ml), the mixture was heated under reflux for 4 hours. After allowing to cool, the liquid reaction mixture was concentrated under reduced pressure and the residue was dissolved in chloroform (200 ml) and washed with a 10 % aqueous solution of citric acid (200 ml). The organic layer was dried over anhydrous sodium sulfate. After filtering, the filtrate was concentrated under reduced pressure. Under ice cooling, conc. hydrochloric acid (10 ml) was dropped into the residue followed by stirring at room temperature for 10 minutes. After adding 1 N hydrochloric acid (30 ml), the liquid reaction mixture was washed with chloroform (50 ml x 2) and its pH value was adjusted to 12.0 with an aqueous solution of sodium hydroxide. The pH value of this aqueous solution was adjusted to 7.4 with 1 N hydrochloric acid followed by extraction with chloroform (500 ml x 3). The combined organic layer was dried over anhydrous sodium sulfate and filtered. The filtrate was concentrated under reduced pressure and the residue was recrystallized from ethanol and dried under reduced pressure to give 459 mg (56.4 %) of the title compound as pale yellow crystals.
Melting point: 230 - 231 °C (decomp.).
$^1$H-NMR (400 MHz, 0.1 N-NaOD) δ: 0.55 - 0.75 (4H, m), 1.52 (3H, d, J = 6.84 Hz), 2.25 (1H, dm, J = 36.62 Hz), 3.49 (1H, t, J = 8.79 Hz), 3.70 (1H, dd, J = 26.37, 11.72 Hz), 3.88 (1H, t, J = 8.79 Hz), 4.10 (1H, dd, J = 40.53, 12.70 Hz), 4.30 (1H, d, J = 9.27 Hz), 4.50 (1H, d, J = 9.28 Hz), 4.55 - 4.65 (1H, m), 5.47 (1H, dt, J = 55.17, 3.42 Hz), 7.53 (1H, d, J = 14.16 Hz), 8.33 (1H, s).

[Referential Example 3-1]

Ethyl 1-acetylcyclopropanecarboxylate

Ethyl acetoacetate (100 g, 0.77 mol) was dissolved in acetone (500 ml). To the obtained solution was added dibromoethane (361 g, 1.92 mol) and potassium carbonate (266 g, 1.92 mol) and the mixture was heated under reflux for 4 days. After filtering off the insoluble matters, the filtrate was distilled under reduced pressure (80 °C/8 mmHg) to give 78.1 g (65.1 %) of the title compound as a colorless oily substance.
$^1$H-NMR (400 MHz, $CDCl_3$) δ: 1.29 (3H, t, J = 7.33 Hz), 1.47 (4H, s), 2.47 (3H, s), 4.21 (2H, q, J = 7.33 Hz).

[Referential Example 3-2]

Ethyl 3-(1-ethoxycarbonylcyclopropyl)-2-fluoro-2-butenoate

To a solution (1500 ml) of ethyl 1-acetylcyclopropanecarboxylate (124.5 g, 0.797 mmol) in benzene was added

zinc powder (156.4 g, 2.39 mmol). While heating under reflux, a catalytic amount of iodine was added thereto. Subsequently, a solution of ethyl bromofluoroacetate (94.23 ml, 0.797 mol) in benzene (200 ml) was dropped thereinto over 1 hour followed by heating under reflux for 1 hour. Under ice cooling, 1 N hydrochloric acid (1000 ml) was added to the liquid reaction mixture and the mixture was stirred for 1 hour. The organic layer taken up by phase separation was washed successively with 1 N hydrochloric acid, water and a saturated aqueous solution of sodium chloride, dried over anhydrous sodium sulfate and filtered. The filtrate was concentrated under reduced pressure and the residue was dissolved in pyridine (387 ml, 4.78 mol). After adding thionyl chloride (69.8 ml, 0.957 mol) at - 10 °C, the resultant mixture was stirred under ice cooling for 3 hours. Under ice cooling, the liquid reaction mixture was poured into 1 N hydrochloric acid (2000 ml) and ethyl acetate (1500 ml) was added thereto. The organic layer taken up by phase separation was washed successively with 1 N hydrochloric acid, water and a saturated aqueous solution of sodium chloride, dried over anhydrous sodium sulfate and filtered. The filtrate was concentrated under reduced pressure and the residue was dissolved in dichloromethane (500 ml). Under ice cooling, 1,8-azabicyclo[5.4.0]-7-undecene (131 ml, 0.877 mol) was dropped thereinto and then the resultant mixture was stirred at room temperature for 17 hours. After adding 1 N hydrochloric acid (2000 ml) and chloroform (1000 ml), the organic layer taken up by phase separation was washed with a saturated aqueous solution of sodium chloride, dried over anhydrous sodium sulfate and filtered. The filtrate was concentrated under reduced pressure and the residue thus obtained was subjected to flash silica gel column chromatography (eluent: n-hexane : ethyl acetate = 4 : 1) 152.78 g (78.5 %) of the title compound as an oily substance. The compound thus obtained, which was a mixture of geometrical isomers (about 1 : 1), was not separated but employed in the subsequent reaction as such.

[Referential Example 3-3]

(E)-Ethyl 4-bromo-3-(1-ethoxycarbonylcyclopropyl)-2-fluoro-2-butenoate

To a solution of ethyl 3-(1-ethoxycarbonylcyclopropyl)-2-fluoro-2-butenoate (152.78 g, 0.625 mol) in chloroform (1500 ml) were added N-bromosuccinimide (111.33 g, 0.625 mol) and a catalytic amount of 2,2'-azobis-(isobutyronitrile) and then the resultant mixture was heated under reflux for 16 hours. Then the liquid reaction mixture was cooled and concentrated under reduced pressure. After adding benzene (300 ml), the insoluble matters were filtered off and the filtrate was concentrated under reduced pressure. The residue was subjected to flash silica gel column chromatography (eluent: n-hexane : ethyl acetate = 4 : 1) to give 100.5 g (49.7 %) of the title compound as a yellow oily substance. On the other hand, 75 g (37.1 %) of (Z)-ethyl 4-bromo-3-(1-ethoxycarbonylcyclopropyl)-2-fluoro-2-butenoate (the geometrical isomer of the title compound) was obtained as a yellow oily substance with the use of another eluent (n-hexane : ethyl acetate = 2 : 1).

(E)-isomer:

$^1$H-NMR (400 MHz, $CDCl_3$) δ: 1.23 (3H, t, J = 7.08 Hz), 1.38 (3H, t, J = 7.08 Hz), 1.52 - 1.62 (4H, br), 4.11 (2H, q, J = 7.08 Hz), 4.35 (2H, q, J = 7.08 Hz), 4.54 (2H, s).

(Z)-isomer:

$^1$H-NMR (400 MHz, $CDCl_3$) δ: 1.21 (3H, t, J = 7.08 Hz), 1.32 (3H, t, J = 7.08 Hz), 1.52 - 1.62 (4H, br), 4.11 (2H, q, J = 7.08 Hz), 4.13 (2H, s), 4.29 (2H, q, J = 7.08 Hz).

[Referential Example 3-4]

4-(1-Ethoxycarbonylcyclopropyl)-3-fluoro-1-[1-(S)-phenylethyl]-3-pyrrolin-2-one

To a solution of (E)-ethyl 4-bromo-3-(1-ethoxycarbonylcyclopropyl)-2-fluoro-2-butenoate (143 mmol) in ethanol (1000 ml) was added sodium hydrogencarbonate (30.08 g, 358 mmol). After dropping 1-(S)-phenylethylamine (20.31 ml, 158 mmol) thereinto at room temperature, the mixture was heated under reflux for 3 hours. Then the liquid reaction mixture was cooled and filtered through celite. The filtrate was concentrated under reduced pressure and the residue was subjected to flash silica gel column chromatography (eluent: n-hexane : ethyl acetate = 2 : 1) to give 36.95 g (81.2 %) of the title compound as an oily substance.

$^1$H-NMR (400 MHz, $CDCl_3$) δ: 1.16 (3H, t, J = 7.08 Hz), 1.22 - 1.30 (2H, m), 1.55 - 1.59 (2H, m), 1.62 (3H, d, J = 7.33 Hz), 3.76 (2H, ddd, J = 128.42, 18.07, 5.37 Hz), 4.08 (2H, q, J = 7.08 Hz), 5.56 (1H, q, J = 7.33 Hz).

[Referential Example 3-5]

4-(S)-(-1-Ethoxycarbonylcyclopropyl)-3-(S)-fluoro-1-[1-(S)-phenylethyl]-2-pyrrolidone

To a solution of 4-(i-ethoxycarbonylcyclopropyl)-3-fluoro-1-[1-(S)-phenylethyl]-3-pyrrolin-2-one (587 mg, 1.85 mmol) in ethanol (5 ml) was added Raney nickel (R-100, 2 ml). Under a hydrogen atmosphere of 5 kg/$cm^2$, the mixture was stirred at room temperature for 1 hour. Next, Raney nickel (R-100, 3 ml) was further added and stirring was continued under the same conditions for 2.5 hours. After eliminating the catalyst by filtering through celite (washed with ethanol), the filtrate was concentrated under reduced pressure. The residue was subjected to flash silica gel column chromatography (eluent: n-hexane : ethyl acetate = 3 : 1) to give 382 mg (64.6 %) of the title compound as a colorless oily substance. The $^1$H-NMR data of this compound agreed with the data of the compound obtained in Referential Example 2-2.

$^1$H-NMR (400 MHz, $CDCl_3$) δ: 0.57 - 0.63 (1H, m), 0.78 - 0.84 (1H, m), 1.07 - 1.13 (1H, m), 1.26 (3H, t, J = 7.09 Hz), 1.23 - 1.29 (1H, m), 1.54 (3H, d, J = 7.32 Hz), 2.59 (1H, t, J = 8.30 Hz), 3.05 (1H, dq, J = 28.81, 8.30 Hz), 3.25 (1H, t, J = 8.30 Hz), 4.00 - 4.16 (2H, m), 5.15 (1H, dd, J = 52.73, 6.35 Hz), 5.53 (1H, q, J = 7.32 Hz), 7.27 - 7.38 (5H, m).

[Referential Example 3-6]

4-(S)-(1-Carboxycyclopropyl)-3-(S)-fluoro-1-[1-(S)-phenylethyl]-2-pyrrolidone

4-(S)-(1-ethoxycarbonylcyclopropyl)-3-(S)-fluoro-1-[1-(S)-phenylethyl]-2-pyrrolidone (12.56 g, 39.33 mmol) was dissolved in ethanol (120 ml) and a 1 N aqueous solution of sodium hydroxide (120 ml) was dropped thereinto. After stirring at 40 °C for 6 hours, ethanol was evaporated under reduced pressure. The residue was washed with chloroform (100 ml x 2). Under ice cooling, the separated aqueous layer was acidified by dropping 1 N hydrochloric acid thereinto and then extracted successively with chloroform (300 ml x 2) and diethyl ether (300 ml). The combined organic layer was dried over anhydrous sodium sulfate and filtered. The filtrate was concentrated under reduced pressure to give 10.24 g (89.4 %) of the title compound as colorless needles.

$^1$H-NMR (400 MHz, $CDCl_3$) δ: 0 . 65 - 0.75 (1H, m) , 0.85 - 0.95 (1H, m), 1.15 - 1.25 (1H, m), 1.26 - 1.36 (1H, m), 1.54 (3H, d, J = 7.32 Hz), 2.60 (1H, t, J = 7.8 Hz), 3.01 (1H, dq, J = 27.83, 7.81 Hz), 3.28 (1H, t, J = 7.81 Hz), 5.16 (1H, dd, J = 52.74, 6.35 Hz), 5.53 (1H, q, J = 7.32 Hz), 7.27 - 7.38 (5H, m).

[Referential Example 3-7]

4-(R)-(1-tert-Butoxycarbonylaminocyclopropyl)-3-(S)-fluoro-1-[1-(S)-phenylethyl]-2-pyrrolidone

§ Process by Hoffman rearrangement:

To a solution of 4-(S)-(1-carboxycyclopropyl)-3-(S)-fluoro-1-[1-(S)-phenylethyl]-2-pyrrolidone (11.90 g, 40.85 mmol) in acetonitrile (160 ml) was added 1,1'-carbonyldiimidazole (13.25 g, 81.70 ml). The obtained mixture was stirred at room temperature for 30 minutes and then at 40 °C for additional 30 minutes. After cooling the liquid reaction mixture to room temperature, ammonia gas was bubbled thereinto for 30 minutes. After distilling off the solvent, chloroform (500 ml) was added to the residue followed by washing with water. The organic layer was dried over anhydrous sodium sulfate and filtered. The filtrate was concentrated under reduced pressure and the residue thus obtained was dissolved in tert-butyl alcohol (200 ml) and heated to 70 °C. Then lead tetraacetate (purity 90 % or more, 24.15 g, 49.02 mmol) was added thereto and the mixture was heated under reflux for 20 minutes. After cooling, sodium hydrogencarbonate was added followed by dilution with ethyl acetate (300 ml). Then the insoluble matters were filtered off and the filtrate was washed with a saturated aqueous solution of sodium bicarbonate. The organic layer was dried over anhydrous sodium sulfate and the solvent was evaporated under reduced pressure to give 10.52 g (71.7 %) of the title compound.

§ Process by Curtius rearrangement:

Under a nitrogen gas stream, toluene (100 ml) was added to 4-(S)-(1-carboxycyclopropyl)-3-(S)-fluoro-1-[1-(S)-phenylethyl]-2-pyrrolidone (3.66 g, 12.56 mmol). Next, triethylamine (3.50 ml, 25.13 mmol) was dropped thereinto at room temperature. When the liquid reaction mixture became a homogeneous system, diphenylphosphoric acid azide (2.71 ml, 12.56 mmol) was added and the resultant mixture was stirred at room temperature for 1 hour and then heated under reflux for 2 hours. Then tert-butyl alcohol (100 ml) was added to the liquid reaction mixture and the mixture was further heated under reflux for 21 hours. The liquid reaction mixture was cooled and concentrated under reduced

pressure. The residue thus obtained was subjected to flash silica gel column chromatography (eluent: n-hexane : ethyl acetate = 1 : 1) to give 3.30 g (72.5 %) of the title compound as a colorless oily substance.

$^1$H-NMR (400 MHz, $CDCl_3$) δ: 0.58 - 0.66 (1H, m), 0.70 - 0.82 (2H, m), 0.88 - 0.96 (1H, m), 1.31 (9H, s), 1.54 (3H, d, J = 7.33 Hz), 2.36 - 2.52 (1H, m), 2.86 (1H, t, J = 8.30 Hz), 3.32 (1H, t, J = 8.30 Hz), 4.99 (1H, dd, J = 52.73, 6.35 Hz), 4.99 (1H, s), 5.46 (1H, q, J = 7.33 Hz), 7.27 - 7.42 (5H, m).

[Referential Example 3-8]

4-(R)-1-tert-Butoxycarbonylaminocyclopropyl)-3-(S)-fluoro-1-[1-(S)-phenylethyl]pyrrolidine

Under a nitrogen atmosphere, a 1 M solution of borane-tetrahydrofuran complex in tetrahydrofuran (120 ml) was dropped under ice cooling into a solution of 4-(R)-(1-tert-butoxycarbonylaminocyclopropyl)-3-(S)-fluoro-1-[1-(S)-phenylethyl)-2-pyrrolidone in tetrahydrofuran (120 ml) and the mixture was stirred at room temperature for 5 hours. After evaporating the solvent under reduced pressure, a solvent mixture (200 ml) of ethanol with water (4 : 1) and triethylamine (20 ml) were added to the residue followed by heating under reflux for 2 hours. Then the liquid reaction mixture was concentrated under reduced pressure and chloroform (400 ml) was added to the residue. After washing with a saturated aqueous solution of sodium chloride, the organic layer was dried over anhydrous sodium sulfate and filtered. The filtrate was concentrated under reduced pressure and the residue was subjected to flash silica gel column chromatography (eluent: n-hexane : ethyl acetate = 1 : 2) to give 7.84 g (99.4 %) of the title compound as a colorless oily substance.

$^1$H-NMR (400 MHz, $CDCl_3$) δ: 0.54 - 0.62 (1H, m), 0.70 - 0.95 (3H, m), 1.35 (3H, d, J = 6.35 Hz), 1.42 (9H, s), 2.27 - 2.45 (2H, m), 2.46 - 2.56 (1H, m), 2.60 - 2.75 (1H, m), 3.00 - 3.15 (1H, m), 3.29 (1H, q, J = 6.35 Hz), 5.06 (1H, s), 5.05 - 5.20 (1H, m), 7.20 - 7.32 (5H, m).

[Example 5] ; for reference purpose

2-[4-(R)-(1-Aminocyclopropyl)-3-(S)-fluoro-1-fluoro-1-pyrrolidinyl]-6-fluoro-1-[2-(S)-fluoro-1-(R)-cyclopropyl]-1,4-dihydro-8-methoxy-4-oxoquinoline-3-carboxylic acid

4-(R)-(1-tert-butoxycarbonylaminocyclopropyl)-3-(S)-fluoro-1-[1-(S)-phenylethyl] pyrrolidine (6.32 g, 18.14 mmol) was dissolved in ethanol (150 ml). After adding 10 % palladium-carbon catalyst (moisture content: 50.2 %, 6.0 g), the mixture was stirred at 40 °C under a hydrogen atmosphere for 36 hours. After filtering off the catalyst through celite (washed with ethanol), the filtrate was concentrated under reduced pressure. The residue thus obtained was dissolved in dimethyl sulfoxide (20 ml) and 6,7-difluoro-1-[2-(S)-fluoro-1-(R)-cyclopropyl)-1,4-dihydro-8-methoxy-4-oxoquinoline-3-carboxylic acid-$BF_2$ chelate (4.37 g, 12.09 mmol) and triethylamine (5.05 ml, 36.23 mmol) were added thereto. Then the mixture was stirred at room temperature for 23 hours. After concentrating the liquid reaction mixture under reduced pressure, water was added to the residue. The solid matter thus precipitated was collected by filtration and washed with water. The obtained solid was suspended in a solution (400 ml) of methanol : water = 10 : 1. After adding triethylamine (20 ml), the mixture was heated under reflux for 4 hours. After allowing to cool, the liquid reaction mixture was concentrated under reduced pressure and the residue was dissolved in chloroform (500 ml) and washed with a 10 % aqueous solution of citric acid (500 ml). The organic layer was dried over anhydrous sodium sulfate. After filtering, the filtrate was concentrated under reduced pressure. Under ice cooling, conc. hydrochloric acid (30 ml) was dropped into the residue followed by stirring at room temperature for 2 hours. After adding 1 N hydrochloric acid (30 ml), the liquid reaction mixture was washed with chloroform (100 ml x 2) and its pH value was adjusted to 12.0 with an aqueous solution of sodium hydroxide. The pH value of this aqueous solution was adjusted to 7.4 with 1 N hydrochloric acid followed by extraction with chloroform (500 ml x 4). The combined organic layer was dried over anhydrous sodium sulfate and filtered. The filtrate was concentrated under reduced pressure and the residue was recrystallized from ethanol and dried under reduced pressure to give 4.09 g (77.3 %) of the title compound as pale yellow crystals.

Melting point: 218.5 - 219.8 °C (decomp.).

$^1$H-NMR (400 MHz, 0.1 N-NaOD) δ: 0.57 - 0.74 (4H, m), 1.32 - 1.45 (1H, m), 1.48 - 1.60 (1H, m), 2.20 - 2.38 (1H, m), 3.53 - 3.58 (1H, m), 3.58 (3H, s), 3.72 (1H, dd, J = 25.88, 13.19 Hz), 3.86 - 3.93 (1H, m), 4.00 - 4.18 (2H, m), 5.50 (1H, dm, J = 63.96 Hz), 5.51 (1h, brd. J = 54.68 Hz), 7.68 (1H, d, 14.16 Hz), 8.19 (1H, d, J = 3.91 Hz).

| Elemental analysis data: as $C_{21}H_{22}F_3N_3O_4$ | | | |
|---|---|---|---|
| calcd. | C, 57.66; | H, 5.07; | N, 9.61 |
| found | C, 57-52; | H, 5.02; | N, 9.48. |

[Example 6] ; for reference purpose

10-[4-(R)-(1-Aminocyclopropyl)-3-(S)-fluoro-1-pyrrolidinyl] 9-fluoro-2,3-dihydro-3-(S)-methyl-7-oxo-7H-pyrido [1,2,3-de][1,4]benzoxazine-6-carboxylic acid hydrochloride

4-(R)-(1-tert-butoxycarbonylaminocyclopropyl)-3-(S)-fluoro-1-[1-(S)=phenylethyl]pyrrolidine (1.12 g, 3.21 mmol) was dissolved in ethanol (20 ml). After adding 10 % palladium-carbon catalyst (moisture content: 50.2 %, 1.12 g), the mixture was stirred at 40 °C under a hydrogen atmosphere for 4 hours. After filtering off the catalyst through celite (washed with ethanol), the filtrate was concentrated under reduced pressure. The residue thus obtained was dissolved in dimethyl sulfoxide (10 ml) and 9.10-difluoro-2,3-dihydro-3-(S)-methyl-7-oxo-7H-pyrido{1,2,3-de][1,4]benzoxazine-6-carboxylic acid-$BF_2$ chelate (705 mg, 2.14 mmol) and triethylamine (0.60 ml, 4.29 mmol) were added thereto. Then the mixture was stirred at room temperature for 3 hours. After concentrating the liquid reaction mixture under reduced pressure, water was added to the residue. The yellow crystals thus precipitated were collected by filtration and washed with water. The obtained crystals were suspended in methanol (moisture content: 10 %, 100 ml). After adding triethylamine (5 ml), the mixture was heated under reflux for 14 hours. After allowing to cool, the liquid reaction mixture was concentrated under reduced pressure and the residue was dissolved in chloroform (200 ml) and washed with a 10 % aqueous solution of citric acid (200 ml). The organi layer was dried over anhydrous sodium sulfate. After filtering, the filtrate was concentrated under reduced pressure. Under ice cooling, conc. hydrochloric acid (10 ml was dropped into the residue followed by stirring at room temperature for 10 minutes. After adding 1 N hydrochloric acid (30 ml), the liquid reaction mixture was washed with chloroform (50 ml x 2) and its pH value was adjusted to 12.0 with an aqueous solution of sodium hydroxide. The pH value of this aqueous solution was adjusted to 7.4 with 1 N hydrochloric acid followed by extraction with chloroform (500 ml x 3). The combined organic layer was dried over anhydrous sodium sulfate and filtered. The filtrate was concentrated under reduced pressure and 1 N hydrochloric acid (5.0 ml) was dropped into the residue under ice cooling. After stirring at the same temperature for 5 minutes, the liquid reaction mixture was concentrated under reduced pressure (azeotropic distillation with ethanol, thrice). The residue was recrystallized from ethanol and dried under reduced pressure to give 685 mg (68.9 %) of the title compound as a pale yellow powder.

Melting point: 197 - 199 °C (decomp.).

$^1$H-NMR (400 MHz, 0.1 N-NaOD) δ: 0.59 - 0.68 (4H, m), 1.52 (3H, d, J = 6.84 Hz), 2.39 (1H, dt, J = 29.30, 7.81 Hz), 3.37 (1H, t, J = 7.81 Hz), 3.74 - 3.90 (3H, m), 3.95 (1H, t, J = 9.76 Hz), 4.36 (1H, d, J = 10.26 Hz), 4.53 (1H, d, J = 11.23 Hz), 4.62 (1H, q, J = 6.84 Hz), 5.34 (1H, brd, J = 54.02 Hz), 7.57 (1H, d, J = 13.67 Hz), 8.35 (1H, s).

| Elemental analysis data: as $C_{20}H_{21}F_2N_3O_4 \cdot HCl \cdot 1.25H_2O$ | | | |
|---|---|---|---|
| calcd. | C, 51.73; | H, 5.32; | N, 9.05 |
| found | C, 51.97; | H, 5.34; | N, 9.10. |

[Example 7]; for reference purpose

5-Amino-7-[4-(R)-(1-aminocyclopropyl)-3-(S)-fluoro-1-pyrrolidinyl]-6-fluoro-1-[2-(S)-fluoro-1-(R)-cyclopropyl]-1,4-dihydro-8-methyl-4-oxoquinoline-3-carboxylic acid hydrochloride

4-(R)-(1-tert-butoxycarbonylaminocyclopropyl)-3-(S)-fluoro-1-[1-(S)-phenylethyl]pyrrolidine (2.11 g, 6.06 mmol) was dissolved in ethanol (40 ml). After adding 10 % palladium-carbon catalyst (moisture content: 50.2 %, 2.11 g), the mixture was stirred under a hydrogen atmosphere for 5 hours. After filtering off the catalyst through celite (washed with ethanol), the filtrate was concentrated under reduced pressure. The residue thus obtained was dissolved in dimethyl sulfoxide (6 ml) and 5-amino-6,7-difluoro-1-[2-(S)-fluoro-1-(R)-cyclopropyl]-1,4-dihydro-8-methyl-4-oxoquinoline-3-carboxylic acid (1.26 g, 4.04 mmol) and triethylamine (14 ml) were added thereto. Then the mixture was stirred in a nitrogen atmosphere in an oil bath at 150 °C for 8 days. After allowing to cool, dimethyl sulfoxide was evaporated under reduced pressure and the residue was dissolved in chloroform (80 ml) and washed with a 10 % aqueous solution of citric acid (80 ml). The organic layer was dried over anhydrous sodium sulfate. After filtering, the filtrate was concentrated under reduced pressure. Under ice cooling, conc. hydrochloric acid (10 ml) was dropped into the residue followed by stirring at room temperature for 30 minutes. After adding 1 N hydrochloric acid (30 ml), the liquid reaction mixture was washed with chloroform (50 ml x 2) and its pH value was adjusted to 12.0 with an aqueous solution of sodium hydroxide. The pH value of this aqueous solution was adjusted to 7.4 with 1 N hydrochloric acid followed by extraction with chloroform (500 ml x 3). The combined organic layer was dried over anhydrous sodium sulfate and filtered. The filtrate was concentrated under reduced pressure and 1 N hydrochloric acid (5.0 ml) was dropped into the residue under ice cooling. After stirring at the same temperature for 5 minutes, the liquid reaction mixture was concentrated under reduced pressure (azeotropic distillation with ethanol, thrice). The residue was recrystallized from ethanol and dried under reduced pressure to give 561 mg (28.8 %) of the title compound as a pale yellow powder.

$^1$H-NMR (400 MHz, 0.1 N-NaOD) δ: 0.55 - 0.71 (4H, m), 1.10 - 1.21 (1H, m), 1.46 - 1.58 (1H, m), 2.30 (3H, s), 2.21 - 2.35 (1H, m), 3.32 (1H, t, J = 8.79 Hz), 3.49 (1H, dd, J = 25.88, 12.21 Hz), 3.85 - 3.97 (2H, m), 4.11 (1H, ddm, J = 40.77, 12.45 Hz), 4.97 (1H, dm, J = 70.31 Hz), 5.49 (1H, brd, J = 55.18 Hz), 8.27 (1H, d, J = 3.42 Hz).

| Elemental analysis data: as $C_{21}H_{23}F_3N_4O_3 \cdot HCl \cdot 0.5H_2O$ | | | |
|---|---|---|---|
| calcd. | C, 52.34; | H, 5.23; | N, 11.63 |
| found | C, 52.32; | H, 5.36; | N, 11.76. |

[Example 8]

8-Amino-10-[4-(R)-(1-aminocyclopropyl)-3-(S)-fluoro-1-pyrrolidinyl]-9-fluoro-2,3-dihydro-3-(S)-methyl-7-oxo-7H-pyrido[1,2,3-de][1,4]benzoxazine-6-carboxylic acid

4-(R)-(1-tert-Butoxycarbonylaminocyclopropyl)-3-(S)-fluoro-1-[1-(S)-phenylethyl]pyrrolidine (900 mg, 2.58 mmol) was dissolved in ethanol (20 ml). After adding 10 % palladium-carbon catalyst (moisture content: 50.2 %, 900 mg), the mixture was stirred under a hydrogen atmosphere for 4 hours. After filtering off the catalyst through celite (washed with ethanol), the filtrate was concentrated under reduced pressure. The residue thus obtained was dissolved in dimethyl sulfoxide (20 ml) and 8-amino-9,10-difluoro-2,3-dihydro-3-(S)-methyl-7-oxo-7H-pyrido[1,2,3-de][1,4]benzoxazine-6-carboxylic acid (593 mg, 2.00 mmol) and triethylamine (3 ml) were added thereto. Then the mixture was stirred in a nitrogen atmosphere in an oil bath at 100 °C for 25 hours. After allowing to cool, dimethyl sulfoxide was evaporated under reduced pressure and the residue was dissolved in chloroform (100 ml) and washed with a 10 % aqueous solution of citric acid (80 ml). The organic layer was dried over anhydrous sodium sulfate. After filtering, the filtrate was concentrated under reduced pressure. Under ice cooling, conc. hydrochloric acid (10 ml) was dropped intc the residue followed by stirring at room temperature for 30 minutes. After adding 1 N hydrochloric acid (30 ml), the liquid reaction mixture was washed with chloroform (50 ml x 4) and its pH value was adjusted to 12.0 with an aqueous solution of sodium hydroxide. The pH value of this aqueous solution was adjusted to 7.4 with 1 N hydrochloric acid followed by extraction with chloroform (500 ml x 3). The combined organic layer was dried over anhydrous sodium sulfate and filtered. The filtrate was concentrated under reduced pressure and the residue was recrystallized from ethanol and dried under reduced pressure to give 640 mg (76.0 %) of the title compound as a yellow powder.

Melting point: 247 - 250 °C (decomp.).

$^1$H-NMR (400 MHz, 0.1 N-NaOD) δ: 0.53 - 0.68 (4H, m), 1.45 (3H, d, J = 6.98 Hz), 2.18 (1H, dt, J = 36.14, 7.81 Hz), 3.38 (1H, t, J = 7.81 Hz), 3.66 (1H, dd, J = 25.63, 12.94 Hz), 3.82 (1H, t, J = 10.01 Hz), 3.99 - 4.12 (3H, m), 4.32 (1H, d, J = 11.24 Hz), 4.44 (1H, d, J = 6.98 Hz), 5.43 (1H, d, J = 54.69 Hz), 8.13 (1H, s).

| Elemental analysis data: as $C_{20}H_{22}F_2N_4O_4$ | | | |
|---|---|---|---|
| calcd. | C, 57.14; | H, 5.27; | N, 13.33 |
| found | C, 56.86; | H, 5.26; | N, 13.39. |

[Referential Example 4-1]

Ethyl 1-acetylcyclobutanecarboxylate

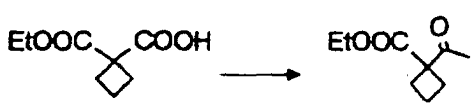

Ethyl hydrogen 1,1-cyclobutanecarboxylate (64.43 g, 374 mmol) was dissolved in methylene chloride (500 ml). Under ice cooling, oxalyl chloride (65.29 ml, 748 mmol) was added thereto followed by the addition of a catalytic

amount of N,N-dimethylformamide. Then the resultant mixture was stirred at room temperature for 1.5 hours. After evaporating the solvent, the residue was subjected to azeotropic distillation together with toluene twice to give an acid chloride.

On the other hand, copper (I) iodide (85.52 g, 449 mmol) was suspended in tetrahydrofuran (1 l) under a nitrogen gas stream. At - 20 °C, a 1.4 M solution (294 ml) of methyllithium in diethyl ether was dropped thereinto and the mixture was stirred at the same temperature for 1 hour. Subsequently, the above-mentioned acid chloride was dissolved in tetrahydrofuran (300 ml) and dropped thereinto at the same temperature followed by stirring for 1.5 hours. After the completion of the reaction, the reaction temperature was brought back to room temperature and a 10 % aqueous solution of citric acid (500 ml) was added to the mixture. After evaporating tetrahydrofuran, ethyl acetate (1 l) was added to the residue. Then the insoluble matters were filtered off and the residue was washed successively with a 5 % aqueous solution of sodium thiosulfate (300 ml) and a saturated aqueous solution of sodium chloride (300 ml) and dried over anhydrous sodium sulfate. After evaporating the solvent, the obtained residue was subjected to silica gel column chromatography (eluent: n-hexane : ethyl acetate = 4 : 1) to give 56.70 g (89 %) of the title compound as an oily substance.

$^1$H-NMR (400 MHz, $CDCl_3$) δ: 1.27 (3H, t, J = 7.33 Hz), 1.82 - 2.01 (2H, m), 2.12 (3H, s), 2.45 - 2.55 (4H, m), 4.20 - 4.24 (2H, m).

[Referential Example 4-2]

Ethyl 1-ethoxycarbonyl-β-hydroxy-β-methylcyclobutylpropanoate

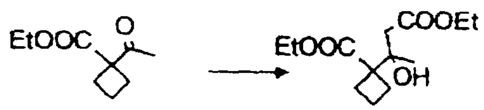

Ethyl 1-acetylcyclobutanecarboxylate (13.79 g, 81 mmol) was dissolved in tetrahydrofuran (50 ml) and zinc powder (10.59 g) and a catalytic amount of iodine were added thereto. While heating under reflux, a solution (100 ml) of ethyl bromoacetate (13.48 ml, 121 mmol) in tetrahydrofuran was dropped thereinto. Then the liquid reaction mixture was heated under reflux for additional 1 hour and allowed to cool. After adding 1 N hydrochloric acid (100 ml), the solvent was evaporated and ethyl acetate (500 ml) was added. The insoluble matters were filtered off, washed with a saturated aqueous solution of sodium chloride (300 ml) and dried over anhydrous sodium sulfate. After evaporating the solvent, the title compound was obtained in a quantitative amount as an oily substance.

$^1$H-NMR (400 MHz, $CDCl_3$) δ: 1.24 - 1.32 (9H, m), 1.73 - 1.87 (2H, m), 2,21 - 2.34 (2H, m), 2.41 - 2.57 (5H, m), 4.16 - 4.21 (4H, m).

[Referential Example 4-3]

(E)-Ethyl 3-(1-ethoxycarbonylcyclobutyl)-2-butenoate

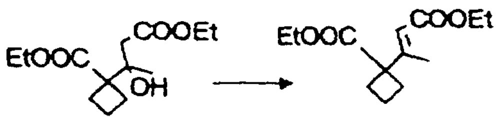

Ethyl 1-ethoxycarbonyl-β-hydroxy-β-methylcyclobutylpropanoate (22.27 g, 86 mmol) was dissolved in pyridine (42 ml) and thionyl chloride (8.18 ml, 112 mmol) was dropped thereinto at - 10°C. After the completion of the reaction, the liquid reaction mixture was poured into ice water (250 ml) and extracted with ethyl acetate (100 ml x 3).

The combined organic layer was washed with 1 N hydrochloric acid (100 ml) and a saturated aqueous solution of sodium chloride (100 ml) and dried over anhydrous sodium sulfate. After evaporating the solvent, the residue thus obtained was dissolved in methylene chloride (250 ml). Then 1,8-diazabicyclo[5,4,0]-7-undecene (12.89 ml) was dropped thereinto at 0 °C and the obtained mixture was stirred at room temperature for 18 hours. After the completion of the reaction, the solvent was evaporated. Ice water (100 ml) was added to the residue followed by extraction with ethyl acetate (200 ml x 3). The combined organic layer was washed with 1 N hydrochloric acid (100 ml) and a saturated aqueous solution of sodium chloride (100 ml) and dried over anhydrous sodium sulfate. After evaporating the solvent, the residue was subjected to silica gel column chromatography (eluent: n-hexane : ethyl acetate = 4 : 1) to give 16.91 g (82 %) of the title compound as an oily substance.

$^1$H-NMR (400 MHz, $CDCl_3$) δ: 1.24 (3H, t, J = 6.83 Hz), 1.29 (3H, t, J = 7.32 Hz), 1.74 - 1.80 (2H, m), 1.94 - 2.04 (1H, m), 2.07 (3H, d, J = 1.47 Hz), 2.12 - 2.30 (2H, m), 2.12 - 2.30 (2H, m), 2.50 - 2.57 (2H, m), 4.13 - 4.20 (4H, m).

[Referential Example 4-4]

4-(1-Ethoxycarbonylcyclobutyl)-1-(1-(S)-phenylethyl]-3-pyrrolin-2-one

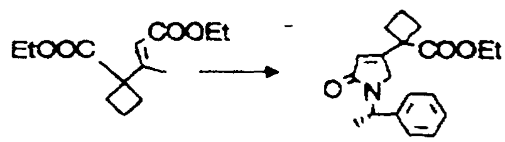

(E)-Ethyl 3-(1-ethoxycarbonylcyclobutyl)-2-butenoate (16.91 g, 70 mmol) was dissolved in chloroform (180 ml) and N-bromosuccinimide (12.53 g, 70 mmol) and a catalytic amount of azobisisobutyronitrile were added thereto. The obtained mixture was heated under reflux for 18 hours. After the completion of the reaction, the solvent was evaporated and carbon tetrachloride (100 ml) was added to the residue. Then the insoluble matters were filtered off and the filtrate was concentrated. The residue was dissolved in ethanol (100 ml) and sodium hydrogencarbonate (11.82 g, 140 mmol) was added thereto. Next, (S)-phenylethylamine (9.87 ml, 77 mmol) was dropped thereinto at room temperature. After the completion of the addition, the resultant mixture was heated under reflux for 3 hours. After the completion of the reaction, the solvent was evaporated and methylene chloride (300 ml) was added to the residue. The insoluble matters were filtered off and the solvent was evaporated. The residue thus obtained was subjected to silica gel column chromatography (eluent: n-hexane : ethyl acetate = 1 : 1) to give 19.57 g (43 %) of the title compound as an oily substance.

$^1$H-NMR (400 MHz, $CDCl_3$) δ: 1.17 (3H, t, J = 7.33 Hz), 1.74 - 1.80 (2H, m), 1.59 (3H, d,J = 6.84 Hz), 1.84 - 2.01 (2H, m), 2.15 - 2.28 (2H, m), 2.60 - 2.69 (2H, m), 3.56 (2H, d, J = 9.04 Hz), 3.88 (2H, d, J = 9.04 Hz), 4.13 (2H, q, J = 7.32 Hz), 5.50 - 5.59 (1H, m), 6.03 (2H, s), 7.26 - 7.35 (5H, m).

[Referential Example 4-5]

4-(1-Ethoxycarbonylcyclobutyl)-1-[(S)-phenylethyl]-2-pyrrolidone

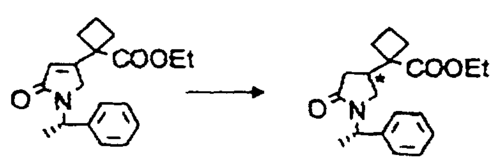

4-(1-Ethoxycarbonylcyclobutyl)-1-[(S)-phenylethyl]-3-pyrrolin-2-one (9.57 g, 31 mmol) was dissolved in ethanol (150 ml) and platinum oxide (230 mg) was added thereto. The obtained mixture was stirred in a hydrogen atmosphere for 18 hours. After the completion of the reaction, the liquid reaction mixture was filtered and concentrated. The residue thus obtained was subjected to silica gel column chromatography thrice (eluent: n-hexane : ethyl acetate = 1 : 1) to give 2.3 g (24 %) of an optical isomer A of the title compound and 7.1 g (74 %) of another optical isomer B thereof each as an oily substance.

Optical isomer A:

$^1$H-NMR (400 MHz, $CDCl_3$) δ: 1.26 (3H, t, J = 6.83 Hz), 1.49 (2H, d, J = 7.32 Hz), 1.83 - 1.95 (4H, m), 2.38 - 2.54 (4H, m), 2.66 - 2.74 (1H, m), 3.01 (1H, t, 8.30 Hz), 3.14 (1H, d, J = 5.86, 9.77 Hz), 4.09 - 4.18 (2H, m), 5.48 (1H, dd, J = 7.32, 14.16 Hz), 7.27 - 7.35 (5H, m).

Optical isomer B:

$^1$H-NMR (400 MHz, $CDCl_3$) δ: 1.17 (3H, t, J = 7.32 Hz), 1.52 (2H, d, J = 7.33 Hz), 1.68 - 1.92 (4H, m), 2.23 - 2.43 (3H, m), 2.50 - 2.57 (1H, m), 2.73 - 2.86 (2H, m), 3.37 (1H, t, J = 8.30 Hz), 4.05 (2H, q, J = 7.32 Hz), 5.50 (1H, dd, J = 7.32, 14.16 Hz), 7.24 - 7.35 (5H, m).

[Referential Example 4-6]

trans 4-(1-Ethoxycarbonylcyclobutyl)-3-fluoro-1-(1-(S)-phenylethyl]-2-pyrrolidone (optical isomer B)

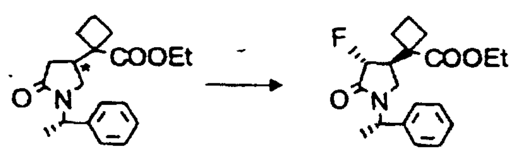

Under a nitrogen atmosphere, diisopropylamine (2.55 ml, 18.2 mmol) was dissolved in dry tetrahydrofuran (120 ml). After cooling the solution to - 78 °C, a 1.63 M solution of n-butyllithium in n-hexane (11.2 ml, 18.2 mmol) was dropped thereinto over 10 minutes. Then the liquid reaction mixture was stirred at 0 °C for 15 minutes and cooled to - 78 °C. Next, a solution (30 ml) of 4-(1-ethoxycarbonylcyclopropyl)-1-[1-(S)-phenylethyl]-2-pyrrolidone (optical isomer B; 4.42 g, 14.01 mmol) in dry tetrahydrofuran was dropped thereinto over 15 minutes. The liquid reaction mixture was stirred at - 78 °C for 1 hour. Then a solution (25 ml) of N-fluorobenzenedisulfonimide (7.07 g, 22,42 mmol) in dry tetrahydrofuran was dropped thereinto over 5 minutes. The liquid reaction mixture was stirred at - 78 °C for 30 minutes and then heated to room temperature followed by stirring for additional 20 minutes. Under ice cooling, a saturated aqueous solution of ammonium chloride (200 ml) was added to the liquid reaction mixture. After evaporating tetrahydrofuran, the aqueous layer was extracted with ethyl acetate (200 ml x 2). The combined organic layer was washed with water (200 ml x 3), dried over anhydrous sodium sulfate and filtered. The filtrate was concentrated under reduced pressure and the residue was subjected to silica gel column chromatography (eluent: n-hexane : ethyl acetate = 1 : 1) to give 3.88 g (83 %) of the title compound as an oily substance.

$^1$H-NMR (400 MHz, $CDCl_3$) δ: 1.14 (3H, t, J = 6.83 Hz), 1.57 (2H, d, J = 6.83 Hz), 1.88 - 2.08 (4H, m), 2.33 - 2.58 (3H, m), 2.81 - 2.92 (1H, m), 3.42 (1H, t, J = 9.77 Hz), 3.93 - 4.07 (2H, m), 5.18 (1H, dd, J = 6.83, 53.22 Hz), 5.51 (1H, dd, J = 7.32, 14.16 Hz), 7.25 - 7.34 (5H, m).

[Referential Example 4-7]

cis 4-(1-Ethoxycarbonylcyclobutyl)-3-fluoro-1-[1-(S)-Phenylethyl]-2-pyrrolidone (optical isomer B)

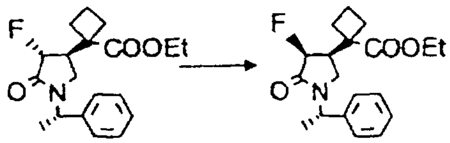

Under a nitrogen atmosphere, diisopropylamine (2.97 ml, 21.19 mmol) was dissolved in dry tetrahydrofuran (30 ml). After cooling the solution to - 78 °C, a 1.63 M solution of n-butyllithium in n-hexane (10.8 ml, 17.60 mmol) was dropped thereinto over 5 minutes. Then the liquid reaction mixture was stirred at 0 °C for 15 minutes and cooled to - 78 °C. Next, a solution (30 ml) of trans 4-(1-ethoxycarbonylcyclopropyl)-3-fluoro-1-[1-(S)-phenylethyl]-2-pyrrolidone (optical isomer B; 4.71 g, 14.13 mmol) in dry tetrahydrofuran was dropped thereinto over 5 minutes. The liquid reaction mixture was stirred at - 78 °C for 3 minutes. Then it was dropped into a solution (40 ml) of 2,6-di-tert-butylphenol (4.37 g, 21.18 mmol) in dry tetrahydrofuran over 5 minutes. The liquid reaction mixture was stirred at - 78 °C for 10 minutes and a saturated aqueous solution of ammonium chloride (200 ml) was added thereto. Next, the liquid reaction mixture was brought back to room temperature and the organic layer was taken up. The aqueous layer was extracted with chloroform (100 ml x 2). The combined organic layer was washed with water (100 ml x 2), dried over anhydrous sodium sulfate and filtered. The filtrate was concentrated under reduced pressure and the residue was subjected to silica gel column chromatography to give 1.96 g (42 %) of the starting compound (eluent: n-hexane : ethyl acetate = 2 : 1) and 1.79 g (38 %) of the title compound (eluent: n-hexane : ethyl acetate = 3 : 2) each as an oily substance.

$^1$H-NMR (400 MHz, $CDCl_3$) δ: 1.22 (3H, t, J = 6.83 Hz), 1.56 - 1.58 (3H, d, J = 6.83 Hz), 1.84 - 2.42 (6H, m), 2.83-2.97 (1H, m), 3.15 - 3.24 (1H, m), 3.36 - 3.43 (1H, m), 4.11 - 4.17 (2H, m), 5.07 (1H, dd, J = 6.83, 52.24 Hz), 5.56 (1H, q, J = 7.33 Hz), 7.26 - 7.36 (5H, m).

[Referential Example 4-8]

cis 4-(1-Carboxycyclobutyl)-3-fluoro-1-[1-(S)-phenylethyl)-2-pyrrolidone (optical isomer B)

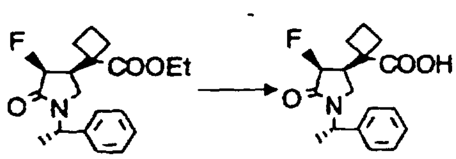

cis 4-(1-Ethoxycarbonylcyclobutyl)-3-fluoro-1-[1-(S)-phenylethyl]-2-pyrrolidone (optical isomer B; 1.79 g, 5.37 mmol) was dissolved in methanol (10 ml) and a 1 N aqueous solution of sodium hydroxide was dropped thereinto. The liquid reaction mixture was stirred at 40 °C for 18 hours and then methanol was evaporated under reduced pressure. Water (50 ml) was added to the residue followed by washing with chloroform (100 ml). The aqueous layer thus separated was acidified by dropping 1 N hydrochloric acid thereinto and then extracted with chloroform (100 ml x 2). The combined organic layer was dried over anhydrous sodium sulfate and filtered. The filtrate was concentrated under reduced pressure to give the title compound in a quantitative amount as a crude product.

[Referential Example 4-9]

cis 4-(1-tert-Butoxycarbonylaminocyclobutyl)-3-fluoro-1-[1-(S)-phenylethyl)-2-pyrrolidone (optical isomer B)

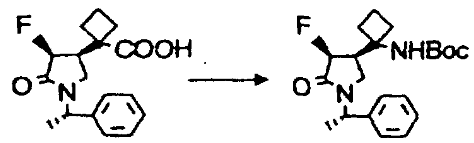

cis 4-(1-Carboxycyclobutyl)-3-fluoro-1-(1-(S)-phenylethyl]-2-pyrrolidone (optical isomer B; 1.92 g, 6.29 mmol) was dissolved in dry acetonitrile (30 ml) and N,N'-carbonyldiimidazole (1.33 g, 8.20 mmol) was added thereto. The liquid reaction mixture was stirred at 60 °C for 1 hour. Then ammonia was bubbled thereinto at room temperature for 10 minutes. After concentrating the liquid reaction mixture under reduced pressure, water (100 ml) was added to the residue followed by washing with chloroform (100 ml x 2). The combined organic layer was dried over anhydrous sodium sulfate and filtered. The filtrate was concentrated under reduced pressure and the residue thus obtained was dissolved in tert-butyl alcohol (50 ml). After adding lead tetraacetate (6.32 g, 14.25 mmol), the mixture was heated under reflux for 1 hour. The liquid reaction mixture was then allowed to cool followed by the addition of diethyl ether (50 ml) and sodium hydrogencarbonate (6 g). Next, it was stirred at room temperature for 10 minutes and filtered. The filtrate was concentrated under reduced pressure and ethyl acetate (100 ml) was added to the residue. The obtained mixture was washed with a saturated aqueous solution of sodium bicarbonate, dried over anhydrous sodium sulfate and filtered. The filtrate was concentrated under reduced pressure to give 1.74 g (65 %) of the title compound as an oily substance.

$^1$H-NMR (400 MHz, $CDCl_3$) δ: 1.40 (9H, s), 1.92 - 2.21 (6H, m), 3.04 - 3.12 (1H, m), 3.31 - 3.38 (1H, m), 4.87 (1H, brs), 5.01 (1H, dd, J = 5.86, 52.73 Hz), 5.52 (1H, dd, J = 7.32, 14.16 Hz), 7.30 - 7.38 (5H, m).

[Referential Example 4-10]

cis 1-[1-(S)-phenylethyl]-4-(1-tert-butoxycarbonylaminocyclobutyl)-3-fluoropyrrolidone (optical isomer B)

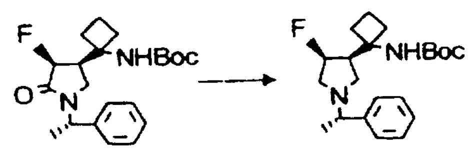

cis 4-(1-tert-Butoxycarbonylaminocyclobutyl)-3-fluoro-1-[1-(S)-phenylethyl)-2-pyrrolidone (optical isomer B; 1.74 g, 4.62 mmol) was dissolved in tetrahydrofuran (30 ml). At 0 °C, borane-tetrahydrofuran complex salt (13.86 ml) was added thereto and the resultant mixture was stirred at room temperature for 2 days. After the completion of the reaction, the solvent was evaporated and water (50 ml) was added to the residue followed by extraction with chloroform (100 ml x 2). The combined organic layer was dried over anhydrous sodium sulfate and filtered. The filtrate was concentrated under reduced pressure and the residue thus obtained was dissolved in 80 % moisture-containing methanol (40 ml). After adding triethylamine (10 ml), the mixture was beated under reflux for 2 hours. After evaporating the solvent, the obtained residue was subjected to silica gel column chromatography (eluent: n-hexane : ethyl acetate = 2 : 1) to give 1.13 g (67 %) of the title compound as an oily substance.

$^1$H-NMR (400 MHz, $CDCl_3$) δ: 1.37 (3H, d, J = 6.35 Hz), 1.44 (9H, s), 1.65 - 2.58 (7H, m), 2.70 - 2.92 (4H, m), 3.27 - 3.32 (1H, m), 5.14 (1H, brd), 5.53 (1H, brs), 7.22 - 7.33 (5H, m).

[Example 9] ; for reference purpose

5-Amino-7-[cis 4-(1-aminocyclobutyl)-3-fluoro-1-pyrrolidinyl)-6-fluoro-1-[2-(S)-fluoro-1-(R)-cyclopropyl]-1,4-dihydro-8-methyl-4-oxoguinoline-3-carboxylic acid (optical isomer B)

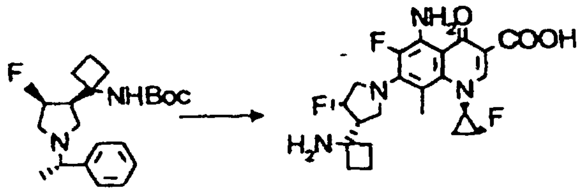

cis 1-[1-(S)-phenylethyl]-4-(1-tert-butoxycarbonylaminocyclobutyl)-3-fluoropyrrolidine (optical isomer B; 1.13 g, 3.12 mmol) was dissolved in ethanol (20 ml). After adding 10 % palladium-carbon catalyst (moisture content: 55.6 %, 1.0 g), the mixture was stirred under a hydrogen atmosphere at 50 °C for 18 hours. After filtering off the catalyst through celite (washed with methanol), the filtrate was concentrated under reduced pressure. The residue thus obtained was dissolved in dimethyl sulfoxide (10 ml) and 5-amino-6,7-difluoro-1-[2-(S)-fluoro-1-(R)-cyclopropyl]-1,4-dihydro-8-methyl-4-oxoquinoline-3-carboxylic acid (1.18 g, 3.78 mmol) and triethylamine (5 ml) were added thereto. Then the mixture was stirred in a nitrogen atmosphere at 140 °C for 4 days. After allowing to cool, dimethyl sulfoxide was evaporated under reduced pressure and the residue was dissolved in chloroform (50 ml) and washed successively with a 10 % aqueous solution of citric acid (50 ml) and a saturated aqueous solution of sodium chloride (100 ml). The organic layer was dried over anhydrous sodium sulfate. After filtering, the filtrate was concentrated under reduced pressure. The residue was then subjected to flash silica gel column chromatography (eluent: chloroform : methanol = 9 : 1) and the eluate was concentrated under reduced pressure. Under ice cooling, conc. hydrochloric acid (5 ml) was dropped into the residue followed by stirring at room temperature for 30 minutes. After adding 1 N hydrochloric acid (30 ml), the liquid reaction mixture was washed with chloroform (50 ml x 2) and its pH value was adjusted to 12.0 with an aqueous solution of sodium hydroxide. The liquid reaction mixture was washed with chloroform (100 ml) and then its pH value was adjusted to 7.4 with 1 N hydrochloric acid followed by extraction with chloroform (150 ml x 3). The combined organic layer was dried over anhydrous sodium sulfate and filtered. The filtrate was concentrated under reduced pressure and the residue was purified by preparative TLC (developed at the bottom layer of chloroform : methanol : water = 7 : 3 : 1) to give the title compound as a crude product. After recrystallized from ethanol/ether, 157 mg (17 %) of the title compound was obtained.

Melting point: 177 - 184 °C.

$^1$H-NMR (400 MHz, $CDCl_3$) δ: 1.16 - 2.34 (13H, m), 2.47 - 2.60 (1H, m), 3.35 (1H, t, J = 8.79 Hz), 3.53 (1H, q, J = 12.21 Hz), 3.78 - 3.83 (1H, m), 4.09 - 4.21 (2H, m), 4.76 - 4.95 (1H, m), 5.42 (1H, dt, J = 3.41, 55.18 Hz), 6.53 (2H, brs), 8.60 (1H, d, J = 3.41 Hz).

| Elemental analysis data: as $C_{22}H_{25}F_3N_4O_3 \cdot 0.5H_2O$ | | | |
|---|---|---|---|
| calcd. | C, 57.51; | H, 5.70; | N, 12.19 |
| found | C, 57.59; | H, 5.52; | N, 11.89. |

[Acute Toxicity]

A solution of 5-amino-7-[4-(R)-(1-aminocyclopropyl)-3-(S)-fluoro-1-pyrrolidinyl]-6-fluoro-1-(2-(S)-fluoro-1-(R)-cyclopropyl]-1,4-dihydro-8-methyl-4-oxoquinoline-3-carboxylic acid (abbreviated as cis) hydrochloride (Example 2) or 5-amino-7-[4-(R)-(1-aminocyclopropyl)-3-(R)-fluoro-1-pyrrolidinyl]-6-fluoro-1-[2-(S)-fluoro-1-(R)-cyclopropyl]-1,4-dihydro-8-methyl-4-oxoquinoline-3-carboxylic acid (abbreviated as trans) hydrochloride in distilled water for injection was administered intravenously to Slc:ddy male mice (five mice per group) and the symptoms are observed. The results are as follows.

Results;

| Compound | Dose | Number of death |
|---|---|---|
| cis | 150 mg/kg | 0/5 |
| trans | 150 mg/kg | 2/2* |
| trans | 100 mg/kg | 2/2* |
| trans | 50 mg/kg | 0/5 |

"*": The mouse died immediately after the administration is finished. The test was stopped at the second mouse died.

The same result was obtained for other compounds (Examples 1, 3, 4, 5, 8 and 9) of the present invention at a dose of 150 mg/kg in the test illustrated above.

| Strain/Compound (Example No.) | 2 | 3 | 5 |
|---|---|---|---|
| E. coli, NIHJ | ≤ 0.003 | 0.006 | ≤ 0.003 |
| S. flexneli, 2A 5503 | ≤ 0.003 | 0.006 | 0.006 |
| Pr. vulgaris, 08601 | 0.013 | 0.05 | 0.006 |
| Pr. mirabilis, IFO-3849 | 0.025 | 0.10 | 0.05 |
| Ser. marcescens, 10100 | 0.05 | 0.20 | 0.10 |
| Ps. aeruginosa, 32104 | 0.10 | 0.39 | 0.20 |
| Ps. aeruginosa, 32121 | 0.05 | 0.20 | 0.10 |
| X. maltophilia, 11D-1275 | 0.05 | 0.20 | 0.20 |
| S. aureus, 209P | ≤ 0.003 | ≤ 0.003 | ≤ 0.003 |
| S. epidermidis, 56500 | ≤ 0.003 | 0.006 | 0.013 |
| Str. pyogenes, G-36 | ≤ 0.003 | 0.013 | 0.006 |
| Str. faecalis, ATCC-19433 | 0.025 | 0.05 | 0.025 |
| S. aureus, 870307 | 0.025 | 0.025 | 0.025 |

| Strain/Compound (Example No.) | 6 | 8 |
|---|---|---|
| E. coli, NIHJ | 0.010 | 0.006 |
| S. flexneli, 2A 5503 | 0.010 | 0.013 |
| Pr. vulgaris, 08601 | 0.025 | 0.10 |
| Pr. mirabilis, IFO-3849 | 0.10 | 0.10 |
| Ser. marcescens, 10100 | 0.10 | 0.20 |
| Ps. aeruginosa, 32104 | 0.39 | 0.39 |
| Ps. aeruginosa, 32121 | 0.10 | 0.20 |

| | | |
|---|---|---|
| X. maltophilia, 11D-1275 | 0.39 | 0.39 |
| S. aureus, 209P | 0.006 | 0.006 |
| S. epidermidis, 56500 | 0.025 | 0.013 |
| Str. pyogenes, G-36 | 0.010 | 0.025 |
| Str. faecalis, ATCC-19433 | 0.05 | 0.05 |
| S. aureus, 870307 | 0.20 | 0.20 |

INDUSTRIAL APPLICABILITY:

Because of being excellent in antimicrobial activity and safety, the compounds of the present invention are useful as drugs.

**Claims**

**1.** A compound represented by formula (I), its salts and hydrates thereof:

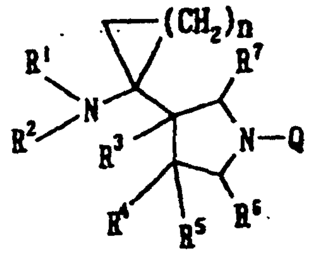

(I)

wherein

$R^1$ represents a hydrogen atom or an alkyl group having 1 to 6 carbon atoms;

$R^2$ represents a hydrogen atom or an alkyl group having 1 to 6 carbon atoms, provided that said alkyl group may have one or more substituents selected from the group consisting of a hydroxyl group, a halogen atom and an alkoxyl group having 1 to 6 carbon atoms;

$R^3$ and $R^5$, each represents a hydrogen atom;

$R^4$ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, provided that said alkyl group may have one or more substituents selected from the group consisting of a hydroxyl group, a halogen atom and an alkoxyl group having 1 to 6 carbon atoms and $R^4$ and the substituent on the pyrrolidine ring of the following formula:

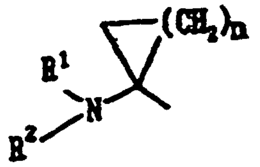

are located in the cis-configuration;

$R^6$ and $R^7$, each independently represents a hydrogen atom or an alkyl group having 1 to 6 carbon atoms;

n is an integer of from 1 to 3; and

Q represents a partial structure represented by the following formula:

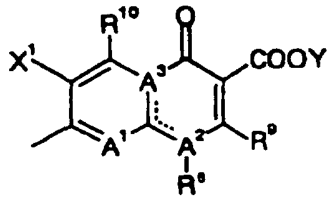

wherein $R^8$ represents an alkyl group having 1 to 6 carbon atoms, an alkenyl group having 2 to 6 carbon atoms, a halogenoalkyl group having 1 to 6 carbon atoms, a substituted or unsubstituted cyclic alkyl group having 3 to 6 carbon atoms, a substituted or unsubstituted aryl group, a substituted or unsubstituted heteroaryl group, an alkoxyl group having 1 to 6 carbon atoms or an alkylamino group having 1 to 6 carbon atoms;

$R^9$ represents a hydrogen atom or an alkylthio group having 1 to 6 carbon atoms;

$R^9$ and $R^8$ may form together with a part of the mother nucleus a cyclic structure optionally containing a sulfur atom as a constituting atom thereof and optionally having an alkyl group having 1 to 6 carbon atoms as a substituent;

$R^{10}$ represents a hydrogen atom, an amino group, a hydroxyl group, a thiol group, a halogenomethyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms, provided that said amino group may have one or more substituents selected from the group consisting of a formyl group, an alkyl group having 1 to 6 carbon atoms and an acyl group having 2 to 5 carbon atoms;

$X^1$ represents a halogen atom or a hydrogen atom;

$A^1$ represents a nitrogen atom or a partial structure represented by the following formula (II):

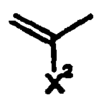

(II)

wherein $X^2$ represents a hydrogen atom, an amino group, a halogen atom, a cyano group, a halogenomethyl group, a halogenomethoxyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms, provided that said amino group may have one or more substituents selected from the group consisting or a formyl group, an alkyl group having 1 to 6 carbon atoms and an acyl group having 2 to 5 carbon atoms, and $X^2$ and $R^8$ may form together with a part of the mother nucleus a cyclic structure optionally containing an oxygen atom, a nitrogen atom or a sulfur atom as a constituting atom thereof and optionally having an alkyl group having 1 to 6 carbon atoms as a substituent;

$A^2$ and $A^3$, each represents a nitrogen atom or a carbon atom, provided that $A^2$ and $A^3$ may form together with the carbon atom to which they are bonded a partial structure represented by the following formula:

$$>C=C(A^1=)-N(R^8)-$$

or a partial structure represented by the following formula:

$$>N-C(A^1=)=C(R^8)-;$$

and

Y represents a hydrogen atom, a phenyl group, an acetoxymethyl group, a pivaloyloxymethyl group, an ethoxycarbonyl group, a choline group, a dimethylaminoethyl group, a 5-indanyl group, a phthalidinyl group, a 5-alkyl-2-oxo- 1,3-dioxol-4-ylmethyl group, a 3-acetoxy-2-oxobutyl group, an alkyl group having 1 to 6 carbon atoms, an alkoxylmethyl group having 2 to 7 carbon atoms or a phenylalkyl group composed of an alkyl group having 1 to 6 carbon atoms and a phenyl group, provided that the following compounds are excluded:

5-amino-7-[cis-4-(1-aminocyclopropyl)-3-hydroxymethyl-1-pyrrolidinyl]-1-cyclopropyl-6-fluoro-1,4-dihydro-8-methyl-4-oxoquinoline-3-carboxylic acid;
5-amino-7-[4-(R)-(1-aminocyclopropyl)-3-(S)-fluoro-1-pyrrolidinyl]-6-fluoro-1-[2-(S)-fluoro-1-(R)-cyclopropyl]-1,4-dihydro-8-methyl-4-oxoquinoline-3-carboxylic acid hydrochloride;
5-amino-7-[4-(R)-(1-aminocyclopropyl)-3-(S)-fluoro-1-pyrrolidinyl]-6-fluoro-1-[2-(S)-fluoro-1-(R)-cyclopropyl]-1,4-dihydro-8-methoxy-4-oxoquinoline-3-carboxylic acid;
10-{4-(R)-(1-aminocyclopropyl)-3-(S)-nuoro-1-pyrrolidinyl]-9-fluoro-2,3-dihydro-3-(S)-methyl-7-oxo-7H-pyrido[ 1.2.3-de] [1.4]benzoxazin-6-carboxylic acid;
7-[4-(R)-(1-aminocyclopropyl)-3-(S)-nuoro-1-pyrrolidinyl]-6-fluoro-1-[2-(S)-fluoro-1-(R)-cyclopropyl]-1,4-dihydro-8-methoxy-4-oxoquinoline-3-carboxylic acid;
5-amino-7-[cis-4-(1-aminocyclobutyl)-3-fluoro-1-pyrrolidinyl]-6-fluoro-1-[2-(S)-fluoro-1-(R)-cyclopropyl]-1,4-dihydro-8-methyl-4-oxoquinoline-3-carboxylic acid.

2. The compound as claimed in Claim 1 represented by formula (Ia), its salts and hydrates thereof:

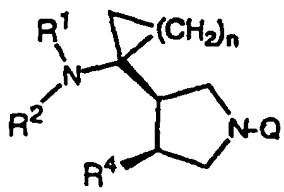

wherein $R^1$, $R^2$, $R^4$, n and Q are each as defined in Claim 1.

3. The compound as claimed in Claim 1 or 2, wherein Q in the formula (I) has a structure represented by the following formulae, its salts and hydrates thereof:

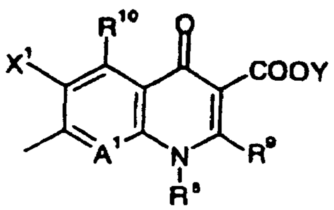

or

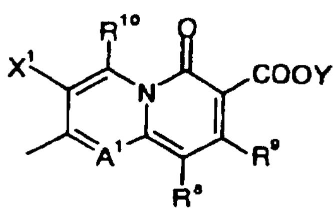

wherein $R^8$, $R^9$, $R^{10}$, $A^1$, $X^1$ and Y are each as defined in Claim 1.

4. The compound as claimed in Claim 1 or 2, wherein Q in the formula (I) has a structure represented by the following formula, its salts and hydrates thereof:

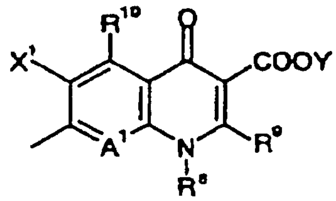

wherein $R^8$, $R^9$, $R^{10}$, $A^1$, $X^1$ and Y are each as defined in Claim 1.

**5.** The compound as claimed in Claim 1, 2, 3 or 4, wherein Q in the formula (I) is:

a 6-carboxy-9-fluoro-2,3-dihydro-3-(S)-methyl-7-oxo-7H-pyrido[1,2,3-de][1,4]-benzoxazin-10-yl group;
an 8-amino-6-carboxy-9-fluoro-2,3-dihydro-3-(S)-methyl-7-oxo-7H-pyrido[1,2,3-de][1,4]benzoxazin-10-yl group;
a 5-amino-3-carboxy-6-fluoro-1-[2-(S)-fluoro-1-(R)-cyclopropyl]-1,4-dihydro-8-methoxy-4-oxoquinolin-7-yl group;
a 5-amino-3-carboxy-6-fluoro-1-[2-(S)-fluoro-1-(R)-cyclopropyl}-1,4-dihydro-8-methyl-4-oxoquinolin-7-yl group;
or a 3-carboxy-6-fluoro-1-[2-(S)-fluoro-1-(R)-cyclopropyl]-1,4-dihydro-8-methoxy-4-oxoquinolin-7-yl group.

**6.** A compound represented by formula (I), its salts and hydrates thereof:

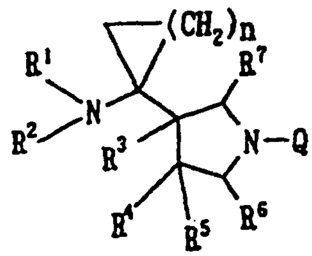

(I)

wherein

$R^1$ represents a hydrogen atom or an alkyl group having 1 to 6 carbon atoms;

$R^2$ represents a hydrogen atom or an alkyl group having 1 to 6 carbon atoms, provided that said alkyl group may have one or more substituents selected from the group consisting of a hydroxyl group, a halogen atom and an alkoxyl group having 1 to 6 carbon atoms;

$R^3$ and $R^5$, each represents a hydrogen atom;

$R^4$ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, provided that said alkyl group may have one or more substituents selected from the group consisting of a hydroxyl group, a halogen atom and an alkoxyl group having 1 to 6 carbon atoms and $R^4$ and the substituent on the pyrrolidine ring of the following formula:

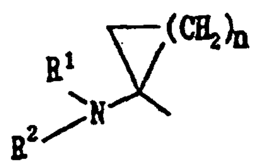

are located in the cis-configuration;

$R^6$ and $R^7$, each independently represents a hydrogen atom or an alkyl group having 1 to 6 carbon atoms;

n is an integer of from 1 to 3; and

Q represents a partial structure represented by the following formula:

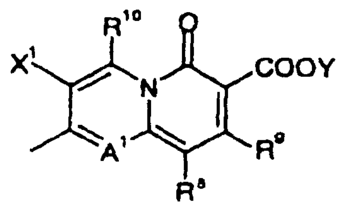

wherein $R^8$ represents an alkyl group having 1 to 6 carbon atoms, an alkenyl group having 2 to 6 carbon atoms, a halogenoalkyl group having 1 to 6 carbon atoms, a substituted or unsubstituted cyclic alkyl group having 3 to 6 carbon atoms, a substituted or unsubstituted aryl group, a substituted or unsubstituted heteroaryl group, an alkoxyl group having 1 to 6 carbon atoms or an alkylamino group having 1 to 6 carbon atoms;

$R^9$ represents a hydrogen atom or an alkylthio group having 1 to 6 carbon atoms;

$R^9$ and $R^8$ may form together with a part of the mother nucleus a cyclic structure optionally containing a sulfur atom as a constituting atom thereof and optionally having an alkyl group having 1 to 6 carbon atoms as a substituent;

$R^{10}$ represents a hydrogen atom, an amino group, a hydroxyl group, a thiol group, a halogenomethyl group , an alkyl group having 1 to 6 carbon atoms, an alkenyl group having 2 to 6 carbon atoms, an alkynyl group having 2 to 6 carbon atoms or an alkoxyl group having 1 to 6 carbon atoms, provided that said amino group may have one or more substituents selected from the group consisting of a formyl group, an alkyl group having 1 to 6 carbon atoms and an acyl group having 2 to 5 carbon atoms;

$X^1$ represents a halogen atom or a hydrogen atom;

$A^1$ represents a nitrogen atom or a partial structure represented by the following formula (II):

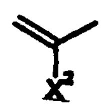

(II)

wherein $X^2$ represents a hydrogen atom, an amino group, a halogen atom, a cyano group, a halogenomethyl group, a halogenomethoxyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms, provided that said amino group may have one or more substituents selected from the group consisting of a formyl group, an alkyl group having 1 to 6 carbon atoms and an acyl group having 2 to 5 carbon atoms, and $X^2$ and $R^8$ may form together with a part of the mother nucleus a cyclic structure optionally containing an oxygen atom, a nitrogen atom or a sulfur atom as a constituting atom thereof and optionally having an alkyl group having 1 to 6 carbon atoms as a substituent;

Y represents a hydrogen atom, a phenyl group, an acetoxymethyl group, a pivaloyloxymethyl group, an ethoxycarbonyl group, a choline group, a dimethylaminoethyl group, a 5-indanyl group, a phthalidinyl group, a 5-alkyl-2-oxo-1,3-dioxol-4-ylmethyl group, a 3-acetoxy-2-oxobutyl group, an alkyl group having 1 to 6 carbon atoms, an alkoxylmethyl group having 2 to 7 carbon atoms or a phenylalkyl group composed of an alkyl group having 1 to 6 carbon atoms and a phenyl group.

**7.** A compound represented by formula (I), its salts and hydrates thereof:

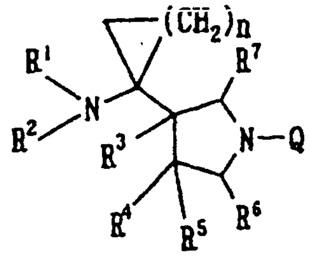

(I)

wherein

$R^1$ represents a hydrogen atom or an alkyl group having 1 to 6 carbon atoms;

$R^2$ represents a hydrogen atom or an alkyl group having 1 to 6 carbon atoms, provided that said alkyl group may have one or more substituents selected from the group consisting of a hydroxyl groups a halogen atom and an alkoxyl group having 1 to 6 carbon atoms;

$R^3$ and $R^5$, each represents a hydrogen atom;

$R^4$ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, provided that said alkyl group may have one or more substituents selected from the group consisting of a hydroxyl group, a halogen atom and an alkoxyl group having 1 to 6 carbon atoms and $R^4$ and the substituent on the pyrrolidine ring of the following formula:

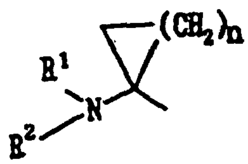

are located in the cis-configuration;

$R^6$ and $R^7$, each independently represents a hydrogen atom or an alkyl group having 1 to 6 carbon atoms;

n is an integer of from 1 to 3; and

Q represents a partial structure represented by the following formula:

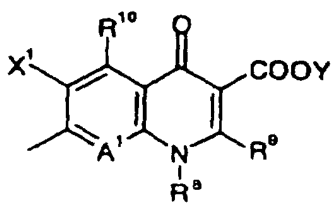

wherein $R^8$ represents an alkyl group having 1 to 6 carbon atoms, an alkenyl group having 2 to 6 carbon atoms, a halogenoalkyl group having 1 to 6 carbon atoms, a substituted or unsubstituted cyclic alkyl group having 3 to 6 carbon atoms, a substituted or unsubstituted aryl group, a substituted or unsubstituted heteroaryl group, an alkoxyl group having 1 to 6 carbon atoms or an alkylamino group having 1 to 6 carbon atoms;

$R^9$ represents a hydrogen atom or an alkylthio group having 1 to 6 carbon atoms;

$R^9$ and $R^8$ may form together with a part of the mother nucleus a cyclic structure optionally containing a sulfur atom as a constituting atom thereof and optionally having an alkyl group having 1 to 6 carbon atoms as a substituent;

$R^{10}$ represents an amino group having one or more substituents selected from the group consisting of a formyl group, an alkyl group having 1 to 6 carbon atoms and an acyl group having 2 to 5 carbon atoms, a hydroxyl group, a thiol group, a halogenomethyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms;

$X^1$ represents a halogen atom or a hydrogen atom;

$A^1$ represents a nitrogen atom or a partial structure represented by the following formula (II):

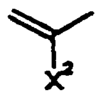

(II)

wherein $X^2$ represents a hydrogen atom, an amino group, a halogen atom, a cyano group, a halogenomethyl group, a halogenomethoxyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms, provided that said amino group may have one or more substituents selected from the group consisting or a formyl group, an alkyl group having 1 to 6 carbon atoms and an acyl group having 2 to 5 carbon atoms;

Y represents a hydrogen atom, a phenyl group, an acetoxymethyl group, a pivaloyloxymethyl group, an ethoxycarbonyl group, a choline group, a dimethylaminoethyl group, a 5-indanyl group, a phthalidinyl group, a 5-alkyl-2-oxo-1,3-dioxol-4-ylmethyl group, a 3-acetoxy-2-oxobutyl group, an alkyl group having 1 to 6 carbon atoms, an alkoxylmethyl group having 2 to 7 carbon atoms or a phenylalkyl group composed of an alkyl group having 1 to 6 carbon atoms and a phenyl group.

**8.** A compound represented by formula (I), its salts and hydrates thereof:

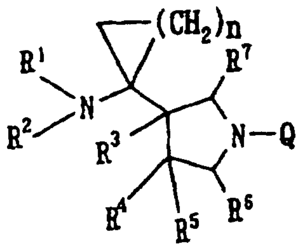

(I)

wherein

$R^1$ represents a hydrogen atom or an alkyl group having 1 to 6 carbon atoms;

$R^2$ represents a hydrogen atom or an alkyl group having 1 to 6 carbon atoms, provided that said alkyl group may have one or more substituents selected from the group consisting of a hydroxyl group, a halogen atom and an alkoxyl group having 1 to 6 carbon atoms;

$R^3$ and $R^5$, each represents a hydrogen atom;

$R^4$ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, provided that said alkyl group may have one or more substituents selected from the group consisting of a hydroxyl group, a halogen atom and an alkoxyl group having 1 to 6 carbon atoms or an alkylthio group having 1 to 6 carbon atoms, provided that said alkyl group may have one or more substituents selected from the group consisting of a hydroxyl group, a halogen atom and an alkoxyl group having 1 to 6 carbon atoms and $R^4$ and the substituent on the pyrrolidine ring of the following formula:

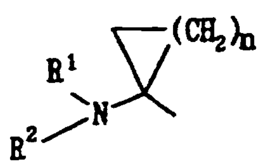

are located in the cis-configuration;

$R^6$ and $R^7$, each independently represents a hydrogen atom or an alkyl group having 1 to 6 carbon atoms;

n is an integer of from 1 to 3; and

Q represents a partial structure represented by the following formula:

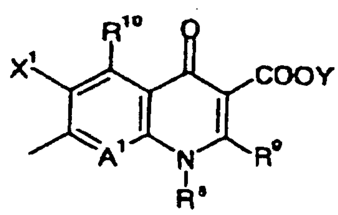

wherein $R^8$ represents an alkyl group having 1 to 6 carbon atoms, an alkenyl group having 2 to 6 carbon atoms, a halogenoalkyl group having 1 to 6 carbon atoms, a substituted or unsubstituted cyclic alkyl group having 3 to 6 carbon atoms, a substituted or unsubstituted aryl group, a substituted or unsubstituted heteroaryl group, an alkoxyl group having 1 to 6 carbon atoms or an alkylamino group having 1 to 6 carbon atoms;

$R^9$ represents a hydrogen atom or an alkylthio group having 1 to 6 carbon atoms;

$R^9$ and $R^8$ may form together with a part of the mother nucleus a cyclic structure optionally containing a sulfur atom as a constituting atom thereof and optionally having an alkyl group having 1 to 6 carbon atoms as a substituent;

$R^{10}$ represents a hydrogen atom, an amino group, a hydroxyl group, a thiol group, a halogenomethyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms, provided that said amino group may have one or more substituents selected from the group consisting of a formyl group, an alkyl group having 1 to 6 carbon atoms and an acyl group having 2 to 5 carbon atoms;

$X^1$ represents a halogen atom or a hydrogen atom;

$A^1$ represents a nitrogen atom or a partial structure represented by the following formula (II):

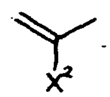

(II)

wherein $X^2$ represents a hydrogen atom, an amino group, a halogen atom, a cyano group, a halogenomethyl group, a halogenomethoxyl group, an alkenyl group having 2 to 6 carbon atoms or an alkynyl group having 2 to 6 carbon atoms, provided that said amino group may have one or more substituents selected from the group consisting or a formyl group, an alkyl group having 1 to 6 carbon atoms and an acyl group having 2 to 5 carbon atoms; and

Y represents a hydrogen atom, a phenyl group, an acetoxymethyl group, a pivaloyloxymethyl group, an ethoxycarbonyl group, a choline group, a dimethylaminoethyl group, a 5-indanyl group, a phthalidinyl group, a 5-alkyl-2-oxo-1,3-dioxol-4-ylmethyl group, a 3-acetoxy-2-oxobutyl group, an alkyl group having 1 to 6 carbon atoms, an alkoxylmethyl group having 2 to 7 carbon atoms or a phenylalkyl group composed of an alkyl group having 1 to 6 carbon atoms and a phenyl group.

**9.** A compound represented by formula (I), its salts and hydrates thereof:

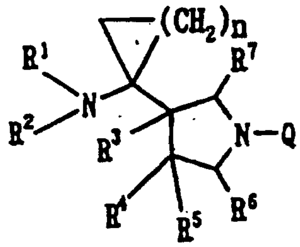

(I)

wherein

$R^1$ represents a hydrogen atom or an alkyl group having 1 to 6 carbon atoms;

$R^2$ represents a hydrogen atom or an alkyl group having 1 to 6 carbon atoms, provided that said alkyl group may have one or more substituents selected from the group consisting of a hydroxyl group, a halogen atom and an alkoxyl group having 1 to 6 carbon atoms;

$R^3$ and $R^5$, each represents a hydrogen atom;

$R^4$ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, provided that said alkyl group may have one or more substituents selected from the group consisting of a hydroxyl group, a halogen atom and an alkoxyl group having 1 to 6 carbon atoms and $R^4$ and the substituent on the pyrrolidine ring of the following formula:

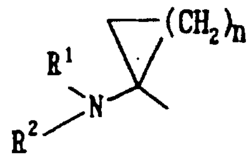

are located in the cis-configuration;

$R^6$ and $R^7$, each independently represents a hydrogen atom or an alkyl group having 1 to 6 carbon atoms;

n is an integer of from 1 to 3; and

Q represents a partial structure represented by the following formula:

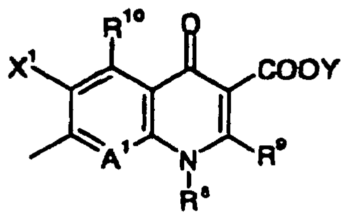

wherein $R^9$ represents a hydrogen atom or an alkylthio group having 1 to 6 carbon atoms;

$R^{10}$ represents an amino group, a hydroxyl group, a thiol group, a halogenomethyl group, an alkyl group having 1 to 6 carbon atoms, an alkenyl group having 2 to 6 carbon atoms, an alkynyl group having 2 to 6 carbon atoms or an alkoxyl group having 1 to 6 carbon atoms, provided that said amino group may have one or more substituents selected from the group consisting of a formyl group, an alkyl group having 1 to 6 carbon atoms and an acyl group having 2 to 5 carbon atoms;

$X^1$ represents a halogen atom or a hydrogen atom;

$A^1$ represents a partial structure represented by the following formula (II):

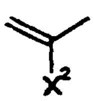

(II)

wherein $X^2$ and $R^8$ form together with a part of the mother nucleus a cyclic structure optionally containing an oxygen atom, a nitrogen atom or a sulfur atom as a constituting atom thereof and optionally having an alkyl group having 1 to 6 carbon atoms as a substituent; and

Y represents a hydrogen atom, a phenyl group, an acetoxymethyl group, a pivaloyloxymethyl group, an ethoxycarbonyl group, a choline group, a dimethylaminoethyl group, a 5-indanyl group, a phthalidinyl group, a 5-alkyl-2-oxo-1,3-dioxol-4-ylmethyl group, a 3-acetoxy-2-oxobutyl group, an alkyl group having 1 to 6 carbon atoms, an alkoxylmethyl group having 2 to 7 carbon atoms or a phenylalkyl group composed of an alkyl group having

1 to 6 carbon atoms and a phenyl group.

**10.** The compound as claimed in Claim 9, wherein Q in the formula (I) forms a 2,3-dihydro-7-oxo-7H-pyrido[1.2.3-de][1.4]benzoxacin-6-carboxylic acid structure.

**11.** The compound as claimed in Claim 10, wherein the structure has a 3-(S)-methyl substituent.

**12.** The compound as claimed in Claim 9, wherein Q in the formula (I) is an 8-amino-6-carboxy-9-fluoro-2,3-dihydro-3-(S)-methyl-7-oxo-7H-pyrido[1.2.3-de][1.4]benzoxacin-10-yl group.

**13.** The compound as claimed in any one of Claims 1 to 12, wherein the substituent $R^4$ is a halogen atom, in particular a fluorine atom.

**14.** The compound as claimed in any one of Claims 1 to 13, wherein n is 1 or 2.

**15.** The compound as claimed in any one of Claims 1 to 13, wherein the substituent $R^4$ is a fluorine atom and n is 1 or 2.

**16.** The compound as claimed in any one of Claims 1 to 8, wherein the substituent $R^8$ is:

a halogenocyclopropyl group;
a 1,2-cis-2-halogenocyclopropyl group;
a stereochemically pure one;
a (1R,2S)-2-halogenocyclopropyl group;
or a (1R,2S)-2-fluorocyclopropyl group.

**17.** The compound as claimed in any one of claims 1 to 16, wherein the substituent $X^1$ is a halogen atom, in particular a fluorine atom.

**18.** The compound as claimed in any one of claims 1 to 16, wherein the substituents $X^1$ and $X^2$ are each a halogen atom.

**19.** The compound as claimed in any one of claims 1 to 18, wherein $R^{10}$ is an alkyl group having 1 to 6 carbon atoms.

**20.** The compound as claimed in any one of claims 1 to 19, which is a stereochemically pure one, its salts and hydrates thereof.

**21.** 8-Amino-10-[4-(R)-(1-aminocyclopropyl)-3-(S)-fluoro-1-pyrrolidinyl]-9-fluoro-2,3-dihydro-3-(S)-methyl-7-oxo-7H-pyrido[ 1,2,3-de][1,4]benzoxazine-6-carboxylic acid, its salts and hydrates thereof.

**22.** A drug containing as the active ingredient at least one compound as claimed in any one of Claims 1 to 21, hydrates thereof, salts of said compound or hydrates of said salts.

**23.** An antimicrobial agent containing as the active ingredient at least one compound as claimed in any one of Claims 1 to 21, hydrates thereof, salts of said compound or hydrates of said salts.

**24.** The use of a compound as claimed in any one of claims 1 to 21 in the manufacture of a drug for treating infectious diseases.

**25.** A compound represented by the following formula (VI), its salts and hydrates thereof:

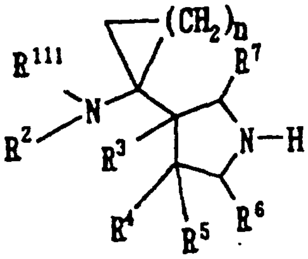

(VI)

wherein $R^{111}$ represents a hydrogen atom, an alkyl group having 1 to 6 carbon atoms or a protective group of the amino group;

$R^2$ represents a hydrogen atom or an alkyl group having 1 to 6 carbon atoms, provided that said alkyl group may have one or more substituents selected from the group consisting of a hydroxyl group, a halogen atom and an alkoxyl group having 1 to 6 carbon atoms;

$R^3$ and $R^5$, each represents a hydrogen atom;

$R^4$ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, provided that said alkyl group may have one or more substituents selected from the group consisting of a hydroxyl group, a halogen atom and an alkoxyl group having 1 to 6 carbon atoms and $R^4$ and the substituent on the pyrrolidine ring of the following formula:

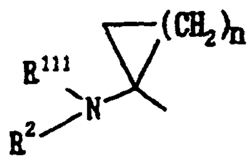

are located in the cis-configuration;

$R^6$ and $R^7$, each independently represents a hydrogen atom or an alkyl group having 1 to 6 carbon atoms;

n is an integer of from 1 to 3;

provided that the following compounds and addition salts thereof are excluded:

cis-4-(1-aminocyclopropyl)-3-(hydroxymethylpyrrolidine);
4-(R)-(1-aminocyclopropyl)-3-(S)-fluoropyrrolidine;
cis-4-(1-aminocyclobutyl)-3-fluoropyrrolidine;

in which compounds the amino group may be protected.

**26.** The compound as claimed in Claim 25, wherein the substituent $R^4$ is a halogen atom, in particular a fluorine atom.

**27.** A compound represented by the following formula (VI)

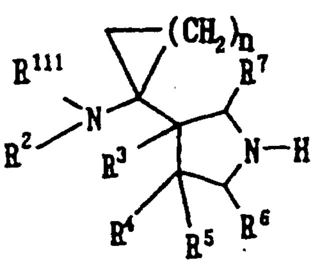

(VI)

wherein $R^{111}$ represents a hydrogen atom, an alkyl group having 1 to 6 carbon atoms or a protective group of the

amino group;

$R^2$ represents a hydrogen atom or an alkyl group having 1 to 6 carbon atoms, provided that said alkyl group may have one or more substituents selected from the group consisting of a hydroxyl group, a halogen atom and an alkoxyl group having 1 to 6 carbon atoms;

$R^3$ and $R^5$, each represents a hydrogen atom;

$R^4$ represents a hydroxyl group, a chlorine atom, a carbamoyl group, an alkyl group having 1 to 6 carbon atoms, an alkoxyl group having 1 to 6 carbon atoms; 2-hydroxyethyl, 3-hydroxypropyl or an alkylthio group having 1 to 6 carbon atoms, provided that said alkyl group may have one or more substituents selected from the group consisting of a halogen atom and an alkoxyl group having 1 to 6 carbon atoms and $R^4$ and the substituent on the pyrrolidine ring of the following formula:

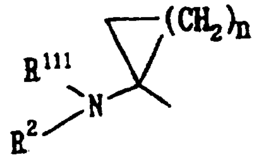

are located in the cis-configuration;

$R^6$ and $R^7$, each independently represents a hydrogen atom or an alkyl group having 1 to 6 carbon atoms; n is an integer of from 1 to 3.

**28.** A compound represented by the following formula (VII):

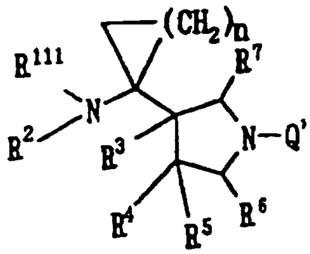

(VII)

wherein $R^{111}$ represents a hydrogen atom, an alkyl group having 1 to 6 carbon atoms or a protective group of the amino group;

$R^2$ represents a hydrogen atom or an alkyl group having 1 to 6 carbon atoms, provided that said alkyl group may have one or more substituents selected from the group consisting of a hydroxyl group, a halogen atom and an alkoxyl group having 1 to 6 carbon atoms;

$R^3$ and $R^5$, each represents a hydrogen atom;

$R^4$ represents a hydroxyl group, a halogen atom, a carbamoyl group, an alkyl group having 1 to 6 carbon atoms, an alkoxyl group having 1 to 6 carbon atoms or an alkylthio group having 1 to 6 carbon atoms, provided that said alkyl group may have one or more substituents selected from the group consisting of a hydroxyl group, a halogen atom and an alkoxyl group having 1 to 6 carbon atoms; and $R^4$ and the substituent on the pyrrolidine ring of the following formula:

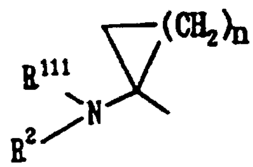

are located in the cis-configuration;

$R^6$ and $R^7$, each independently represents a hydrogen atom or an alkyl group having 1 to 6 carbon atoms;
n is an integer of from 1 to 3; and
Q' represents a protective group for the amino group selected from the group consisting of optionally substituted alkoxylcarbonyl groups, optionally substituted aralkyloxycarbonyl groups, optionally substituted acyl groups, optionally substituted alkyl groups, optionally substituted aralkyl groups and substituted silyl groups,
provided that the following compounds are excluded:

1-benzyloxycarbonyl-4-(R)-(1-tert-butoxycarbonylaminocyclopropyl)-3-(S)-fluoropyrrolidine;
cis-1-[1-(S)-phenylethyl)-4-(1-tert-butoxycarbonylaminocyclobutyl)-3-fluoropyrrolidine.

**29.** A compound represented by the following formula (VII):

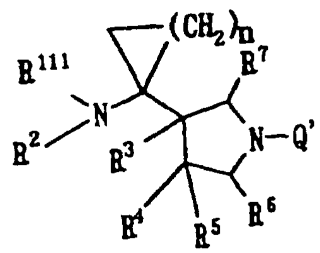

(VII)

wherein $R^{111}$ represents a hydrogen atom, an alkyl group having 1 to 6 carbon atoms or a protective group of the amino group;
$R^2$ represents a hydrogen atom or an alkyl group having 1 to 6 carbon atoms, provided that the alkyl group may have one or more substituents selected from the group consisting of a hydroxyl group, a halogen atom and an alkoxyl group having 1 to 6 carbon atoms;
$R^3$ and $R^5$, each represents a hydrogen atom;
$R^4$ represents a hydroxyl group, a chlorine atom, a carbamoyl group, an alkyl group having 1 to 6 carbon atoms, an alkoxyl group having 1 to 6 carbon atoms or an alkylthio group having 1 to 6 carbon atoms, provided that the alkyl group may have one or more substituents selected from the group consisting of a hydroxyl group, a halogen atom and an alkoxyl group having 1 to 6 carbon atoms; and
$R^4$ and the substituent on the pyrrolidine ring of the following formula:

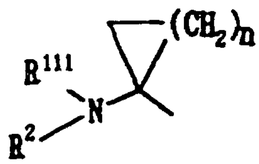

are located in the cis-configuration;
$R^6$ and $R^7$, each independently represents a hydrogen atom or an alkyl group having 1 to 6 carbon atoms;
n is an integer of from 1 to 3;
Q' represents a protective group for the amino group selected from the group consisting of optionally substituted alkoxylcarbonyl groups, optionally substituted aralkyloxycarbonyl groups, optionally substituted acyl groups, optionally substituted alkyl groups, optionally substituted aralkyl groups and substituted silyl groups.

**30.** The compound as claimed in any one of Claims 25 to 29, wherein n is 1 or 2.

**31.** The compound as claimed in Claim 25 or 28, wherein the substituent $R^4$ is a fluorine atom and n is 1 or 2.

**32.** The compound as claimed in any one of Claims 25 to 31, wherein the protective group of the amino group is selected from the group consisting of optionally substituted alkoxylcarbonyl groups, optionally substituted aralkyloxycarbonyl groups, optionally substituted acyl groups, optionally substituted alkyl groups, optionally substituted aralkyl groups and substituted silyl groups.

**Patentansprüche**

**1.** Verbindung der Formel (I), deren Salze und Hydrate davon:

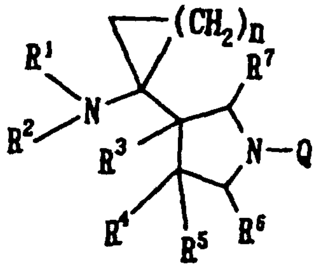

(I)

worin

$R^1$ für ein Wasserstoffatom oder eine Alkylgruppe mit 1 bis 6 Kohlenstoffatomen steht;

$R^2$ für ein Wasserstoffatom oder eine Alkylgruppe mit 1 bis 6 Kohlenstoffatomen steht, mit der Maßgabe, dass die Alkylgruppe einen oder mehrere, unter einer Hydroxylgruppe, einem Halogenatom und einer Alkoxylgruppe mit 1 bis 6 Kohlenstoffatomen ausgewählte Substituenten aufweisen kann;

$R^3$ und $R^5$ jeweils für ein Wasserstoffatom stehen;

$R^4$ für eine Hydroxylgruppe, ein Halogenatom, eine Carbamoylgruppe, eine Alkylgruppe mit 1 bis 6 Kohlenstoffatomen, eine Alkoxylgruppe mit 1 bis 6 Kohlenstoffatomen oder eine Alkylthiogruppe mit 1 bis 6 Kohlenstoffatomen steht, mit der Maßgabe, dass die Alkylgruppe einen oder mehrere, unter einer Hydroxylgruppe, einem Halogenatom und einer Alkoxylgruppe mit 1 bis 6 Kohlenstoffatomen ausgewählte Substituenten aufweisen kann und $R^4$ und der Substituent am Pyrrolidinring der folgenden Formel:

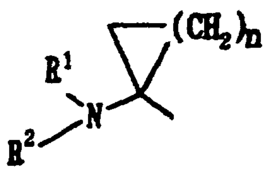

in cis-Konfiguration stehen;

$R^6$ und $R^7$ jeweils unabhängig voneinander für ein Wasserstoffatom oder eine Alkylgruppe mit 1 bis 6 Kohlenstoffatomen stehen;

n eine ganze Zahl mit einem Wert von 1 bis 3 ist; und

Q für eine Partialstruktur der folgenden Formel:

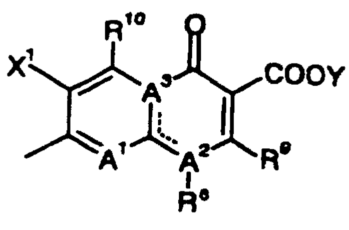

steht,

worin $R^8$ für eine Alkylgruppe mit 1 bis 6 Kohlenstoffatomen, eine Alkenylgruppe mit 2 bis 6 Kohlenstoffatomen, eine Halogenalkylgruppe mit 1 bis 6 Kohlenstoffatomen, eine substituierte oder unsubstituierte Cycloalkylgruppe mit 3 bis 6 Kohlenstoffatomen, eine substituierte oder unsubstituierte Arylgruppe, eine substituierte oder unsubstituierte Heteroarylgruppe, eine Alkoxylgruppe mit 1 bis 6 Kohlenstoffatomen oder eine Alkylaminogruppe mit 1 bis 6 Kohlenstoffatomen steht;

$R^9$ für ein Wasserstoffatom oder eine Alkylthiogruppe mit 1 bis 6 Kohlenstoffatomen steht;

$R^9$ und $R^8$ zusammen mit einem Teil des Grundgerüsts eine cyklische Struktur bilden, die gegebenenfalls ein Schwefelatom als ein die Struktur bildendes Atom enthält und gegebenenfalls eine Alkylgruppe mit 1 bis 6 Kohlenstoffatomen als Substituent aufweist;

$R^{10}$ für ein Wasserstoffatom, eine Aminogruppe, eine Hydroxylgruppe, eine Thiolgruppe, eine Halogenmethylgruppe, eine Alkylgruppe mit 1 bis 6 Kohlenstoffatomen, eine Alkenylgruppe mit 2 bis 6 Kohlenstoffatomen, eine Alkinylgruppe mit 2 bis 6 Kohlenstoffatomen oder eine Alkoxylgruppe mit 1 bis 6 Kohlenstoffatomen steht, mit der Maßgabe, dass die Aminogruppe einen oder mehrere unter einer Formylgruppe, einer Alkylgruppe mit 1 bis 6 Kohlenstoffatomen und einer Acylgruppe mit 2 bis 5 Kohlenstoffatomen ausgewählte Substituenten aufweisen kann;

$X^1$ für ein Halogenatom oder ein Wasserstoffatom steht;

$A^1$ für ein Stickstoffatom oder eine Partialstruktur der folgenden Formel (II):

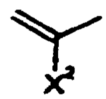

(II)

steht,

worin $X^2$ für ein Wasserstoffatom, eine Aminogruppe, ein Halogenatom, eine Cyanogruppe, eine Halogenmethylgruppe, eine Halogenmethoxylgruppe, eine Alkylgruppe mit 1 bis 6 Kohlenstoffatomen, eine Alkenylgruppe mit 2 bis 6 Kohlenstoffatomen, eine Alkinylgruppe mit 2 bis 6 Kohlenstoffatomen oder eine Alkoxylgruppe mit 1 bis 6 Kohlenstoffatomen steht, mit der Maßgabe, dass die Aminogruppe einen oder mehrere, unter einer Formylgruppe, einer Alkylgruppe mit 1 bis 6 Kohlenstoffatomen und einer Acylgruppe mit 2 bis 5 Kohlenstoffatomen ausgewählte Substituenten aufweisen kann, und $X^2$ und $R^8$ zusammen mit einem Teil des Grundgerüsts eine cyklische Struktur bilden können, die gegebenenfalls ein Sauerstoffatom, ein Stickstoffatom oder ein Schwefelatom als ein die Struktur bildendes Atom enthält und gegebenenfalls eine Alkylgruppe mit 1 bis 6 Kohlenstoffatomen als Substituent aufweist;

$A^2$ und $A^3$ jeweils für ein Stickstoffatom oder ein Kohlenstoffatom stehen, mit der Maßgabe, dass $A^2$ und $A^3$ zusammen mit dem Kohlenstoffatom, an das sie gebunden sind, eine Partialstruktur der folgenden Formel:

$$>C=C(A^1=)-N(R^8)-$$

oder eine Partialstruktur der folgenden Formel:

$$>N-C(A^1=)=C(R^8)-$$

bilden können; und

Y für ein Wasserstoffatom, eine Phenylgruppe, eine Acetoxymethylgruppe, eine Pivaloyloxymethylgruppe, eine Ethoxycarbonylgruppe, eine Cholingruppe, eine Dimethylaminomethylgruppe, eine 5-Indanylgruppe, eine Phthalidinylgruppe, eine 5-Alky-2-oxo-1,3-dioxol-4-ylmethylgruppe, eine 3-Acetoxy-2-oxobutylgruppe, eine Alkylgruppe mit 1 bis 6 Kohlenstoffatomen, eine Alkoxylmethylgruppe mit 2 bis 7 Kohlenstoffatomen oder eine aus einer Alkylgruppe mit 1 bis 6 Kohlenstoffatomen und einer Phenylgruppe zusammengesetzte Phenylalkylgruppe steht, mit der Maßgabe, dass die folgenden Verbindungen ausgeschlossen sind:

5-Amino-7-[cis-4-(1-aminocyclopropyl)-3-hydroxymethyl-1-pyrrolidinyl]-1-cyclopropyl-6-fluor-1,4-dihydro-8-methyl-4-oxochinolin-3-carbonsäure;

5-Amino-7-[4-(R)-(1-aminocyclopropyl)-3-(S)-fluor-1-pyrrolidinyl]-6-fluor-1-[2-(S)-fluor-1-(R)-cyclopropyl]-1,4-dihydro-8-methyl-4-oxochinolin-3-carbonsäure-Hydrochlorid;

5-Amino-7-[4-(R)-(1 -aminocyclopropyl)-3-(S)-fluor-1-pyrrolidinyl]-6-fluor-1-[2-(S)-fluor-1-(R)-cyclopropyl]-1,4-dihydro-8-methoxy-4-oxochinolin-3-carbonsäure;

10-[4-(R)-(1-Aminocyclopropyl)-3-(S)-fluor-1-pyrrolidinyl]-9-fluor-2,3-dihydro-3-(S)-methyl-7-oxo-7H-pyrido [1.2.3-de][1.4]benzoxazin-6-carbonsäure;

7-[4-(R)-(1-Aminocyclopropyl)-3-(S)-fluor-1-pyrrolidinyl]-6-fluor-1-[2-(S)-fluor-1-(R)-cyclopropyl]-1,4-dihydro-8-methoxy-4-oxochinolin-3-carbonsäure;
5-Amino-7-[cis-4-(1-aminocyclobutyl)-3-fluor-1-pyrrolidinyl]-6-fluor-1-[2-(S)-fluor-1-(R)-cyclopropyl]-1,4-dihydro-8-methyl-4-oxochinolin-3-carbonsäure.

**2.** Verbindung nach Anspruch 1 der Formel (Ia), deren Salze und Hydrate davon:

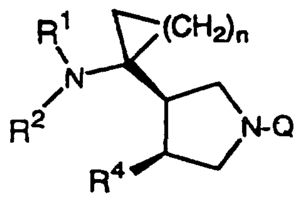

worin $R^1$, $R^2$, $R^4$, n und Q jeweils wie in Anspruch 1 definiert sind.

**3.** Verbindung nach Anspruch 1 oder 2, worin Q in der Formel (I) eine Struktur der folgenden Formeln aufweist, deren Salze und Hydrate davon:

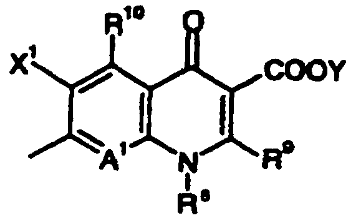

oder

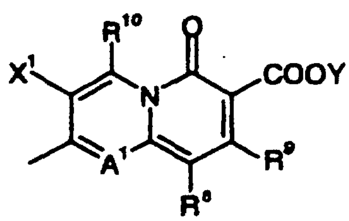

worin $R^8$, $R^9$, $R^{10}$, $A^1$, $X^1$ und Y jeweils wie in Anspruch 1 definiert sind.

**4.** Verbindung nach Anspruch 1 oder 2, worin Q in der Formel (I) eine Struktur der folgenden Formel aufweist, ihre Salze und Hydrate davon:

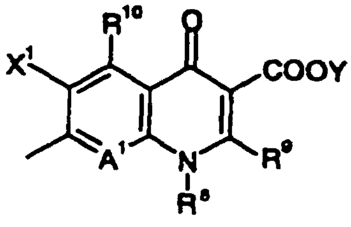

worin $R^8$, $R^9$, $R^{10}$. $A^1$, $X^1$ und Y jeweils wie in Anspruch 1 definiert sind.

**5.** Verbindung nach Anspruch 1, 2, 3 oder 4, worin Q in der Formel (I):

eine 6-Carboxy-9-fluor-2,3-dihydro-3-(S)-methyl-7-oxo-7H-pyrido[1,2,3-de][1,4]-benzoxazin-10- yl-Gruppe;
eine 8-Amino-6-carboxy-9-fluor-2,3-dihydro-3-(S)-methyl-7-oxo-7Hpyrido[1,2,3-de][1,4]benzoxazin-10-yl-Gruppe;
eine 5-Amino-3-carboxy-6-fluor-1-[2-(S)-fluor-1-(R)-cyclopropyl]-1,4-dihydro-8-methoxy-4-oxochinolin-7-yl-Gruppe;
eine 5-Amino-3-carboxy-6-fluor-1-[2-(S)-f)uor-1-(R)-cyclopropyl]-1,4-dihydro-8-methyl-4-oxochinolin-7-yl-Gruppe;
oder eine 3-Carboxy-6-fluor-1-[2-(S)-fluor-1-(R)-cyclopropyl]-1,4-dihydro-8-methoxy-4-oxochinolin-7-yl-Gruppe ist.

**6.** Verbindung der Formel (I), deren Salze und Hydrate davon:

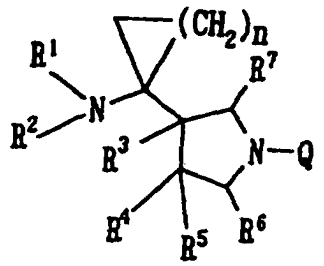

(I)

worin

$R^1$ für ein Wasserstoffatom oder eine Alkylgruppe mit 1 bis 6 Kohlenstoffatomen steht;

$R^2$ für ein Wasserstoffatom oder eine Alkylgruppe mit 1 bis 6 Kohlenstoffatomen steht, mit der Maßgabe, dass die Alkylgruppe einen oder mehrere, unter einer Hydroxylgruppe, einem Halogenatom und einer Alkoxylgruppe mit 1 bis 6 Kohlenstoffatomen ausgewählte Substituenten aufweisen kann;

$R^3$ und $R^5$ jeweils für ein Wasserstoffatom stehen;

$R^4$ für eine Hydroxylgruppe, ein Halogenatom, eine Carbamoylgruppe, eine Alkylgruppe mit 1 bis 6 Kohlenstoffatomen, eine Alkoxylgruppe mit 1 bis 6 Kohlenstoffatomen oder eine Alkylthiogruppe mit 1 bis 6 Kohlenstoffatomen steht, mit der Maßgabe, dass die Alkylgruppe einen oder mehrere, unter einer Hydroxylgruppe, einem Halogenatom und einer Alkoxylgruppe mit 1 bis 6 Kohlenstoffatomen ausgewählte Substituenten aufweisen kann und $R^4$ und der Substituent am Pyrrolidinring der folgenden Formel:

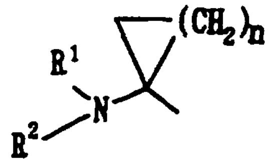

in cis-Konfiguration stehen;

$R^6$ und $R^7$ jeweils unabhängig voneinander für ein Wasserstoffatom oder eine Alkylgruppe mit 1 bis 6 Kohlenstoffatomen stehen;

n eine ganze Zahl mit einem Wert von 1 bis 3 ist; und

Q für eine Partialstruktur der folgenden Formel:

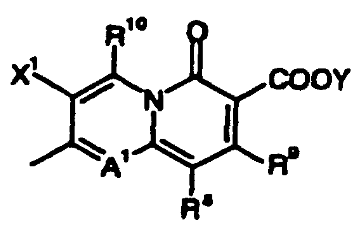

steht,
worin $R^8$ für eine Alkylgruppe mit 1 bis 6 Kohlenstoffatomen, eine Alkenylgruppe mit 2 bis 6 Kohlenstoffatomen, eine Halogenalkylgruppe mit 1 bis 6 Kohlenstoffatomen, eine substituierte oder unsubstituierte Cycloalkylgruppe mit 3 bis 6 Kohlenstoffatomen, eine substituierte oder unsubstituierte Arylgruppe, eine substituierte oder unsubstituierte Heteroarylgruppe, eine Alkoxylgruppe mit 1 bis 6 Kohlenstoffatomen oder eine Alkylaminogruppe mit 1 bis 6 Kohlenstoffatomen steht;

$R^9$ für ein Wasserstoffatom oder eine Alkylthiogruppe mit 1 bis 6 Kohlenstoffatomen steht;

$R^9$ und $R^8$ zusammen mit einem Teil des Grundgerüsts eine cyklische Struktur bilden, die gegebenenfalls ein Schwefelatom als ein die Struktur bildendes Atom enthält und gegebenenfalls eine Alkylgruppe mit 1 bis 6 Kohlenstoffatomen als Substituent aufweist;

$R^{10}$ für ein Wasserstoffatom, eine Aminogruppe, eine Hydroxylgruppe, eine Thiolgruppe, eine Halogenmethylgruppe, eine Alkylgruppe mit 1 bis 6 Kohlenstoffatomen, eine Alkenylgruppe mit 2 bis 6 Kohlenstoffatomen, eine Alkinylgruppe mit 2 bis 6 Kohlenstoffatomen oder eine Alkoxylgruppe mit 1 bis 6 Kohlenstoffatomen steht, mit der Maßgabe, dass die Aminogruppe einen oder mehrere unter einer Formylgruppe, einer Alkylgruppe mit 1 bis 6 Kohlenstoffatomen und einer Acylgruppe mit 2 bis 5 Kohlenstoffatomen ausgewählte Substituenten aufweisen kann;

$X^1$ für ein Halogenatom oder ein Wasserstoffatom steht;

$A^1$ für ein Stickstoffatom oder eine Partialstruktur der folgenden Formel (II):

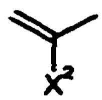

(II)

steht,

worin $X^2$ für ein Wasserstoffatom, eine Aminogruppe, ein Halogenatom, eine Cyanogruppe, eine Halogenmethylgruppe, eine Halogenmethoxylgruppe, eine Alkylgruppe mit 1 bis 6 Kohlenstoffatomen, eine Alkenylgruppe mit 2 bis 6 Kohlenstoffatomen, eine Alkinylgruppe mit 2 bis 6 Kohlenstoffatomen oder eine Alkoxylgruppe mit 1 bis 6 Kohlenstoffatomen steht, mit der Maßgabe, dass die Aminogruppe einen oder mehrere, unter einer Formylgruppe, einer Alkylgruppe mit 1 bis 6 Kohlenstoffatomen und einer Acylgruppe mit 2 bis 5 Kohlenstoffatomen ausgewählte Substituenten aufweisen kann, und $X^2$ und $R^8$ zusammen mit einem Teil des Grundgerüsts eine cyklische Struktur bilden können, die gegebenenfalls ein Sauerstoffatom, ein Stickstoffatom oder ein Schwefelatom als ein die Struktur bildendes Atom enthält und gegebenenfalls eine Alkylgruppe mit 1 bis 6 Kohlenstoffatomen als Substituent aufweist;

Y für ein Wasserstoffatom, eine Phenylgruppe, eine Acetoxymethylgruppe, eine Pivaloyloxymethylgruppe, eine Ethoxycarbonylgruppe, eine Cholingruppe, eine Dimethylaminomethylgruppe, eine 5-Indanylgruppe, eine Phthalidinylgruppe, eine 5-Alky-2-oxo-1,3-dioxol-4-ylmethylgruppe, eine 3-Acetoxy-2-oxobutylgruppe, eine Alkylgruppe mit 1 bis 6 Kohlenstoffatomen, eine Alkoxylmethylgruppe mit 2 bis 7 Kohlenstoffatomen oder eine aus einer Alkylgruppe mit 1 bis 6 Kohlenstoffatomen und einer Phenylgruppe zusammengesetzte Phenylalkylgruppe steht.

**7.** Verbindung der Formel (I), deren Salze und Hydrate davon:

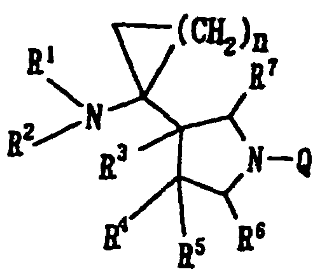

(I)

worin

$R^1$ für ein Wasserstoffatom oder eine Alkylgruppe mit 1 bis 6 Kohlenstoffatomen steht;

$R^2$ für ein Wasserstoffatom oder eine Alkylgruppe mit 1 bis 6 Kohlenstoffatomen steht, mit der Maßgabe, dass die Alkylgruppe einen oder mehrere, unter einer Hydroxylgruppe, einem Halogenatom und einer Alkoxylgruppe mit 1 bis 6 Kohlenstoffatomen ausgewählte Substituenten aufweisen kann;

$R^3$ und $R^5$ jeweils für ein Wasserstoffatom stehen;

$R^4$ für eine Hydroxylgruppe, ein Halogenatom, eine Carbamoylgruppe, eine Alkylgruppe mit 1 bis 6 Kohlenstoffatomen, eine Alkoxylgruppe mit 1 bis 6 Kohlenstoffatomen oder eine Alkylthiogruppe mit 1 bis 6 Kohlenstoffatomen steht, mit der Maßgabe, dass die Alkylgruppe einen oder mehrere, unter einer Hydroxylgruppe, einem Halogenatom und einer Alkoxylgruppe mit 1 bis 6 Kohlenstoffatomen ausgewählte Substituenten aufweisen kann und $R^4$ und der Substituent am Pyrrolidinring der folgenden Formel:

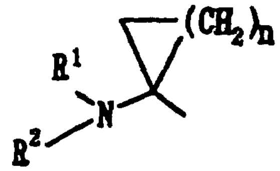

in cis-Konfiguration stehen;

$R^6$ und $R^7$ jeweils unabhängig voneinander für ein Wasserstoffatom oder eine Alkylgruppe mit 1 bis 6 Kohlenstoffatomen stehen;

n eine ganze Zahl mit einem Wert von 1 bis 3 ist; und

Q für eine Partialstruktur der folgenden Formel:

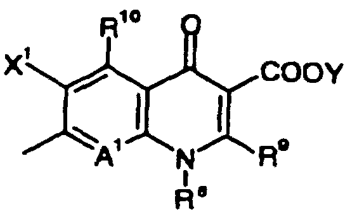

steht,

worin $R^8$ für eine Alkylgruppe mit 1 bis 6 Kohlenstoffatomen, eine Alkenylgruppe mit 2 bis 6 Kohlenstoffatomen, eine Halogenalkylgruppe mit 1 bis 6 Kohlenstoffatomen, eine substituierte oder unsubstituierte Cycloalkylgruppe mit 3 bis 6 Kohlenstoffatomen, eine substituierte oder unsubstituierte Arylgruppe, eine substituierte oder unsubstituierte Heteroarylgruppe, eine Alkoxylgruppe mit 1 bis 6 Kohlenstoffatomen oder eine Alkylaminogruppe mit 1 bis 6 Kohlenstoffatomen steht;

$R^9$ für ein Wasserstoffatom oder eine Alkylthiogruppe mit 1 bis 6 Kohlenstoffatomen steht;

$R^9$ und $R^8$ zusammen mit einem Teil des Grundgerüsts eine cyklische Struktur bilden, die gegebenenfalls ein Schwefelatom als ein die Struktur bildendes Atom enthält und gegebenenfalls eine Alkylgruppe mit 1 bis 6 Kohlenstoffatomen als Substituent aufweist;

$R^{10}$ für eine Aminogruppe, die einen oder mehrere unter einer Formylgruppe, einer Alkylgruppe mit 1 bis 6 Kohlenstoffatomen und einer Acylgruppe mit 2 bis 5 Kohlenstoffatomen ausgewählte Substituenten aufweisen kann, eine Hydroxylgruppe, eine Thiolgruppe, eine Halogenmethylgruppe, eine Alkylgruppe mit 1 bis 6 Kohlenstoffatomen, eine Alkenylgruppe mit 2 bis 6 Kohlenstoffatomen, eine Alkinylgruppe mit 2 bis 6 Kohlenstoffatomen oder eine Alkoxylgruppe mit 1 bis 6 Kohlenstoffatomen steht;

$X^1$ für ein Halogenatom oder ein Wasserstoffatom steht;

$A^1$ für ein Stickstoffatom oder eine Partialstruktur der folgenden Formel (II):

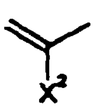

(II)

steht,

worin $X^2$ für ein Wasserstoffatom, eine Aminogruppe, ein Halogenatom, eine Cyanogruppe, eine Halogenmethylgruppe, eine Halogenmethoxylgruppe, eine Alkylgruppe mit 1 bis 6 Kohlenstoffatomen, eine Alkenylgruppe mit 2 bis 6 Kohlenstoffatomen, eine Alkinylgruppe mit 2 bis 6 Kohlenstoffatomen oder eine Alkoxylgruppe mit 1 bis 6 Kohlenstoffatomen steht, mit der Maßgabe, dass die Aminogruppe einen oder mehrere, unter einer Formylgruppe, einer Alkylgruppe mit 1 bis 6 Kohlenstoffatomen und einer Acylgruppe mit 2 bis 5 Kohlenstoffatomen ausgewählte Substituenten aufweisen kann;

Y für ein Wasserstoffatom, eine Phenylgruppe, eine Acetoxymethylgruppe, eine Pivaloyloxymethylgruppe, eine Ethoxycarbonylgruppe, eine Cholingruppe, eine Dimethylaminomethylgruppe, eine 5-Indanylgruppe, eine Phthalidinylgruppe, eine 5-Alky-2-oxo-1,3-dioxol-4-ylmethylgruppe, eine 3-Acetoxy-2-oxobutylgruppe, eine Alkylgruppe mit 1 bis 6 Kohlenstoffatomen, eine Alkoxylmethylgruppe mit 2 bis 7 Kohlenstoffatomen oder eine aus einer Alkylgruppe mit 1 bis 6 Kohlenstoffatomen und einer Phenylgruppe zusammengesetzte Phenylalkylgruppe steht.

**8.** Verbindung der Formel (I), deren Salze und Hydrate davon:

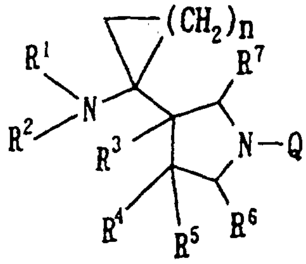

(I)

worin

$R^1$ für ein Wasserstoffatom oder eine Alkylgruppe mit 1 bis 6 Kohlenstoffatomen steht;

$R^2$ für ein Wasserstoffatom oder eine Alkylgruppe mit 1 bis 6 Kohlenstoffatomen steht, mit der Maßgabe, dass die Alkylgruppe einen oder mehrere, unter einer Hydroxylgruppe, einem Halogenatom und einer Alkoxylgruppe mit 1 bis 6 Kohlenstoffatomen ausgewählte Substituenten aufweisen kann;

$R^3$ und $R^5$ jeweils für ein Wasserstoffatom stehen;

$R^4$ für eine Hydroxylgruppe, ein Halogenatom, eine Carbamoylgruppe, eine Alkylgruppe mit 1 bis 6 Kohlenstoffatomen, eine Alkoxylgruppe mit 1 bis 6 Kohlenstoffatomen oder eine Alkylthiogruppe mit 1 bis 6 Kohlenstoffatomen steht, mit der Maßgabe, dass die Alkylgruppe einen oder mehrere, unter einer Hydroxylgruppe, einem Halogenatom und einer Alkoxylgruppe mit 1 bis 6 Kohlenstoffatomen ausgewählte Substituenten aufweisen kann und $R^4$ und der Substituent am Pyrrolidinring der folgenden Formel:

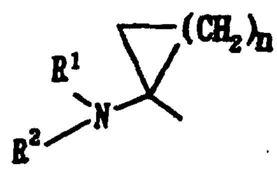

in cis-Konfiguration stehen;

$R^6$ und $R^7$ jeweils unabhängig voneinander für ein Wasserstoffatom oder eine Alkylgruppe mit 1 bis 6 Kohlenstoffatomen stehen;

n eine ganze Zahl mit einem Wert von 1 bis 3 ist; und

Q für eine Partialstruktur der folgenden Formel:

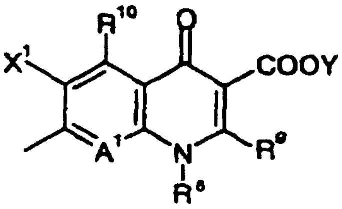

steht,

worin $R^8$ für eine Alkylgruppe mit 1 bis 6 Kohlenstoffatomen, eine Alkenylgruppe mit 2 bis 6 Kohlenstoffatomen, eine Halogenalkylgruppe mit 1 bis 6 Kohlenstoffatomen, eine substituierte oder unsubstituierte Cycloalkylgruppe mit 3 bis 6 Kohlenstoffatomen, eine substituierte oder unsubstituierte Arylgruppe, eine substituierte oder unsubstituierte Heteroarylgruppe, eine Alkoxylgruppe mit 1 bis 6 Kohlenstoffatomen oder eine Alkylaminogruppe mit 1 bis 6 Kohlenstoffatomen steht;

$R^9$ für ein Wasserstoffatom oder eine Alkylthiogruppe mit 1 bis 6 Kohlenstoffatomen steht;

$R^9$ und $R^8$ zusammen mit einem Teil des Grundgerüsts eine cyklische Struktur bilden, die gegebenenfalls ein Schwefelatom als ein die Struktur bildendes Atom enthält und gegebenenfalls eine Alkylgruppe mit 1 bis 6 Kohlenstoffatomen als Substituent aufweist;

$R^{10}$ für ein Wasserstoffatom, eine Aminogruppe, eine Hydroxylgruppe, eine Thiolgruppe, eine Halogenmethylgruppe, eine Alkylgruppe mit 1 bis 6 Kohlenstoffatomen, eine Alkenylgruppe mit 2 bis 6 Kohlenstoffatomen, eine Alkinylgruppe mit 2 bis 6 Kohlenstoffatomen oder eine Alkoxylgruppe mit 1 bis 6 Kohlenstoffatomen steht, mit der Maßgabe, dass die Aminogruppe einen oder mehrere unter einer Formylgruppe, einer Alkylgruppe mit 1 bis 6 Kohlenstoffatomen und einer Acylgruppe mit 2 bis 5 Kohlenstoffatomen ausgewählte Substituenten aufweisen kann;

$X^1$ für ein Halogenatom oder ein Wasserstoffatom steht;

$A^1$ für ein Stickstoffatom oder eine Partialstruktur der folgenden Formel (II):

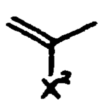

(II)

steht,

worin $X^2$ für ein Wasserstoffatom, eine Aminogruppe, ein Halogenatom, eine Cyanogruppe, eine Halogenmethylgruppe, eine Halogenmethoxylgruppe, eine Alkenylgruppe mit 2 bis 6 Kohlenstoffatomen oder eine Alkinylgruppe mit 2 bis 6 Kohlenstoffatomen steht, mit der Maßgabe, dass die Aminogruppe einen oder mehrere, unter einer Formylgruppe, einer Alkylgruppe mit 2 bis 6 Kohlenstoffatomen und einer Acylgruppe mit 2 bis 5 Kohlenstoffatomen ausgewählte Substituenten aufweisen kann; und

Y für ein Wasserstoffatom, eine Phenylgruppe, eine Acetoxymethylgruppe, eine Pivaloyloxymethylgruppe, eine Ethoxycarbonylgruppe, eine Cholingruppe, eine Dimethylaminomethylgruppe, eine 5-Indanylgruppe, eine Phthalidinylgruppe, eine 5-Alky-2-oxo-1,3-dioxol-4-ylmethylgruppe, eine 3-Acetoxy-2-oxobutylgruppe, eine Alkylgruppe mit 1 bis 6 Kohlenstoffatomen, eine Alkoxylmethylgruppe mit 2 bis 7 Kohlenstoffatomen oder eine aus einer Alkylgruppe mit 1 bis 6 Kohlenstoffatomen und einer Phenylgruppe zusammengesetzte Phenylalkylgruppe steht.

**9.** Verbindung der Formel (I), deren Salze und Hydrate davon:

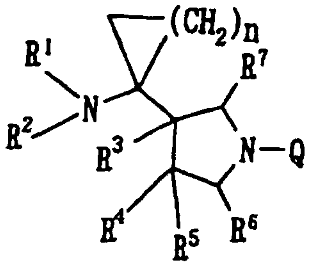

(I)

worin

$R^1$ für ein Wasserstoffatom oder eine Alkylgruppe mit 1 bis 6 Kohlenstoffatomen steht;

$R^2$ für ein Wasserstoffatom oder eine Alkylgruppe mit 1 bis 6 Kohlenstoffatomen steht, mit der Maßgabe, dass die Alkylgruppe einen oder mehrere, unter einer Hydroxylgruppe, einem Halogenatom und einer Alkoxylgruppe mit 1 bis 6 Kohlenstoffatomen ausgewählte Substituenten aufweisen kann;

$R^3$ und $R^5$ jeweils für ein Wasserstoffatom stehen;

$R^4$ für eine Hydroxylgruppe, ein Halogenatom, eine Carbamoylgruppe, eine Alkylgruppe mit 1 bis 6 Kohlenstoffatomen, eine Alkoxylgruppe mit 1 bis 6 Kohlenstoffatomen oder eine Alkylthiogruppe mit 1 bis 6 Kohlenstoffatomen steht, mit der Maßgabe, dass die Alkylgruppe einen oder mehrere, unter einer Hydroxylgruppe, einem Halogenatom und einer Alkoxylgruppe mit 1 bis 6 Kohlenstoffatomen ausgewählte Substituenten aufweisen kann und $R^4$ und der Substituent am Pyrrolidinring der folgenden Formel:

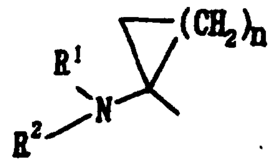

in cis-Konfiguration stehen;

$R^6$ und $R^7$ jeweils unabhängig voneinander für ein Wasserstoffatom oder eine Alkylgruppe mit 1 bis 6 Kohlenstoffatomen stehen;

n eine ganze Zahl mit einem Wert von 1 bis 3 ist; und

Q für eine Partialstruktur der folgenden Formel:

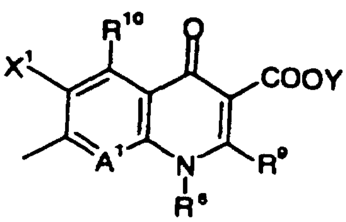

steht,

$R^9$ für ein Wasserstoffatom oder eine Alkylthiogruppe mit 1 bis 6 Kohlenstoffatomen steht;

$R^{10}$ für eine Aminogruppe, eine Hydroxylgruppe, eine Thiolgruppe, eine Halogenmethylgruppe, eine Alkylgruppe mit 1 bis 6 Kohlenstoffatomen, eine Alkenylgruppe mit 2 bis 6 Kohlenstoffatomen, eine Alkinylgruppe mit 2 bis 6 Kohlenstoffatomen oder eine Alkoxylgruppe mit 1 bis 6 Kohlenstoffatomen steht, mit der Maßgabe, dass die Aminogruppe einen oder mehrere unter einer Formylgruppe, einer Alkylgruppe mit 1 bis 6 Kohlenstoffatomen und einer Acylgruppe mit 2 bis 5 Kohlenstoffatomen ausgewählte Substituenten aufweisen kann;

$X^1$ für ein Halogenatom oder ein Wasserstoffatom steht;

$A^1$ für eine Partialstruktur der folgenden Formel (II):

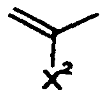 (II)

steht,

worin $X^2$ und $R^8$ zusammen mit einem Teil des Grundgerüsts eine cyklische Struktur bilden, die gegebenenfalls ein Sauerstoffatom, ein Stickstoffatom oder ein Schwefelatom als ein die Struktur bildendes Atom enthält und gegebenenfalls eine Alkylgruppe mit 1 bis 6 Kohlenstoffatomen als Substituent aufweist; und

Y für ein Wasserstoffatom, eine Phenylgruppe, eine Acetoxymethylgruppe, eine Pivaloyloxymethylgruppe, eine Ethoxycarbonylgruppe, eine Cholingruppe, eine Dimethylaminomethylgruppe, eine 5-Indanylgruppe, eine Phthalidinylgruppe, eine 5-Alky-2-oxo-1,3-dioxol-4-ylmethylgruppe, eine 3-Acetoxy-2-oxobutylgruppe, eine Alkylgruppe mit 1 bis 6 Kohlenstoffatomen, eine Alkoxylmethylgruppe mit 2 bis 7 Kohlenstoffatomen oder eine aus einer Alkylgruppe mit 1 bis 6 Kohlenstoffatomen und einer Phenylgruppe zusammengesetzte Phenylalkylgruppe steht.

**10.** Verbindung nach Anspruch 9, worin Q in der Formel (I) eine 2,3-Dihydro-7-oxo-7H-pyrido[1.2.3-de][1.4]benzoxazin-6-carbonsäure-Struktur bildet.

**11.** Verbindung nach Anspruch 10, worin die Struktur einen 3-(S)-Methyl-Substituenten aufweist.

**12.** Verbindung nach Anspruch 9, worin Q in der Formel (I) eine 8-Amino-6-carboxy-9-fluor-2,3-dihydro-3-(S)-methyl-7-oxo-7H-pyrido[1.2.3-de][1.4]benzoxazin-10-yl-Gruppe ist.

**13.** Verbindung nach einem der Ansprüche 1 bis 12, worin der Substituent $R^4$ ein Halogenatom, insbesondere ein Fluoratom, ist.

**14.** Verbindung nach einem der Ansprüche 1 bis 13, worin n = 1 oder 2 ist.

**15.** Verbindung nach einem der Ansprüche 1 bis 13, worin der Substituent $R^4$ ein Fluoratom ist und n = 1 oder 2 ist.

**16.** Verbindung nach einem der Ansprüche 1 bis 8, worin der Substituent $R^8$:

eine Halogencyclopropylgruppe
eine 1,2-cis-2-Halogencyclopropylgruppe;
ein stereochemisch reiner Substituent;
eine (1 R,2S)-2-Halogencyclopropylgruppe;
oder eine (1R,2S)-2-Fluorcyclopropylgruppe

ist.

**17.** Verbindung nach einem der Ansprüche 1 bis 16, worin der Substituent $X^1$ ein Halogenatom, insbesondere ein Fluoratom, ist.

**18.** Verbindung nach einem der Ansprüche 1 bis 16, worin die Substituenten $X^1$ und $X^2$ jeweils ein Halogenatom sind.

**19.** Verbindung nach einem der Ansprüche 1 bis 18, worin $R^{10}$ eine Alkylgruppe mit 1 bis 6 Kohlenstoffatomen ist.

**20.** Verbindung nach einem der Ansprüche 1 bis 19, die stereochemisch rein ist, ihre Salze und Hydrate davon.

**21.** 8-Amino-10-[4-(R)-(1-aminocyclopropyl)-3-(S)-fluor-1-pyrrolidinyl]-9-fluor-2,3-dihydro-3-(S)-methyl-7-oxo-7H-pyrido[1,2,3-de][1,4]benzoxazin-6-carbonsäure, ihre Salze und Hydrate davon.

**22.** Arzneimittel, das als aktiven Inhaltsstoff wenigstens eine Verbindung nach einem der Ansprüche 1 bis 21, Hydrate davon, Salze der Verbindung oder Hydrate der Salze enthält.

**23.** Antimikrobielles Mittel, das als aktiven Inhaltsstoff wenigstens eine Verbindung nach einem der Ansprüche 1 bis 21, Hydrate davon, Salze der Verbindung oder Hydrate der Salze enthält.

**24.** Verwendung einer Verbindung nach einem der Ansprüche 1 bis 21 bei der Herstellung eines Arzneimittels zur Behandlung infektiöser Erkrankungen.

**25.** Verbindung der folgenden Formel (VI), ihre Salze und Hydrate davon:

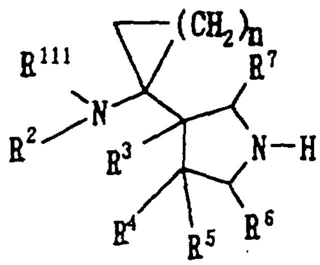

(VI)

worin $R^{111}$ für ein Wasserstoffatom, eine Alkylgruppe mit 1 bis 6 Kohlenstoffatomen oder eine Schutzgruppe der Aminogruppe steht;
$R^2$ für eine Wasserstoffatom oder eine Alkylgruppe mit 1 bis 6 Kohlenstoffatomen steht, mit der Maßgabe, dass die Alkylgruppe einen oder mehrere, unter einer Hydroxylgruppe, einem Halogenatom und einer Alkoxylgruppe mit 1 bis 6 Kohlenstoffatomen ausgewählte Substituenten aufweisen kann;
$R^3$ und $R^5$ jeweils für ein Wasserstoffatom stehen;
$R^4$ für eine Hydroxylgruppe, ein Halogenatom, eine Carbamoylgruppe, eine Alkylgruppe mit 1 bis 6 Kohlenstoffatomen, eine Alkoxylgruppe mit 1 bis 6 Kohlenstoffatomen oder eine Alkylthiogruppe mit 1 bis 6 Kohlenstoffatomen steht, mit der Maßgabe, dass die Alkylgruppe einen oder mehrere, unter einer Hydroxylgruppe, einem Halogenatom und einer Alkoxylgruppe mit 1 bis 6 Kohlenstoffatomen ausgewählte Substituenten aufweisen kann und $R^4$ und der Substituent am Pyrrolidinring der folgenden Formel:

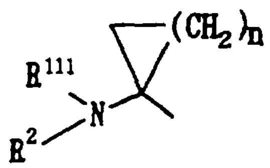

in cis-Konfiguration stehen;
$R^6$ and $R^7$ jeweils unabhängig voneinander für ein Wasserstoffatom oder eine Alkylgruppe mit 1 bis 6 Kohlenstoffatomen stehen;
n für eine ganze Zahl mit einem Wert von 1 bis 3 steht;
mit der Maßgabe, dass die folgenden Verbindungen und Säureadditionssalze davon ausgeschlossen sind:

cis-4-(1-Aminocyclopropyl)-3-(hydroxymethylpyrrolidin);
4-(R)-(1-Aminocyclopropyl)-3-(S)-fluorpyrrolidin;
cis-4-(1-Aminocyclobutyl)-3-fluorpyrrolidin;

wobei in diesen Verbindungen die Aminogruppe geschützt sein kann.

**26.** Verbindung nach Anspruch 25, worin der Substituent $R^4$ ein Halogenatom, insbesondere ein Fluoratom, ist.

**27.** Verbindung der folgenden Formel (VI), ihre Salze und Hydrate davon:

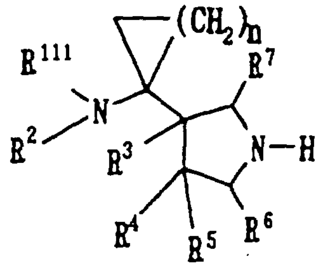

(VI)

worin $R^{111}$ für ein Wasserstoffatom, eine Alkylgruppe mit 1 bis 6 Kohlenstoffatomen oder eine Schutzgruppe der Aminogruppe steht;
$R^2$ für eine Wasserstoffatom oder eine Alkylgruppe mit 1 bis 6 Kohlenstoffatomen steht, mit der Maßgabe, dass die Alkylgruppe einen oder mehrere, unter einer Hydroxylgruppe, einem Halogenatom und einer Alkoxylgruppe mit 1 bis 6 Kohlenstoffatomen ausgewählte Substituenten aufweisen kann;
$R^3$ und $R^5$ jeweils für ein Wasserstoffatom stehen;
$R^4$ für eine Hydroxylgruppe, ein Chloratom, eine Carbamoylgruppe, eine Alkylgruppe mit 1 bis 6 Kohlenstoffatomen, eine Alkoxylgruppe mit 1 bis 6 Kohlenstoffatomen, 2-Hydroxyethyl, 3-Hydroxypropyl oder eine Alkylthiogruppe mit 1 bis 6 Kohlenstoffatomen steht, mit der Maßgabe, dass die Alkylgruppe einen oder mehrere, unter einem Halogenatom und einer Alkoxylgruppe mit 1 bis 6 Kohlenstoffatomen ausgewählte Substituenten aufweisen kann und $R^4$ und der Substituent am Pyrrolidinring der folgenden Formel:

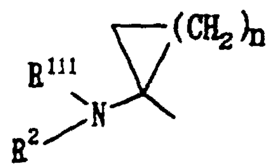

in cis-Konfiguration stehen;
$R^6$ and $R^7$ jeweils unabhängig voneinander für ein Wasserstoffatom oder eine Alkylgruppe mit 1 bis 6 Kohlenstoffatomen stehen;
n für eine ganze Zahl mit einem Wert von 1 bis 3 steht.

**28.** Verbindung der folgenden Formel (VII), ihre Salze und Hydrate davon:

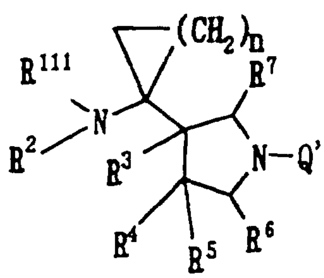

(VII)

worin $R^{111}$ für ein Wasserstoffatom, eine Alkylgruppe mit 1 bis 6 Kohlenstoffatomen oder eine Schutzgruppe der Aminogruppe steht;
$R^2$ für eine Wasserstoffatom oder eine Alkylgruppe mit 1 bis 6 Kohlenstoffatomen steht, mit der Maßgabe, dass die Alkylgruppe einen oder mehrere, unter einer Hydroxylgruppe, einem Halogenatom und einer Alkoxylgruppe mit 1 bis 6 Kohlenstoffatomen ausgewählte Substituenten aufweisen kann;
$R^3$ und $R^5$ jeweils für ein Wasserstoffatom stehen;

$R^4$ für eine Hydroxylgruppe, ein Halogenatom, eine Carbamoylgruppe, eine Alkylgruppe mit 1 bis 6 Kohlenstoffatomen, eine Alkoxylgruppe mit 1 bis 6 Kohlenstoffatomen oder eine Alkylthiogruppe mit 1 bis 6 Kohlenstoffatomen steht, mit der Maßgabe, dass die Alkylgruppe einen oder mehrere, unter einer Hydroxylgruppe, einem Halogenatom und einer Alkoxylgruppe mit 1 bis 6 Kohlenstoffatomen ausgewählte Substituenten aufweisen kann und $R^4$ und der Substituent am Pyrrolidinring der folgenden Formel:

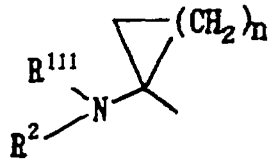

in cis-Konfiguation stehen;
$R^6$ and $R^7$ jeweils unabhängig voneinander für ein Wasserstoffatom oder eine Alkylgruppe mit 1 bis 6 Kohlenstoffatomen stehen;
n für eine ganze Zahl mit einem Wert von 1 bis 3 steht; und
Q' für eine Amino-Schutzgruppe steht, die ausgewählt ist unter gegebenenfalls substituierten Alkoxylcarbonylgruppen, gegebenenfalls substituierten Aralkyloxycarbonylgruppen, gegebenenfalls substituierten Acylgruppen, gegebenenfalls substituierten Alkylgruppen, gegebenenfalls substituierten Aralkyl gruppen und substituierten Silylgruppen,
mit der Maßgabe, dass die folgenden Verbindungen ausgeschlossen sind:

1-Benzyloxycarbonyl-4-(R)-(1-tert-butoxycarbonylaminocyclopropyl)-3-(S)-fluorpyrrolidin;
cis-1-[1-(S)-Phenylethyl]-4-(1-tert-butoxycarbonylaminocyclobutyl)-3-fluorpyrrolidin.

**29.** Verbindung der folgenden Formel (VII), ihre Salze und Hydrate davon:

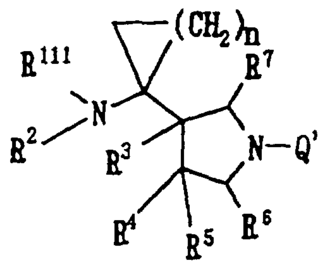

(VII)

worin $R^{111}$ für ein Wasserstoffatom, eine Alkylgruppe mit 1 bis 6 Kohlenstoffatomen oder eine Schutzgruppe der Aminogruppe steht;
$R^2$ für eine Wasserstoffatom oder eine Alkylgruppe mit 1 bis 6 Kohlenstoffatomen steht, mit der Maßgabe, dass die Alkylgruppe einen oder mehrere, unter einer Hydroxylgruppe, einem Halogenatom und einer Alkoxylgruppe mit 1 bis 6 Kohlenstoffatomen ausgewählte Substituenten aufweisen kann;
$R^3$ und $R^5$ jeweils für ein Wasserstoffatom stehen;
$R^4$ für eine Hydroxylgruppe, ein Chloratom, eine Carbamoylgruppe, eine Alkylgruppe mit 1 bis 6 Kohlenstoffatomen, eine Alkoxylgruppe mit 1 bis 6 Kohlenstoffatomen oder eine Alkylthiogruppe mit 1 bis 6 Kohlenstoffatomen steht, mit der Maßgabe, dass die Alkylgruppe einen oder mehrere, unter einer Hydroxylgruppe, einem Halogenatom und einer Alkoxylgruppe mit 1 bis 6 Kohlenstoffatomen ausgewählte Substituenten aufweisen kann und $R^4$ und der Substituent am Pyrrolidinring der folgenden Formel:

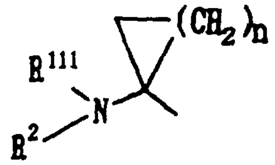

in cis-Konfiguration stehen;
$R^6$ and $R^7$ jeweils unabhängig voneinander für ein Wasserstoffatom oder eine Alkylgruppe mit 1 bis 6 Kohlenstoffatomen stehen;
n für eine ganze Zahl mit einem Wert von 1 bis 3 steht; und
Q' für eine Amino-Schutzgruppe steht, die ausgewählt ist unter gegebenenfalls substituierten Alkoxylcarbonylgruppen, gegebenenfalls substituierten Aralkyloxycarbonylgruppen, gegebenenfalls substituierten Acylgruppen, gegebenenfalls substituierten Alkylgruppen, gegebenenfalls substituierten Aralkyl gruppen und substituierten Silylgruppen.

**30.** Verbindung nach einem der Ansprüche 25 bis 29, worin n = 1 oder 2 ist.

**31.** Verbindung nach Anspruch 25 oder 28, worin der Substituent $R^4$ ein Fluoratom und n = 1 oder 2 ist.

**32.** Verbindung nach einem der Ansprüche 25 bis 31, worin die Schutzgruppe der Aminogruppe ausgewählt ist unter gegebenenfalls substituierten Alkoxycarbonylgruppen, gegebenenfalls substituierten Aralkyloxycarbonylgruppen, gegebenenfalls substituierten Acylgruppen, gegebenenfalls substituierten Alkylgruppen, gegebenenfalls substituierten Aralkylgruppen und substituierten Silylgruppen.

## Revendications

**1.** Composé représenté par la formule (I), et ses sels et hydrates :

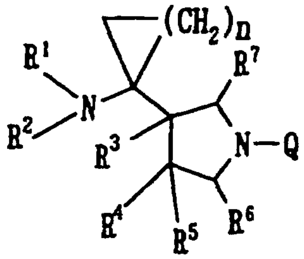

(I)

dans laquelle
$R^1$ représente un atome d'hydrogène ou un groupe alkyle ayant de 1 à 6 atomes de carbone ;
$R^2$ représente un atome d'hydrogène ou un groupe alkyle ayant de 1 à 6 atomes de carbone, dans la mesure où ledit groupe alkyle peut avoir un ou plusieurs substituants choisis dans l'ensemble constitué par un groupe hydroxyle, un atome d'halogène et un groupe alcoxy ayant de 1 à 6 atomes de carbone ;
chacun de $R^3$ et $R^5$ représente un atome d'hydrogène ;
$R^4$ représente un groupe hydroxyle, un atome d'halogène, un groupe carbamoyle, un groupe alkyle ayant de 1 à 6 atomes de carbone, un groupe alcoxy ayant de 1 à 6 atomes de carbone ou un groupe alkylthio ayant de 1 à 6 atomes de carbone, dans la mesure où ledit groupe alkyle peut avoir un ou plusieurs substituants choisis dans l'ensemble constitué par un groupe hydroxyle, un atome d'halogène et un groupe alcoxy ayant de 1 à 6 atomes de carbone ;
$R^4$ et le substituant sur le cycle pyrrolidine de formule

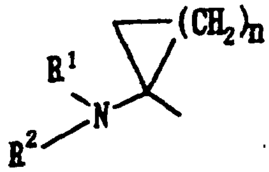

sont localisés en configuration cis ;

chacun de $R^6$ et $R^7$ représente indépendamment un atome d'hydrogène ou un groupe alkyle ayant de 1 à 6 atomes de carbone ;

n est un entier de 1 à 3 ; et

Q représente une structure partielle représentée par la formule suivante :

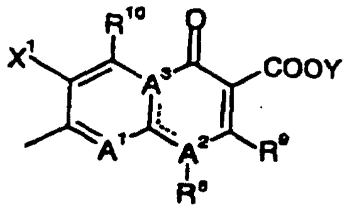

dans laquelle $R^8$ représente un groupe alkyle ayant de 1 à 6 atomes de carbone, un groupe alcényle ayant de 2 à 6 atomes de carbone, un groupe halogénoalkyle ayant de 1 à 6 atomes de carbone, un groupe alkyle cyclique substitué ou non substitué ayant de 3 à 6 atomes de carbone, un groupe aryle substitué ou non substitué, un groupe hétéroaryle substitué ou non substitué, un groupe alcoxy ayant de 1 à 6 atomes de carbone ou un groupe alkylamino ayant de 1 à 6 atomes de carbone ;

$R^9$ représente un atome d'hydrogène ou un groupe alkylthio ayant de 1 à 6 atomes de carbone ;

$R^9$ et $R^8$ peuvent former, ensemble avec une partie du noyau parent, une structure cyclique contenant éventuellement un atome de soufre formant un atome constitutif de celle-ci et ayant éventuellement, à titre de substituant, un groupe alkyle ayant de 1 à 6 atomes de carbone ;

$R^{10}$ représente un atome d'hydrogène, un groupe amino, un groupe hydroxyle, un groupe thiol, un groupe halogénométhyle, un groupe alkyle ayant de 1 à 6 atomes de carbone, un groupe alcényle ayant de 2 à 6 atomes de carbone, un groupe alcynyle ayant de 2 à 6 atomes de carbone ou un groupe alcoxy ayant de 1 à 6 atomes de carbone, dans la mesure où ledit groupe amino peut porter un ou plusieurs substituants choisis dans l'ensemble constitué par un groupe formyle, un groupe alkyle ayant de 1 à 6 atomes de carbone et un groupe acyle ayant de 2 à 5 atomes de carbone ;

$X^1$ représente un atome d'halogène ou un atome d'hydrogène ;

$A^1$ représente un atome d'azote ou une structure partielle représentée par la formule (II) suivante :

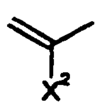

(II)

où $X^2$ représente un atome d'hydrogène, un groupe amino, un atome d'halogène, un groupe cyano, un groupe halogénométhyle, un groupe halogénométhoxy, un groupe alkyle ayant de 1 à 6 atomes de carbone, un groupe alcényle ayant de 2 à 6 atomes de carbone, un groupe alcynyle ayant de 2 à 6 atomes de carbone ou un groupe alcoxy ayant de 1 à 6 atomes de carbone, dans la mesure où ledit groupe amino peut porter un ou plusieurs substituants choisis dans l'ensemble constitué par un groupe formyle, un groupe alkyle ayant de 1 à 6 atomes de carbone et un groupe acyle ayant de 2 à 5 atomes de carbone, et $X^2$ et $R^8$ peuvent former, ensemble avec une partie du noyau parent, une structure cyclique contenant éventuellement un atome d'oxygène, un atome d'azote

ou un atome de soufre à titre d'atome constitutif de celle-ci et ayant éventuellement un groupe alkyle ayant de 1 à 6 atomes de carbone en tant que substituant ;

chacun de $A^2$ et $A^3$ représente un atome d'azote ou un atome de carbone, dans la mesure où $A^2$ et $A^3$ peuvent former, ensemble avec l'atome de carbone auquel ils sont liés, une structure partielle représentée par la formule suivante :

$$>C=C(A^1=)-N(R^8)-$$

ou une structure partielle représentée par la formule suivante :

$$>N-C(A^1=)=C(R^8)-$$

et

Y représente un atome d'hydrogène, un groupe phényle, un groupe acétoxyméthyle, un groupe pivaloyloxyméthyle, un groupe éthoxycarbonyle, un groupe choline, un groupe diméthylaminoéthyle, un groupe 5-indanyle, un groupe phtalidinyle, un groupe 5-alkyl-2-oxo-1,3-dioxol-4-ylméthyle, un groupe 3-acétoxy-2-oxobutyle, un groupe alkyle ayant de 1 à 6 atomes de carbone, un groupe alcoxyméthyle ayant de 2 à 7 atomes de carbone ou un groupe phénylalkyle composé d'un groupe alkyle ayant de 1 à 6 atomes de carbone et d'un groupe phényle,

à l'exclusion des composés suivants :

acide 5-amino-7-[cis-4-{1-aminocyclopropyl}-3-hydroxyméthyl-1-pyrrolidinyl]-1-cyclopropyl-6-fluoro-1,4-dihydro-8-méthyl-4-oxoquinoline-3-carboxylique ;
chlorhydrate d'acide 5-amino-7-[4-(R)-{1-aminocyclopropyl}-3-(S)-fluoro-1-pyrrolidinyl]-6-fluoro-1-[2-(S)-fluoro-3-(R)-cyclopropyl]-1,4-dihydro-8-méthyl-4-oxoquinoline-3-carboxylique ;
acide 5-amino-7-[4-(R)-{1-aminocyclopropyl}-3-(S)-fluoro-1-pyrrolidinyl]-6-fluoro-1-[2-(S)-fluoro-1-(R)-cyclopropyl]-1,4-dihydro-8-méthoxy-4-oxoquinoline-3-carboxylique ;
acide 10-[4-(R)-{1-aminocyclopropyl}-3-(S)-fluoro-1-pyrrolidinyl]-9-fluoro-2,3-dihydro-3-(S)-méthyl-7-oxo-7H-pyrido[1.2.3-de][1.4]benzoxazine-6-carboxylique ;
acide 7-[4-(R)-{1-aminocyclopropyl}-3-(S)-fluoro-1-pyrrolidinyl]-6-fluoro-1-[2-(S)-fluoro-1-(R)-cyclopropyl]-1,4-dihydro-8-méthoxy-4-oxoquinoline-3-carboxylique ;
acide 5-amino-7-[cis-4-{1-aminocyclobutyl}-3-fluoro-1-pyrrolidinyl]-6-fluoro-1-[2-(S)-fluoro-1-(R)-cyclopropyl]-1,4-dihydro-8-méthyl-4-oxoquinoline-3-carboxylique.

**2.** Composé selon la revendication 1 représenté par la formule (Ia) et ses sels et hydrates :

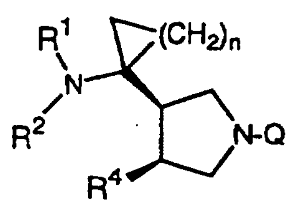

dans laquelle chacun de $R^1$, $R^2$, $R^4$, n et Q est tel que défini dans la revendication 1.

**3.** Composé selon la revendication 1 ou 2, dans lequel Q, dans la formule (I), a une structure représentée par les formules suivantes, et ses sels et hydrates :

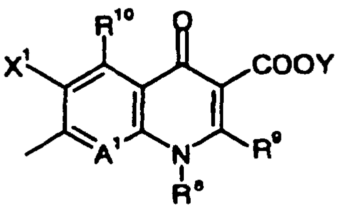

ou

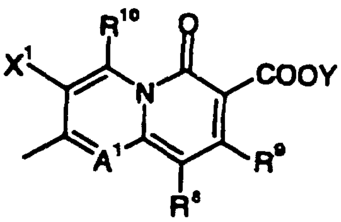

où chacun de $R^8$, $R^9$, $R^{10}$, $A^1$, $X^1$ et Y est tel que défini dans la revendication 1.

**4.** Composé selon la revendication 1 ou 2, dans lequel Q, dans la formule (I), a une structure représentée par la formule suivante, et ses sels et hydrates :

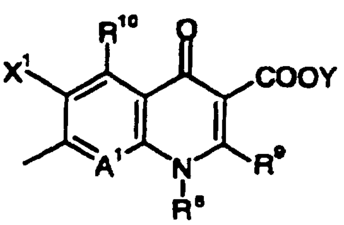

où chacun de $R^8$, $R^9$, $R^{10}$, $A^1$, $X^1$ et Y est tel que défini dans la revendication 1.

**5.** Composé selon la revendication 1, 2, 3 ou 4, dans lequel Q, dans la formule (I), est :

un groupe 6-carboxy-9-fluoro-2,3-dihydro-3-(S)-méthyl-7-oxo-7H-pyrido[1,2,3-de][1,4]-benzoxazin-10-yle ;
un groupe 8-amino-6-carboxy-9-fluoro-2,3-dihydro-3-(S)-méthyl-7-oxo-7H-pyrido[1,2,3-de][1,4]-benzoxazin-10-yle ;
un groupe 5-amino-3-carboxy-6-fluoro-1-[2-(S)-fluoro-1-(R)-cyclopropyl]-1,4-dihydro-8-méthoxy-4-oxoquinolin-7-yle ;
un groupe 5-amino-3-carboxy-6-fluoro-1-[2-(S)-fluoro-1-(R)-cyclopropyl]-1,4-dihydro-8-méthyl-4-oxoquinolin-7-yle ; ou
un groupe 3-carboxy-6-fluoro-1-[2-(S)-fluoro-1-(R)-cyclopropyl]-1,4-dihydro-8-méthoxy-4-oxoquinolin-7-yle.

**6.** Composé représenté par la formule (I), et ses sels et hydrates :

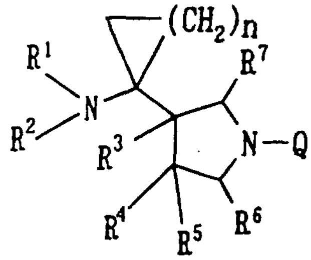

(I)

dans laquelle

$R^1$ représente un atome d'hydrogène ou un groupe alkyle ayant de 1 à 6 atomes de carbone ;

$R^2$ représente un atome d'hydrogène ou un groupe alkyle ayant de 1 à 6 atomes de carbone, dans la mesure où ledit groupe alkyle peut avoir un ou plusieurs substituants choisis dans l'ensemble constitué par un groupe hydroxyle, un atome d'halogène et un groupe alcoxy ayant de 1 à 6 atomes de carbone ;

chacun de $R^3$ et $R^5$ représente un atome d'hydrogène ;

$R^4$ représente un groupe hydroxyle, un atome d'halogène, un groupe carbamoyle, un groupe alkyle ayant de 1 à 6 atomes de carbone, un groupe alcoxy ayant de 1 à 6 atomes de carbone ou un groupe alkylthio ayant de 1 à 6 atomes de carbone, dans la mesure où ledit groupe alkyle peut avoir un ou plusieurs substituants choisis dans l'ensemble, constitué par un groupe hydroxyle, un atome d'halogène et un groupe alcoxy ayant de 1 à 6 atomes de carbone, et $R^4$ et le substituant sur le cycle pyrrolidine de formule

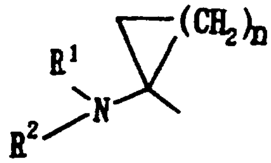

sont localisés en configuration cis ;

chacun de $R^6$ et $R^7$ représente indépendamment un atome d'hydrogène ou un groupe alkyle ayant de 1 à 6 atomes de carbone ;

n est un entier de 1 à 3 ; et

Q représente une structure partielle représentée par la formule suivante :

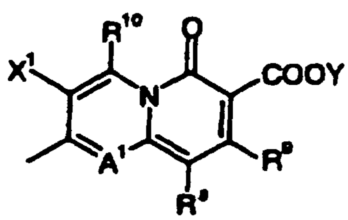

dans laquelle $R^8$ représente un groupe alkyle ayant de 1 à 6 atomes de carbone, un groupe alcényle ayant de 2 à 6 atomes de carbone, un groupe halogénoalkyle ayant de 1 à 6 atomes de carbone, un groupe alkyle cyclique substitué ou non substitué ayant de 3 à 6 atomes de carbone, un groupe aryle substitué ou non substitué, un groupe hétéroaryle substitué ou non substitué, un groupe alcoxy ayant de 1 à 6 atomes de carbone ou un groupe alkylamino ayant de 1 à 6 atomes de carbone ;

$R^9$ représente un atome d'hydrogène ou un groupe alkylthio ayant de 1 à 6 atomes de carbone ;

$R^9$ et $R^8$ peuvent former, ensemble avec une partie du noyau parent, une structure cyclique contenant éventuellement un atome de soufre formant un atome constitutif de celle-ci et ayant éventuellement, à titre de substituant, un groupe alkyle ayant de 1 à 6 atomes de carbone ;

$R^{10}$ représente un atome d'hydrogène, un groupe amino, un groupe hydroxyle, un groupe thiol, un groupe halogénométhyle, un groupe alkyle ayant de 1 à 6 atomes de carbone, un groupe alcényle ayant de 2 à 6 atomes de carbone, un groupe alcynyle ayant de 2 à 6 atomes de carbone ou un groupe alcoxy ayant de 1 à 6 atomes de carbone, dans la mesure où ledit groupe amino peut porter un ou plusieurs substituants choisis dans l'ensemble constitué par un groupe formyle, un groupe alkyle ayant de 1 à 6 atomes de carbone et un groupe acyle ayant de 2 à 5 atomes de carbone ;

$X^1$ représente un atome d'halogène ou un atome d'hydrogène ;

$A^1$ représente un atome d'azote ou une structure partielle représentée par la formule (II) suivante :

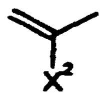

(II)

où $X^2$ représente un atome d'hydrogène, un groupe amino, un atome d'halogène, un groupe cyano, un groupe halogénométhyle, un groupe halogénométhoxy, un groupe alkyle ayant de 1 à 6 atomes de carbone, un groupe alcényle ayant de 2 à 6 atomes de carbone, un groupe alcynyle ayant de 2 à 6 atomes de carbone ou un groupe alcoxy ayant de 1 à 6 atomes de carbone, dans la mesure où ledit groupe amino peut porter un ou plusieurs substituants choisis dans l'ensemble constitué par un groupe formyle, un groupe alkyle ayant de 1 à 6 atomes de carbone et un groupe acyle ayant de 2 à 5 atomes de carbone, et $X^2$ et $R^8$ peuvent former, ensemble avec une partie du noyau parent, une structure cyclique contenant éventuellement un atome d'oxygène, un atome d'azote ou un atome de soufre à titre d'atome constitutif de celle-ci et ayant éventuellement un groupe alkyle ayant de 1 à 6 atomes de carbone en tant que substituant ;

Y représente un atome d'hydrogène, un groupe phényle, un groupe acétoxyméthyle, un groupe pivaloyloxyméthyle, un groupe éthoxycarbonyle, un groupe choline, un groupe diméthylaminoéthyle, un groupe 5-indanyle, un groupe phtalidinyle, un groupe 5-alkyl-2-oxo-1,3-dioxol-4-ylméthyle, un groupe 3-acétoxy-2-oxobutyle, un groupe alkyle ayant de 1 à 6 atomes de carbone, un groupe alcoxyméthyle ayant de 2 à 7 atomes de carbone ou un groupe phénylalkyle composé d'un groupe alkyle ayant de 1 à 6 atomes de carbone et d'un groupe phényle.

**7.** Composé représenté par la formule (I), et ses sels et hydrates :

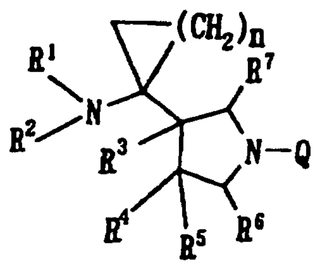

(I)

dans laquelle

$R^1$ représente un atome d'hydrogène ou un groupe alkyle ayant de 1 à 6 atomes de carbone ;

$R^2$ représente un atome d'hydrogène ou un groupe alkyle ayant de 1 à 6 atomes de carbone, dans la mesure où ledit groupe alkyle peut avoir un ou plusieurs substituants choisis dans l'ensemble constitué par un groupe hydroxyle, un atome d'halogène et un groupe alcoxy ayant de 1 à 6 atomes de carbone ;

chacun de $R^3$ et $R^5$ représente un atome d'hydrogène ;

$R^4$ représente un groupe hydroxyle, un atome d'halogène, un groupe carbamoyle, un groupe alkyle ayant de 1 à 6 atomes de carbone, un groupe alcoxy ayant de 1 à 6 atomes de carbone ou un groupe alkylthio ayant de 1 à 6 atomes de carbone, dans la mesure où ledit groupe alkyle peut avoir un ou plusieurs substituants choisis dans l'ensemble constitué par un groupe hydroxyle, un atome d'halogène et un groupe alcoxy ayant de 1 à 6 atomes de carbone, et $R^4$ et le substituant sur le cycle pyrrolidine de formule

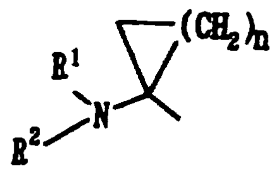

sont localisés en configuration cis ;

chacun de $R^6$ et $R^7$ représente indépendamment un atome d'hydrogène ou un groupe alkyle ayant de 1 à 6 atomes de carbone ;

n est un entier de 1 à 3 ; et

Q représente une structure partielle représentée par la formule suivante :

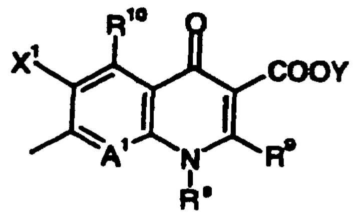

dans laquelle $R^8$ représente un groupe alkyle ayant de 1 à 6 atomes de carbone, un groupe alcényle ayant de 2 à 6 atomes de carbone, un groupe halogénoalkyle ayant de 1 à 6 atomes de carbone, un groupe alkyle cyclique substitué ou non substitué ayant de 3 à 6 atomes de carbone, un groupe aryle substitué ou non substitué, un groupe hétéroaryle substitué ou non substitué, un groupe alcoxy ayant de 1 à 6 atomes de carbone ou un groupe alkylamino ayant de 1 à 6 atomes de carbone ;

$R^9$ représente un atome d'hydrogène ou un groupe alkylthio ayant de 1 à 6 atomes de carbone ;

$R^9$ et $R^8$ peuvent former, ensemble avec une partie du noyau parent, une structure cyclique contenant éventuellement un atome de soufre formant un atome constitutif de celle-ci et ayant éventuellement, à titre de substituant, un groupe alkyle ayant de 1 à 6 atomes de carbone ;

$R^{10}$ représente un groupe amino portant un ou plusieurs substituants choisis dans l'ensemble constitué par un groupe formyle, un groupe alkyle ayant de 1 à 6 atomes de carbone et un groupe acyle ayant de 2 à 5 atomes de carbone, un groupe hydroxyle, un groupe thiol, un groupe halogénométhyle, un groupe alkyle ayant de 1 à 6 atomes de carbone, un groupe alcényle ayant de 2 à 6 atomes de carbone, un groupe alcynyle ayant de 2 à 6 atomes de carbone ou un groupe alcoxy ayant de 1 à 6 atomes de carbone ;

$X^1$ représente un atome d'halogène ou un atome d'hydrogène ;

$A^1$ représente un atome d'azote ou une structure partielle représentée par la formule (II) suivante :

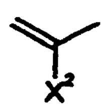

(II)

où $X^2$ représente un atome d'hydrogène, un groupe amino, un atome d'halogène, un groupe cyano, un groupe halogénométhyle, un groupe halogénométhoxy, un groupe alkyle ayant de 1 à 6 atomes de carbone, un groupe alcényle ayant de 2 à 6 atomes de carbone, un groupe alcynyle ayant de 2 à 6 atomes de carbone ou un groupe alcoxy ayant de 1 à 6 atomes de carbone, dans la mesure où ledit groupe amino peut porter un ou plusieurs substituants choisis dans l'ensemble constitué par un groupe formyle, un groupe alkyle ayant de 1 à 6 atomes de carbone et un groupe acyle ayant de 2 à 5 atomes de carbone ;

Y représente un atome d'hydrogène, un groupe phényle, un groupe acétoxyméthyle, un groupe pivaloyloxyméthyle, un groupe éthoxycarbonyle, un groupe choline, un groupe diméthylaminoéthyle, un groupe 5-indanyle,

un groupe phtalidinyle, un groupe 5-alkyl-2-oxo-1,3-dioxol-4-ylméthyle, un groupe 3-acétoxy-2-oxobutyle, un groupe alkyle ayant de 1 à 6 atomes de carbone, un groupe alcoxyméthyle ayant de 2 à 7 atomes de carbone ou un groupe phénylalkyle composé d'un groupe alkyle ayant de 1 à 6 atomes de carbone et d'un groupe phényle.

**8.** Composé représenté par la formule (I), et ses sels et hydrates :

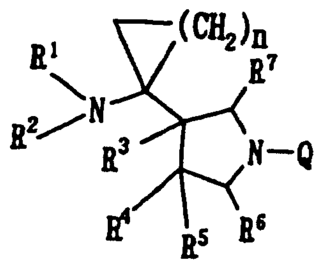

(I)

dans laquelle

$R^1$ représente un atome d'hydrogène ou un groupe alkyle ayant de 1 à 6 atomes de carbone ;

$R^2$ représente un atome d'hydrogène ou un groupe alkyle ayant de 1 à 6 atomes de carbone, dans la mesure où ledit groupe alkyle peut avoir un ou plusieurs substituants choisis dans l'ensemble constitué par un groupe hydroxyle, un atome d'halogène et un groupe alcoxy ayant de 1 à 6 atomes de carbone ;

chacun de $R^3$ et $R^5$ représente un atome d'hydrogène ;

$R^4$ représente un groupe hydroxyle, un atome d'halogène, un groupe carbamoyle, un groupe alkyle ayant de 1 à 6 atomes de carbone, un groupe alcoxy ayant de 1 à 6 atomes de carbone ou un groupe alkylthio ayant de 1 à 6 atomes de carbone, dans la mesure où ledit groupe alkyle peut avoir un ou plusieurs substituants choisis dans l'ensemble constitué par un groupe hydroxyle, un atome d'halogène et un groupe alcoxy ayant de 1 à 6 atomes de carbone, dans la mesure où ledit groupe alkyle peut avoir un ou plusieurs substituants choisis dans l'ensemble constitué par un groupe hydroxyle, un atome d'halogène et un groupe alcoxy ayant de 1 à 6 atomes de carbone, et $R^4$ et le substituant sur le cycle pyrrolidine de formule

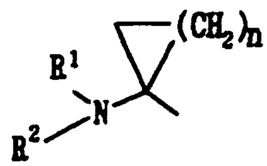

sont localisés en configuration cis ;

chacun de $R^6$ et $R^7$ représente indépendamment un atome d'hydrogène ou un groupe alkyle ayant de 1 à 6 atomes de carbone ;

n est un entier de 1 à 3 ; et

Q représente une structure partielle représentée par la formule suivante :

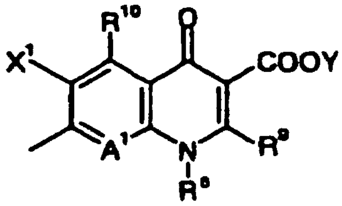

dans laquelle $R^8$ représente un groupe alkyle ayant de 1 à 6 atomes de carbone, un groupe alcényle ayant de 2 à 6 atomes de carbone, un groupe halogénoalkyle ayant de 1 à 6 atomes de carbone, un groupe alkyle

cyclique substitué ou non substitué ayant de 3 à 6 atomes de carbone, un groupe aryle substitué ou non substitué, un groupe hétéroaryle substitué ou non substitué, un groupe alcoxy ayant de 1 à 6 atomes de carbone ou un groupe alkylamino ayant de 1 à 6 atomes de carbone ;

$R^9$ représente un atome d'hydrogène ou un groupe alkylthio ayant de 1 à 6 atomes de carbone ;

$R^9$ et $R^8$ peuvent former, ensemble avec une partie du noyau parent, une structure cyclique contenant éventuellement un atome de soufre formant un atome constitutif de celle-ci et ayant éventuellement, à titre de substituant, un groupe alkyle ayant de 1 à 6 atomes de carbone ;

$R^{10}$ représente un atome d'hydrogène, un groupe amino, un groupe hydroxyle, un groupe thiol, un groupe halogénométhyle, un groupe alkyle ayant de 1 à 6 atomes de carbone, un groupe alcényle ayant de 2 à 6 atomes de carbone, un groupe alcynyle ayant de 2 à 6 atomes de carbone ou un groupe alcoxy ayant de 1 à 6 atomes de carbone, dans la mesure où ledit groupe amino peut porter un ou plusieurs substituants choisis dans l'ensemble constitué par un groupe formyle, un groupe alkyle ayant de 1 à 6 atomes de carbone et un groupe acyle ayant de 2 à 5 atomes de carbone ;

$X^1$ représente un atome d'halogène ou un atome d'hydrogène ;

$A^1$ représente un atome d'azote ou une structure partielle représentée par la formule (II) suivante :

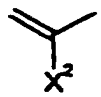

(II)

où $X^2$ représente un atome d'hydrogène, un groupe amino, un atome d'halogène, un groupe cyano, un groupe halogénométhyle, un groupe halogénométhoxy, un groupe alcényle ayant de 2 à 6 atomes de carbone ou un groupe alcynyle ayant de 2 à 6 atomes de carbone, dans la mesure où ledit groupe amino peut porter un ou plusieurs substituants choisis dans l'ensemble constitué par un groupe formyle, un groupe alkyle ayant de 1 à 6 atomes de carbone et un groupe acyle ayant de 2 à 5 atomes de carbone ; et

Y représente un atome d'hydrogène, un groupe phényle, un groupe acétoxyméthyle, un groupe pivaloyloxyméthyle, un groupe éthoxycarbonyle, un groupe choline, un groupe diméthylaminoéthyle, un groupe 5-indanyle, un groupe phtalidinyle, un groupe 5-alkyl-2-oxo-1,3-dioxol-4-ylméthyle, un groupe 3-acétoxy-2-oxobutyle, un groupe alkyle ayant de 1 à 6 atomes de carbone, un groupe alcoxyméthyle ayant de 2 à 7 atomes de carbone ou un groupe phénylalkyle composé d'un groupe alkyle ayant de 1 à 6 atomes de carbone et d'un groupe phényle.

**9.** Composé représenté par la formule (I), et ses sels et hydrates :

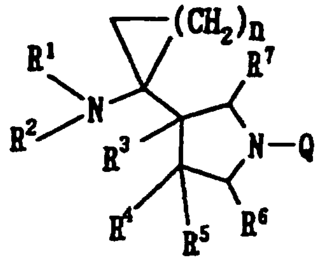

(I)

dans laquelle

$R^1$ représente un atome d'hydrogène ou un groupe alkyle ayant de 1 à 6 atomes de carbone ;

$R^2$ représente un atome d'hydrogène ou un groupe alkyle ayant de 1 à 6 atomes de carbone, dans la mesure où ledit groupe alkyle peut avoir un ou plusieurs substituants choisis dans l'ensemble constitué par un groupe hydroxyle, un atome d'halogène et un groupe alcoxy ayant de 1 à 6 atomes de carbone ;

chacun de $R^3$ et $R^5$ représente un atome d'hydrogène ;

$R^4$ représente un groupe hydroxyle, un atome d'halogène, un groupe carbamoyle, un groupe alkyle ayant de 1 à 6 atomes de carbone, un groupe alcoxy ayant de 1 à 6 atomes de carbone ou un groupe alkylthio ayant de 1 à 6 atomes de carbone, dans la mesure où ledit groupe alkyle peut avoir un ou plusieurs substituants choisis dans l'ensemble constitué par un groupe hydroxyle, un atome d'halogène et un groupe alcoxy ayant de 1 à 6

atomes de carbone, et $R^4$ et le substituant sur le cycle pyrrolidine de formule

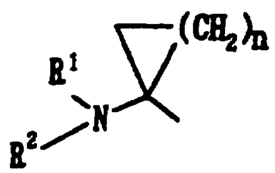

sont localisés en configuration cis ;

chacun de $R^6$ et $R^7$ représente indépendamment un atome d'hydrogène ou un groupe alkyle ayant de 1 à 6 atomes de carbone ;

n est un entier de 1 à 3 ; et

Q représente une structure partielle représentée par la formule suivante :

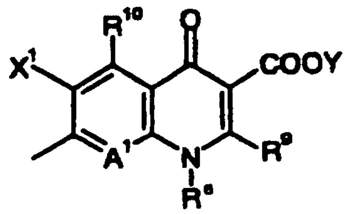

dans laquelle $R^9$ représente un atome d'hydrogène ou un groupe alkylthio ayant de 1 à 6 atomes de carbone ;

$R^{10}$ représente un groupe amino, un groupe hydroxyle, un groupe thiol, un groupe halogénométhyle, un groupe alkyle ayant de 1 à 6 atomes de carbone, un groupe alcényle ayant de 2 à 6 atomes de carbone, un groupe alcynyle ayant de 2 à 6 atomes de carbone ou un groupe alcoxy ayant de 1 à 6 atomes de carbone, dans la mesure où ledit groupe amino peut porter un ou plusieurs substituants choisis dans l'ensemble constitué par un groupe formyle, un groupe alkyle ayant de 1 à 6 atomes de carbone et un groupe acyle ayant de 2 à 5 atomes de carbone ;

$X^1$ représente un atome d'halogène ou un atome d'hydrogène ;

$A^1$ représente une structure partielle représentée par la formule (II) suivante :

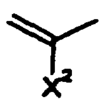

(II)

où $X^2$ et $R^8$ forment, ensemble avec une partie du noyau parent, une structure cyclique contenant éventuellement un atome d'oxygène, un atome d'azote ou un atome de soufre à titre d'atome constitutif de celle-ci et ayant éventuellement un groupe alkyle ayant de 1 à 6 atomes de carbone en tant que substituant ; et

Y représente un atome d'hydrogène, un groupe phényle, un groupe acétoxyméthyle, un groupe pivaloyloxyméthyle, un groupe éthoxycarbonyle, un groupe choline, un groupe diméthylaminoéthyle, un groupe 5-indanyle, un groupe phtalidinyle, un groupe 5-alkyl-2-oxo-1,3-dioxol-4-ylméthyle, un groupe 3-acétoxy-2-oxobutyle, un groupe alkyle ayant de 1 à 6 atomes de carbone, un groupe alcoxyméthyle ayant de 2 à 7 atomes de carbone ou un groupe phénylalkyle composé d'un groupe alkyle ayant de 1 à 6 atomes de carbone et d'un groupe phényle.

**10.** Composé selon la revendication 9, dans lequel Q, dans la formule (I), forme une structure d'acide 2,3-dihydro-7-oxo-7H-pyrido[1.2.3-de][1.4]benzoxacine-6-carboxylique.

**11.** Composé selon la revendication 10, dans lequel la structure a un substituant 3-(S)-méthyle.

**12.** Composé selon la revendication 9, dans lequel Q, dans la formule (I), est un groupe 8-amino-6-carboxy-9-fluoro-2,3-dihydro-3-(S)-méthyl-7-oxo-7H-pyrido[1.2.3-de][1.4]benzoxacine-10-yl.

**13.** Composé selon l'une quelconque des revendications 1 à 12, dans lequel le substituant $R^4$ est un atome d'halogène, en particulier un atome de fluor.

**14.** Composé selon l'une quelconque des revendications 1 à 13, dans lequel n vaut 1 ou 2.

**15.** Composé selon l'une quelconque des revendications 1 à 13, dans lequel le substituant $R^4$ est un atome de fluor et n vaut 1 ou 2.

**16.** Composé selon l'une quelconque des revendications 1 à 8, dans lequel le substituant $R^8$ est :

un groupe halogénocyclopropyle ;
un groupe 1,2-cis-2-halogénocyclopropyle ;
un groupe stéréochimiquement pur ;
un groupe (1R,2S)-2-halogénocyclopropyle ; ou
un groupe (1R,2S)-2-fluorocyclopropyle.

**17.** Composé selon l'une quelconque des revendications 1 à 16, dans lequel le substituant $X^1$ est un atome d'halogène, en particulier un atome de fluor.

**18.** Composé selon l'une quelconque des revendications 1 à 16, dans lequel chacun des substituants $X^1$ et $X^2$ est un atome d'halogène.

**19.** Composé selon l'une quelconque des revendications 1 à 18, dans lequel $R^{10}$ est un groupe alkyle ayant de 1 à 6 atomes de carbone.

**20.** Composé selon l'une quelconque des revendications 1 à 19, qui est stéréochimiquement pur, et ses sels et hydrates.

**21.** Acide 8-amino-10-[4-(R)-(1-aminocyclopropyl)-3-(S)-fluoro-1-pyrrolidinyl]-9-fluoro-2,3-dihydro-3-(S)-méthyl-7-oxo-7H-pyrido[1,2,3-de][1,4]benzoxazine-6-carboxylique, et ses sels et hydrates.

**22.** Médicament contenant, à titre d'ingrédient actif, au moins un composé selon l'une quelconque des revendications 1 à 21, ses hydrates, des sels dudit composé ou des hydrates desdits sels.

**23.** Agent antimicrobien contenant, à titre d'ingrédient actif, au moins un composé selon l'une quelconque des revendications 1 à 21, ses hydrates, des sels dudit composé ou des hydrates desdits sels.

**24.** Utilisation d'un composé selon l'une quelconque des revendications 1 à 21, dans la fabrication d'un médicament destiné au traitement de maladies infectieuses.

**25.** Composé représenté par la formule (VI) suivante, et ses sels et hydrates :

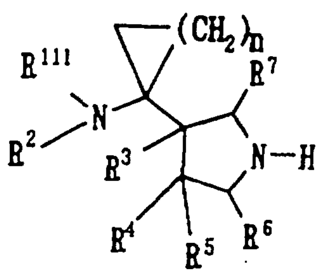

(VI)

dans laquelle

$R^{111}$ représente un atome d'hydrogène, un groupe alkyle ayant de 1 à 6 atomes de carbone ou un groupe protecteur du groupe amino ;

$R^2$ représente un atome d'hydrogène ou un groupe alkyle ayant de 1 à 6 atomes de carbone, dans la mesure où ledit groupe alkyle peut avoir un ou plusieurs substituants choisis dans l'ensemble constitué par un groupe

hydroxyle, un atome d'halogène et un groupe alcoxy ayant de 1 à 6 atomes de carbone ;

chacun de $R^3$ et $R^5$ représente un atome d'hydrogène ;

$R^4$ représente un groupe hydroxyle, un atome d'halogène, un groupe carbamoyle, un groupe alkyle ayant de 1 à 6 atomes de carbone, un groupe alcoxy ayant de 1 à 6 atomes de carbone ou un groupe alkylthio ayant de 1 à 6 atomes de carbone, dans la mesure où ledit groupe alkyle peut avoir un ou plusieurs substituants choisis dans l'ensemble constitué par un groupe hydroxyle, un atome d'halogène et un groupe alcoxy ayant de 1 à 6 atomes de carbone, et $R^4$ et le substituant sur le cycle pyrrolidine de formule

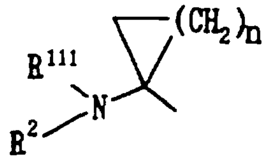

sont localisés en configuration cis ;

chacun de $R^6$ et $R^7$ représente indépendamment un atome d'hydrogène ou un groupe alkyle ayant de 1 à 6 atomes de carbone ;

n est un entier de 1 à 3 ;

à l'exclusion des composés suivants :

cis-4-(1-aminocyclopropyl)-3-(hydroxyméthylpyrrolidine) ;
4-(R)-(1-aminocyclopropyl)-3-(S)-fluoropyrrolidine ;
cis-4-(1-aminocyclobutyl)-3-fluoropyrrolidine ;
composés dans lesquels le groupe amino peut être protégé.

**26.** Composé selon la revendication 25, dans lequel le substituant $R^4$ est un atome d'halogène, en particulier un atome de fluor.

**27.** Composé représenté par la formule (VI) suivante, et ses sels et hydrates :

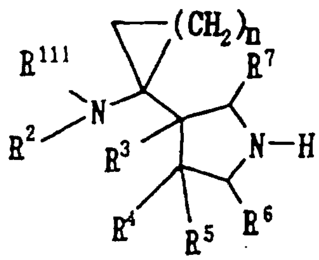

(VI)

dans laquelle

$R^{111}$ représente un atome d'hydrogène, un groupe alkyle ayant de 1 à 6 atomes de carbone ou un groupe protecteur du groupe amino ;

$R^2$ représente un atome d'hydrogène ou un groupe alkyle ayant de 1 à 6 atomes de carbone, dans la mesure où ledit groupe alkyle peut avoir un ou plusieurs substituants choisis dans l'ensemble constitué par un groupe hydroxyle, un atome d'halogène et un groupe alcoxy ayant de 1 à 6 atomes de carbone ;

chacun de $R^3$ et $R^5$ représente un atome d'hydrogène ;

$R^4$ représente un groupe hydroxyle, un atome de chlore, un groupe carbamoyle, un groupe alkyle ayant de 1 à 6 atomes de carbone, un groupe alcoxy ayant de 1 à 6 atomes de carbone, le groupe 2-hydroxyéthyle, le groupe 3-hydroxypropyle ou un groupe alkylthio ayant de 1 à 6 atomes de carbone, dans la mesure où ledit groupe alkyle peut avoir un ou plusieurs substituants choisis dans l'ensemble constitué par un atome d'halogène et un groupe alcoxy ayant de 1 à 6 atomes de carbone, et $R^4$ et le substituant sur le cycle pyrrolidine de formule

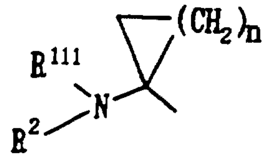

sont localisés en configuration cis ;

chacun de $R^6$ et $R^7$ représente indépendamment un atome d'hydrogène ou un groupe alkyle ayant de 1 à 6 atomes de carbone ;

n est un entier de 1 à 3.

**28.** Composé représenté par la formule (VII) suivante, et ses sels et hydrates :

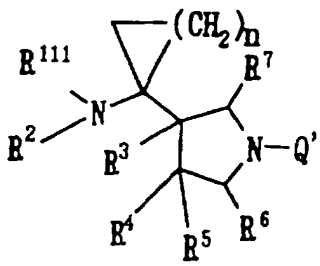

(VII)

dans laquelle

$R^{111}$ représente un atome d'hydrogène, un groupe alkyle ayant de 1 à 6 atomes de carbone ou un groupe protecteur du groupe amino ;

$R^2$ représente un atome d'hydrogène ou un groupe alkyle ayant de 1 à 6 atomes de carbone, dans la mesure où ledit groupe alkyle peut avoir un ou plusieurs substituants choisis dans l'ensemble constitué par un groupe hydroxyle, un atome d'halogène et un groupe alcoxy ayant de 1 à 6 atomes de carbone ;

chacun de $R^3$ et $R^5$ représente un atome d'hydrogène ;

$R^4$ représente un groupe hydroxyle, un atome d'halogène, un groupe carbamoyle, un groupe alkyle ayant de 1 à 6 atomes de carbone, un groupe alcoxy ayant de 1 à 6 atomes de carbone ou un groupe alkylthio ayant de 1 à 6 atomes de carbone, dans la mesure où ledit groupe alkyle peut avoir un ou plusieurs substituants choisis dans l'ensemble constitué par un groupe hydroxyle, un atome d'halogène et un groupe alcoxy ayant de 1 à 6 atomes de carbone, et

$R^4$ et le substituant sur le cycle pyrrolidine de formule

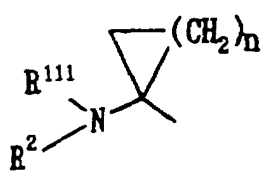

sont localisés en configuration cis ;

chacun de $R^6$ et $R^7$ représente indépendamment un atome d'hydrogène ou un groupe alkyle ayant de 1 à 6 atomes de carbone ;

n est un entier de 1 à 3 ; et

Q' représente un groupe protecteur pour le groupe amino choisi dans l'ensemble constitué par les groupes alcoxycarbonyle éventuellement substitués, les groupes aralkyloxycarbonyle éventuellement substitués, les groupes acyle éventuellement substitués, les groupes alkyle éventuellement substitués, les groupes aralkyle éventuellement substitués et les groupes silyle substitués,

à l'exclusion des composés suivants :

1-benzyloxycarbonyl-4-(R)-(1-tert-butoxycarbonylaminocyclopropyl)-3-(S)-fluoropyrrolidine ;
cis-1-[1-(S)-phényléthyl]-4-(1-tert-butoxycarbonylaminocyclobutyl)-3-fluoropyrrolidine.

**29.** Composé représenté par la formule (VII) suivante, et ses sels et hydrates :

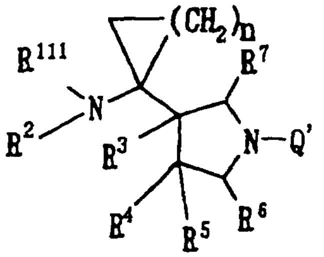

(VII)

dans laquelle

$R^{111}$ représente un atome d'hydrogène, un groupe alkyle ayant de 1 à 6 atomes de carbone ou un groupe protecteur du groupe amino ;

$R^2$ représente un atome d'hydrogène ou un groupe alkyle ayant de 1 à 6 atomes de carbone, dans la mesure où ledit groupe alkyle peut avoir un ou plusieurs substituants choisis dans l'ensemble constitué par un groupe hydroxyle, un atome d'halogène et un groupe alcoxy ayant de 1 à 6 atomes de carbone ;

chacun de $R^3$ et $R^5$ représente un atome d'hydrogène ;

$R^4$ représente un groupe hydroxyle, un atome de chlore, un groupe carbamoyle, un groupe alkyle ayant de 1 à 6 atomes de carbone, un groupe alcoxy ayant de 1 à 6 atomes de carbone ou un groupe alkylthio ayant de 1 à 6 atomes de carbone, dans la mesure où ledit groupe alkyle peut avoir un ou plusieurs substituants choisis dans l'ensemble constitué par un groupe hydroxyle, un atome d'halogène et un groupe alcoxy ayant de 1 à 6 atomes de carbone, et

$R^4$ et le substituant sur le cycle pyrrolidine de formule

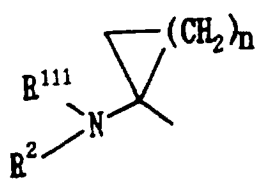

sont localisés en configuration cis ;

chacun de $R^6$ et $R^7$ représente indépendamment un atome d'hydrogène ou un groupe alkyle ayant de 1 à 6 atomes de carbone ;

n est un entier de 1 à 3 ;

Q' représente un groupe protecteur pour le groupe amino choisi dans l'ensemble constitué par les groupes alcoxycarbonyle éventuellement substitués, les groupes aralkyloxycarbonyle éventuellement substitués, les groupes acyle éventuellement substitués, les groupes alkyle éventuellement substitués, les groupes aralkyle éventuellement substitués et les groupes silyle substitués.

**30.** Composé selon l'une quelconque des revendications 25 à 29, dans lequel n vaut 1 ou 2.

**31.** Composé selon l'une quelconque des revendications 25 et 28, dans lequel le substituant $R^4$ est un atome de fluor et n vaut 1 ou 2.

**32.** Composé selon l'une quelconque des revendications 25 à 31, dans lequel le groupe protecteur du groupe amino est choisi dans le groupe constitué par les groupes alcoxycarbonyle éventuellement substitués, les groupes aralkyloxycarbonyle éventuellement substitués, les groupes acyle éventuellement substitués, les groupes alkyle éventuellement substitués, les groupes aralkyle éventuellement substitués et les groupes silyle substitués.